# EUROPEAN PATENT SPECIFICATION

(11) **EP 2 785 870 B1**
(45) Date of publication and mention of the grant of the patent: **03.05.2017**
(21) Application number: 12852815.5
(22) Date of filing: 29.06.2012
(51) Int. Cl.: C12Q 1/68, C12N 15/09, G01N 33/15, G01N 33/574

(54) **SMYD2 AS A TARGET GENE FOR CANCER THERAPY AND DIAGNOSIS**
SMYD2 ALS ZIELGEN FÜR KREBSTHERAPIE UND -DIAGNOSE
SMYD2 EN TANT QUE GÈNE CIBLE POUR UNE THÉRAPIE ANTICANCÉREUSE ET LE DIAGNOSTIC DU CANCER

(30) Priority: 02.12.2011 US 201161566193 P
(43) Date of publication of application: 08.10.2014
(73) Proprietor: OncoTherapy Science, Inc., Kawasaki-shi Kanagawa 213-0012 (JP)
(72) Inventor: HAMAMOTO, Ryuji, Tokyo 108-8639 (JP); NAKAMURA, Yusuke, Tokyo 108-8639 (JP); TSUNODA, Takuya, Kawasaki-shi Kanagawa 213-0012 (JP)
(74) Representative: Dennemeyer & Associates S.A.
(86) International application number: PCT/JP2012/004232
(87) International publication number: WO 2013/080400

(56) References cited:
- JP-A- 2010 220 577
- ANDREWD FERGUSON ET AL: "Structural Basis of Substrate Methylation and Inhibition of SMYD2", STRUCTURE, CURRENT BIOLOGY LTD., PHILADELPHIA, PA, US, vol. 19, no. 9, 13 June 2011 (2011-06-13), pages 1262-1273, XP028284161, ISSN: 0969-2126, DOI: 10.1016/J.STR.2011.06.011 [retrieved on 2011-07-08]
- JIAQUAN WU ET AL: "Biochemical Characterization of Human SET and MYND Domain-Containing Protein 2 Methyltransferase", BIOCHEMISTRY, vol. 50, no. 29, 26 July 2011 (2011-07-26) , pages 6488-6497, XP55196932, ISSN: 0006-2960, DOI: 10.1021/bi200725p
- L. WANG ET AL: "Structure of Human SMYD2 Protein Reveals the Basis of p53 Tumor Suppressor Methylation", JOURNAL OF BIOLOGICAL CHEMISTRY, vol. 286, no. 44, 4 November 2011 (2011-11-04), pages 38725-38737, XP55196933, ISSN: 0021-9258, DOI: 10.1074/jbc.M111.262410
- LISANDRA E WEST ET AL: "Regulation of p53 function by lysine methylation", EPIGENOMICS, vol. 3, no. 3, 1 June 2011 (2011-06-01), pages 361-369, XP55217108, ISSN: 1750-1911, DOI: 10.2217/epi.11.21
- CHO, H.S. ET AL.: 'RB1 methylation by SMYD2 enhances cell cycle progression through an increase of RB1 phosphorylation' NEOPLASIA vol. 14, no. 6, June 2012, pages 476 - 486, XP055071015
- KOMATSU, S. ET AL.: 'Overexpression of SMYD2 relates to tumor cell proliferation and malignant outcome of esophageal squamous cell carcinoma' CARCINOGENESIS vol. 30, no. 7, July 2009, pages 1139 - 1146, XP002603939
- BARROS FILHO, M. C. ET AL.: 'Gene trio signatures as molecular markers to predict response to doxorubicin cyclophosphamide neoadjuvant chemotherapy in breast cancer patients' BRAZILIAN JOURNAL OF MEDICAL AND BIOLOGICAL RESEARCH vol. 43, no. 12, December 2010, pages 1225 - 1231, XP055071016
- ABU-FARHA, M. ET AL.: 'Proteomic analyses of the SMYD family interactomes identify HSP90 as a novel target for SMYD2' JOURNAL OF MOLECULAR CELL BIOLOGY vol. 3, no. 5, October 2011, pages 301 - 308, XP055071017
- BROWN, M.A. ET AL.: 'Identification and characterization of Smyd2: a split SET/MYND domain-containing histone H3 lysine 36-specific methyltransferase that interacts with the Sin3 histone deacetylase complex' MOLECULAR CANCER vol. 5, no. 1, 28 June 2006, pages 1 - 11, XP002616236
- ABU-FARHA, M. ET AL.: 'The tale of two domains: proteomics and genomics analysis of SMYD2, a new histone methyltransferase' MOLECULAR & CELLULAR PROTEOMICS vol. 7, no. 3, March 2008, pages 560 - 572, XP055071019
- SADDIC, L.A. ET AL.: 'Methylation of the retinoblastoma tumor suppressor by SMYD2' JOURNAL OF BIOLOGICAL CHEMISTRY vol. 285, no. 48, 26 November 2010, pages 37733 - 37740, XP055071020
- HUANG, J. ET AL.: 'Repression of p53 activity by Smyd2-mediated methylation' NATURE vol. 444, no. 7119, 30 November 2006, pages 629 - 632, XP002616235

### SEQUENCE LISTING

<110> ONCOTHERAPY SCIENCE, INC.
<120> SMYD2 AS A TARGET GENE FOR CANCER THERAPY AND DIAGNOSIS
<130> ONC-A1115P
<150> US 61/566,193
   <151> 2011-12-02
<160> 83
<170> PatentIn version 3.5
<210> 1
   <211> 20
   <212> DNA
   <213> Artificial Sequence
<220>
   <223> a forward primer sequence
<400> 1
   gcaaattcca tggcaccgtc 20
<210> 2
   <211> 19
   <212> DNA
   <213> Artificial Sequence
<220>
   <223> a reverse primer sequence
<400> 2
   tcgccccact tgattttgg 19
<210> 3
   <211> 20
   <212> DNA
   <213> Artificial Sequence
<220>
   <223> a forward primer sequence
<400> 3
   tgggaacaag agggcatctg 20
<210> 4
   <211> 22
   <212> DNA
   <213> Artificial Sequence
<220>
   <223> a reverse primer sequence
<400> 4
   ccaccactgc atcaaattca tg 22
<210> 5
   <211> 21
   <212> DNA
   <213> Artificial Sequence
<220>
   <223> a forward primer sequence
<400> 5
   atctcctgta cccaacggaa g 21
<210> 6
   <211> 22
   <212> DNA
   <213> Artificial Sequence
<220>
   <223> a reverse primer sequence
<400> 6
   caccttggcc ttatccttgt cc 22
<210> 7
   <211> 19
   <212> RNA
   <213> Artificial Sequence
<220>
   <223> a sense strand sequence of siRNA
<400> 7
   gcagcacgac uucuucaag 19
<210> 8
   <211> 19
   <212> RNA
   <213> Artificial Sequence
<220>
   <223> an antisense strand sequence of siRNA
<400> 8
   cuugaagaag ucgugcugc 19
<210> 9
   <211> 19
   <212> RNA
   <213> Artificial Sequence
<220>
   <223> a sense strand sequence of siRNA
<400> 9
   auccgcgcga uaguacgua 19
<210> 10
   <211> 19
   <212> RNA
   <213> Artificial Sequence
<220>
   <223> an antisense strand sequence of siRNA
<400> 10
   uacguacuau cgcgcggau 19
<210> 11
   <211> 19
   <212> RNA
   <213> Artificial Sequence
<220>
   <223> a sense strand sequence of siRNA
<400> 11
   uuacgcguag cguaauacg 19
<210> 12
   <211> 19
   <212> RNA
   <213> Artificial Sequence
<220>
   <223> an antisense strand sequence of siRNA
<400> 12
   cguauuacgc uacgcguaa 19
<210> 13
   <211> 19
   <212> RNA
   <213> Artificial Sequence
<220>
   <223> a sense strand sequence of siRNA
<400> 13
   uauucgcgcg uauagcggu 19
<210> 14
   <211> 19
   <212> RNA
   <213> Artificial Sequence
<220>
   <223> an antisense strand sequence of siRNA
<400> 14
   accgcuauac gcgcgaaua 19
<210> 15
   <211> 19
   <212> RNA
   <213> Artificial Sequence
<220>
   <223> a sense strand sequence of siRNA
<400> 15
   gauuugauuc agagugaca 19
<210> 16
   <211> 19
   <212> RNA
   <213> Artificial Sequence
<220>
   <223> an antisense strand sequence of siRNA
<400> 16
   ugucacucug aaucaaauc 19
<210> 17
   <211> 19
   <212> RNA
   <213> Artificial Sequence
<220>
   <223> a sense strand sequence of siRNA
<400> 17
   gaaaugaccg guuaagaga 19
<210> 18
   <211> 19
   <212> RNA
   <213> Artificial Sequence
<220>
   <223> an antisense strand sequence of siRNA
<400> 18
   ucucuuaacc ggucauuuc 19
<210> 19
   <211> 41
   <212> DNA
   <213> Artificial Sequence
<220>
   <223> a forward primer sequence
<400> 19
   tgcgcggccg cgggccacca tgagggccga gggcctcggc g 41
<210> 20
   <211> 34
   <212> DNA
   <213> Artificial Sequence
<220>
   <223> a reverse primer sequence
<400> 20
   ccgctcgagg tggctttcaa tttcctgttt gatc 34
<210> 21
   <211> 43
   <212> DNA
   <213> Artificial Sequence
<220>
   <223> a forward primer sequence
<400> 21
   gatatttcct gatgttgcat tgatgtgccc caatgtcatt gtg 43
<210> 22
   <211> 44
   <212> DNA
   <213> Artificial Sequence
<220>
   <223> a forward primer sequence
<400> 22
   ctgtacagga aatcaagccg tttaccagct atattgatct cctg 44
<210> 23
   <211> 20
   <212> DNA
   <213> Artificial Sequence
<220>
   <223> a reverse primer sequence
<400> 23
   tatttgtgag ccagggcatt 20
<210> 24
   <211> 41
   <212> DNA
   <213> Artificial Sequence
<220>
   <223> a forward primer sequence
<400> 24
   tgcgcggccg cgggccacca tgagggccga gggcctcggc g 41
<210> 25
   <211> 31
   <212> DNA
   <213> Artificial Sequence
<220>
   <223> a reverse primer sequence
<400> 25
   ccgctcgagc cagttttccc caaaaacaac c 31
<210> 26
   <211> 41
   <212> DNA
   <213> Artificial Sequence
<220>
   <223> a forward primer sequence
<400> 26
   tgcgcggccg cgggccacca tgagggccga gggcctcggc g 41
<210> 27
   <211> 33
   <212> DNA
   <213> Artificial Sequence
<220>
   <223> a reverse primer sequence
<400> 27
   ccgctcgagt ctatcttccg ttgggtacag gag 33
<210> 28
   <211> 41
   <212> DNA
   <213> Artificial Sequence
<220>
   <223> a forward primer sequence
<400> 28
   tgcgcggccg ctcgccacca tgtggaatcc ctcggagact g 41
<210> 29
   <211> 34
   <212> DNA
   <213> Artificial Sequence
<220>
   <223> a reverse primer sequence
<400> 29
   ccgctcgagg tggctttcaa tttcctgttt gatc 34
<210> 30
   <211> 41
   <212> DNA
   <213> Artificial Sequence
<220>
   <223> a forward primer sequence
<400> 30
   tgcgcggccg ctcgccacca tgagaaatga ccggttaaga g 41
<210> 31
   <211> 34
   <212> DNA
   <213> Artificial Sequence
<220>
   <223> a reverse primer sequence
<400> 31
   ccgctcgagg tggctttcaa tttcctgttt gatc 34
<210> 32
   <211> 44
   <212> DNA
   <213> Artificial Sequence
<220>
   <223> a forward primer sequence
<400> 32
   tgcgcggccg ctcgccacca tgtctgtgtt tgaggacagt aacg 44
<210> 33
   <211> 34
   <212> DNA
   <213> Artificial Sequence
<220>
   <223> a reverse primer sequence
<400> 33
   ccgctcgagg tggctttcaa tttcctgttt gatc 34
<210> 34
   <211> 38
   <212> DNA
   <213> Artificial Sequence
<220>
   <223> a forward primer sequence
<400> 34
   tgccaattgg gccaccatgc ctgaggaagt gcaccatg 38
<210> 35
   <211> 31
   <212> DNA
   <213> Artificial Sequence
<220>
   <223> a reverse primer sequence
<400> 35
   tgcgtcgaca tcgacttctt ccatgcgaga c 31
<210> 36
   <211> 38
   <212> DNA
   <213> Artificial Sequence
<220>
   <223> a forward primer sequence
<400> 36
   tgccaattgg gccaccatgc ctgaggaagt gcaccatg 38
<210> 37
   <211> 30
   <212> DNA
   <213> Artificial Sequence
<220>
   <223> a reverse primer sequence
<400> 37
   tgcgtcgacc acaaaagctg agttggccac 30
<210> 38
   <211> 41
   <212> DNA
   <213> Artificial Sequence
<220>
   <223> a forward primer sequence
<400> 38
   tgccaattgg gccaccatga tcgaagatgt gggttcagat g 41
<210> 39
   <211> 31
   <212> DNA
   <213> Artificial Sequence
<220>
   <223> a reverse primer sequence
<400> 39
   tgcgtcgaca tcgacttctt ccatgcgaga c 31
<210> 40
   <211> 43
   <212> DNA
   <213> Artificial Sequence
<220>
   <223> a forward primer sequence
<400> 40
   tgccaattgg gccaccatgg tggagcgagt gcggaaacgg ggc 43
<210> 41
   <211> 31
   <212> DNA
   <213> Artificial Sequence
<220>
   <223> a reverse primer sequence
<400> 41
   tgcgtcgaca tcgacttctt ccatgcgaga c 31
<210> 42
   <211> 36
   <212> DNA
   <213> Artificial Sequence
<220>
   <223> a forward primer sequence
<400> 42
   caaggaattt gatggggcga gcctggtctc agttac 36
<210> 43
   <211> 36
   <212> DNA
   <213> Artificial Sequence
<220>
   <223> a reverse primer sequence
<400> 43
   gtaactgaga ccaggctcgc cccatcaaat tccttg 36
<210> 44
   <211> 24
   <212> DNA
   <213> Artificial Sequence
<220>
   <223> a forward primer sequence
<400> 44
   gcggttgaga aggtgacaat ctcc 24
<210> 45
   <211> 28
   <212> DNA
   <213> Artificial Sequence
<220>
   <223> a reverse primer sequence
<400> 45
   cttatctaag atttctttca tgagcttg 28
<210> 46
   <211> 30
   <212> DNA
   <213> Artificial Sequence
<220>
   <223> a forward primer sequence
<400> 46
   cgggatccat gagggccgag ggcctcggcg 30
<210> 47
   <211> 34
   <212> DNA
   <213> Artificial Sequence
<220>
   <223> a reverse primer sequence
<400> 47
   ccgctcgagt cagtggcttt caatttcctg tttg 34
<210> 48
   <211> 28
   <212> DNA
   <213> Artificial Sequence
<220>
   <223> a forward primer sequence
<400> 48
   tgccaattga tgcctgagga agtgcacc 28
<210> 49
   <211> 32
   <212> DNA
   <213> Artificial Sequence
<220>
   <223> a reverse primer sequence
<400> 49
   tgcgtcgacc taatcgactt cttccatgcg ag 32
<210> 50
   <211> 28
   <212> DNA
   <213> Artificial Sequence
<220>
   <223> a forward primer sequence
<400> 50
   tgccaattga tgcctgagga agtgcacc 28
<210> 51
   <211> 36
   <212> DNA
   <213> Artificial Sequence
<220>
   <223> a reverse primer sequence
<400> 51
   tgcgtcgacc tagatcttgg gcttttcttc atcatc 36
<210> 52
   <211> 28
   <212> DNA
   <213> Artificial Sequence
<220>
   <223> a forward primer sequence
<400> 52
   tgccaattga tgcctgagga agtgcacc 28
<210> 53
   <211> 33
   <212> DNA
   <213> Artificial Sequence
<220>
   <223> a reverse primer sequence
<400> 53
   tgcgtcgacc tacacaaaag ctgagttggc cac 33
<210> 54
   <211> 34
   <212> DNA
   <213> Artificial Sequence
<220>
   <223> a forward primer sequence
<400> 54
   tgccaattga tgatcgaaga tgtgggttca gatg 34
<210> 55
   <211> 32
   <212> DNA
   <213> Artificial Sequence
<220>
   <223> a reverse primer sequence
<400> 55
   tgcgtcgacc taatcgactt cttccatgcg ag 32
<210> 56
   <211> 36
   <212> DNA
   <213> Artificial Sequence
<220>
   <223> a forward primer sequence
<400> 56
   tgccaattga tggtggagcg agtgcggaaa cggggc 36
<210> 57
   <211> 32
   <212> DNA
   <213> Artificial Sequence
<220>
   <223> a reverse primer sequence
<400> 57
   tgcgtcgacc taatcgactt cttccatgcg ag 32
<210> 58
   <211> 36
   <212> DNA
   <213> Artificial Sequence
<220>
   <223> a forward primer sequence
<400> 58
   caaggaattt gatggggcga gcctggtctc agttac 36
<210> 59
   <211> 36
   <212> DNA
   <213> Artificial Sequence
<220>
   <223> a reverse primer sequence
<400> 59
   gtaactgaga ccaggctcgc ccatcaaat tccttg 36
<210> 60
   <211> 24
   <212> DNA
   <213> Artificial Sequence
<220>
   <223> a forward primer sequence
<400> 60
   gcggttgaga aggtgacaat ctcc 24
<210> 61
   <211> 28
   <212> DNA
   <213> Artificial Sequence
<220>
   <223> a reverse primer sequence
<400> 61
   cttatctaag atttctttca tgagcttg 28
<210> 62
   <211> 1689
   <212> DNA
   <213> Homo sapiens
<220>
   <221> CDS
   <222> (34)..(1335)
<400> 62
   gggcacagcc ggcggccgcg ccccgccgcc acc atg agg gcc gag ggc ctc ggc      54
                                        Met Arg Ala Glu Gly Leu Gly
                                        1               5
   ggc ctg gag cgc ttc tgc agc ccg ggc aaa ggc cgg ggg ctg cgg gct      102
   Gly Leu Glu Arg Phe Cys Ser Pro Gly Lys Gly Arg Gly Leu Arg Ala
           10                  15                  20
   ctg cag ccc ttc cag gtg ggg gac ttg ctg ttc tcc tgc ccg gcc tat      150
   Leu Gln Pro Phe Gln Val Gly Asp Leu Leu Phe Ser Cys Pro Ala Tyr
       25                  30                  35
   gcc tac gtg ctc acg gtc aac gag cgg ggc aac cac tgc gag tac tgc      198
   Ala Tyr Val Leu Thr Val Asn Glu Arg Gly Asn His Cys Glu Tyr Cys
   40                  45                  50                  55
   ttc acc agg aaa gaa gga ttg tcc aaa tgt gga aga tgc aag cag gca      246
   Phe Thr Arg Lys Glu Gly Leu Ser Lys Cys Gly Arg Cys Lys Gln Ala
                   60                  65                  70
   ttt tac tgc aat gtg gag tgt cag aaa gaa gat tgg ccc atg cac aag      294
   Phe Tyr Cys Asn Val Glu Cys Gln Lys Glu Asp Trp Pro Met His Lys
               75                  80                  85
   ctg gaa tgt tct ccc atg gtt gtt ttt ggg gaa aac tgg aat ccc tcg      342
   Leu Glu Cys Ser Pro Met Val Val Phe Gly Glu Asn Trp Asn Pro Ser
           90                  95                  100
   gag act gta aga cta aca gca agg att ctg gcc aaa cag aaa atc cac      390
   Glu Thr Val Arg Leu Thr Ala Arg Ile Leu Ala Lys Gln Lys Ile His
       105                 110                 115
   cca gag aga aca cct tcg gaa aaa ttg tta gct gtg aag gag ttt gaa      438
   Pro Glu Arg Thr Pro Ser Glu Lys Leu Leu Ala Val Lys Glu Phe Glu
   120                 125                 130                 135
   tca cat ctg gat aag tta gac aat gag aag aag gat ttg att cag agt      486
   Ser His Leu Asp Lys Leu Asp Asn Glu Lys Lys Asp Leu Ile Gln Ser
                   140                 145                 150
   gac ata gct gct ctc cat cac ttt tac tcc aag cat ctc gga ttc cct      534
   Asp Ile Ala Ala Leu His His Phe Tyr Ser Lys His Leu Gly Phe Pro
               155                 160                 165
   gac aat gat agc ctc gta gta ctc ttt gca cag gtt aac tgt aat ggc      582
   Asp Asn Asp Ser Leu Val Val Leu Phe Ala Gln Val Asn Cys Asn Gly
           170                 175                 180
   ttc aca att gaa gat gaa gaa ctt tct cat ttg gga tca gcg ata ttt      630
   Phe Thr Ile Glu Asp Glu Glu Leu Ser His Leu Gly Ser Ala Ile Phe
       185                 190                 195
   cct gat gtt gca ttg atg aat cat agc tgt tgc ccc aat gtc att gtg      678
   Pro Asp Val Ala Leu Met Asn His Ser Cys Cys Pro Asn Val Ile Val
   200                 205                 210                 215
   acc tac aaa ggg acc ctg gca gaa gtc aga gct gta cag gaa atc aag      726
   Thr Tyr Lys Gly Thr Leu Ala Glu Val Arg Ala Val Gln Glu Ile Lys
                   220                 225                 230
   ccg gga gag gag gtt ttt acc agc tat att gat ctc ctg tac cca acg      774
   Pro Gly Glu Glu Val Phe Thr Ser Tyr Ile Asp Leu Leu Tyr Pro Thr
               235                 240                 245
   gaa gat aga aat gac cgg tta aga gat tct tat ttc ttt acc tgt gag       822
   Glu Asp Arg Asn Asp Arg Leu Arg Asp Ser Tyr Phe Phe Thr Cys Glu
           250                 255                 260
   tgc cag gag tgt acc acc aag gac aag gat aag gcc aag gtg gaa atc       870
   Cys Gln Glu Cys Thr Thr Lys Asp Lys Asp Lys Ala Lys Val Glu Ile
       265                 270                 275
   cgg aag ctc agc gat ccc cca aag gca gaa gcc atc cga gac atg gtc       918
   Arg Lys Leu Ser Asp Pro Pro Lys Ala Glu Ala Ile Arg Asp Met Val
   280                 285                 290                 295
   aga tat gca cgc aac gtc att gaa gag ttc cgg agg gcc aag cac tat       966
   Arg Tyr Ala Arg Asn Val Ile Glu Glu Phe Arg Arg Ala Lys His Tyr
                   300                 305                 310
   aaa tcc cct agt gag ctg ctg gag atc tgc gag ctc agc cag gag aag      1014
   Lys Ser Pro Ser Glu Leu Leu Glu Ile Cys Glu Leu Ser Gln Glu Lys
               315                 320                 325
   atg agc tct gtg ttt gag gac agt aac gtg tac atg ttg cac atg atg      1062
   Met Ser Ser Val Phe Glu Asp Ser Asn Val Tyr Met Leu His Met Met
           330                 335                 340
   tac cag gcc atg ggt gtc tgc ttg tac atg cag gac tgg gaa gga gcc      1110
   Tyr Gln Ala Met Gly Val Cys Leu Tyr Met Gln Asp Trp Glu Gly Ala
       345                 350                 355
   ctg caa tat gga cag aaa atc att aag ccc tac agt aag cac tat cct      1158
   Leu Gln Tyr Gly Gln Lys Ile Ile Lys Pro Tyr Ser Lys His Tyr Pro
   360                 365                 370                 375
   ttg tac tcc ctc aac gtg gcc tcc atg tgg ttg aag cta ggg aga ctc      1206
   Leu Tyr Ser Leu Asn Val Ala Ser Met Trp Leu Lys Leu Gly Arg Leu
                   380                 385                 390
   tac atg ggc ctg gaa cac aaa gcc gca ggg gag aaa gcc ctg aag aag      1254
   Tyr Met Gly Leu Glu His Lys Ala Ala Gly Glu Lys Ala Leu Lys Lys
               395                 400                 405
   gcc att gca atc atg gaa gta gct cac ggc aaa gat cat cca tat att      1302
   Ala Ile Ala Ile Met Glu Val Ala His Gly Lys Asp His Pro Tyr Ile
           410                 415                 420
   tct gag atc aaa cag gaa att gaa agc cac tga aactatgcag catttcagtt    1355
   Ser Glu Ile Lys Gln Glu Ile Glu Ser His
       425                 430
   ttcatttaaa cacttagttc agaaacctta aaggatttga atatttcaaa ttgcacacgt    1415
   cactccagca tctctgtaaa ataattggaa tgaaaatact tcttgcactt aaacactgca    1475
   catgccgtac tttgaggtta gtctgaatct tgaactttaa taccaaatta attttgaatg    1535
   cttttgtttc ctaagagata atggcatggt ttcatatgtt atactttgga cagacagagt    1595
   tttaaaaatg gaattatttt ttctttcatg cctcttgtaa tgttctgaac aaacttgaat    1655
   gatgaaagta ttaaagagat atcagtattt aaaa                                1689
<210> 63
   <211> 433
   <212> PRT
   <213> Homo sapiens
<400> 63
   Met Arg Ala Glu Gly Leu Gly Gly Leu Glu Arg Phe Cys Ser Pro Gly
   1               5                   10                  15
   Lys Gly Arg Gly Leu Arg Ala Leu Gln Pro Phe Gln Val Gly Asp Leu
               20                  25                  30
   Leu Phe Ser Cys Pro Ala Tyr Ala Tyr Val Leu Thr Val Asn Glu Arg
           35                  40                  45
   Gly Asn His Cys Glu Tyr Cys Phe Thr Arg Lys Glu Gly Leu Ser Lys
       50                  55                  60
   Cys Gly Arg Cys Lys Gln Ala Phe Tyr Cys Asn Val Glu Cys Gln Lys
   65                  70                  75                  80
   Glu Asp Trp Pro Met His Lys Leu Glu Cys Ser Pro Met Val Val Phe
                   85                  90                  95
   Gly Glu Asn Trp Asn Pro Ser Glu Thr Val Arg Leu Thr Ala Arg Ile
               100                 105                 110
   Leu Ala Lys Gln Lys Ile His Pro Glu Arg Thr Pro Ser Glu Lys Leu
           115                 120                 125
   Leu Ala Val Lys Glu Phe Glu Ser His Leu Asp Lys Leu Asp Asn Glu
       130                 135                 140
   Lys Lys Asp Leu Ile Gln Ser Asp Ile Ala Ala Leu His His Phe Tyr
   145                 150                 155                 160
   Ser Lys His Leu Gly Phe Pro Asp Asn Asp Ser Leu Val Val Leu Phe
                   165                 170                 175
   Ala Gln Val Asn Cys Asn Gly Phe Thr Ile Glu Asp Glu Glu Leu Ser
               180                 185                 190
   His Leu Gly Ser Ala Ile Phe Pro Asp Val Ala Leu Met Asn His Ser
           195                 200                 205
   Cys Cys Pro Asn Val Ile Val Thr Tyr Lys Gly Thr Leu Ala Glu Val
       210                 215                 220
   Arg Ala Val Gln Glu Ile Lys Pro Gly Glu Glu Val Phe Thr Ser Tyr
   225                 230                 235                 240
   Ile Asp Leu Leu Tyr Pro Thr Glu Asp Arg Asn Asp Arg Leu Arg Asp
                   245                 250                 255
   Ser Tyr Phe Phe Thr Cys Glu Cys Gln Glu Cys Thr Thr Lys Asp Lys
               260                 265                 270
   Asp Lys Ala Lys Val Glu Ile Arg Lys Leu Ser Asp Pro Pro Lys Ala
           275                 280                 285
   Glu Ala Ile Arg Asp Met Val Arg Tyr Ala Arg Asn Val Ile Glu Glu
       290                 295                 300
   Phe Arg Arg Ala Lys His Tyr Lys Ser Pro Ser Glu Leu Leu Glu Ile
   305                 310                 315                 320
   Cys Glu Leu Ser Gln Glu Lys Met Ser Ser Val Phe Glu Asp Ser Asn
                   325                 330                 335
   Val Tyr Met Leu His Met Met Tyr Gln Ala Met Gly Val Cys Leu Tyr
               340                 345                 350
   Met Gln Asp Trp Glu Gly Ala Leu Gln Tyr Gly Gln Lys Ile Ile Lys
           355                 360                 365
   Pro Tyr Ser Lys His Tyr Pro Leu Tyr Ser Leu Asn Val Ala Ser Met
       370                 375                 380
   Trp Leu Lys Leu Gly Arg Leu Tyr Met Gly Leu Glu His Lys Ala Ala
   385                 390                 395                 400
   Gly Glu Lys Ala Leu Lys Lys Ala Ile Ala Ile Met Glu Val Ala His
                   405                 410                 415
   Gly Lys Asp His Pro Tyr Ile Ser Glu Ile Lys Gln Glu Ile Glu Ser
               420                 425                 430
   His
<210> 64
   <211> 2567
   <212> DNA
   <213> Homo sapiens
<220>
   <221> CDS
   <222> (85)..(2259)
<400> 64
   ctccggcgca gtgttgggac tgtctgggta tcggaaagca agcctacgtt gctcactatt       60
   acgtataatc cttttctttt caag atg cct gag gaa gtg cac cat gga gag        111
                              Met Pro Glu Glu Val His His Gly Glu
                              1               5
   gag gag gtg gag act ttt gcc ttt cag gca gaa att gcc caa ctc atg        159
   Glu Glu Val Glu Thr Phe Ala Phe Gln Ala Glu Ile Ala Gln Leu Met
   10                  15                  20                  25
   tcc ctc atc atc aat acc ttc tat tcc aac aag gag att ttc ctt cgg        207
   Ser Leu Ile Ile Asn Thr Phe Tyr Ser Asn Lys Glu Ile Phe Leu Arg
                   30                  35                  40
   gag ttg atc tct aat gct tct gat gcc ttg gac aag att cgc tat gag        255
   Glu Leu Ile Ser Asn Ala Ser Asp Ala Leu Asp Lys Ile Arg Tyr Glu
               45                  50                  55
   agc ctg aca gac cct tcg aag ttg gac agt ggt aaa gag ctg aaa att        303
   Ser Leu Thr Asp Pro Ser Lys Leu Asp Ser Gly Lys Glu Leu Lys Ile
           60                  65                  70
   gac atc atc ccc aac cct cag gaa cgt acc ctg act ttg gta gac aca        351
   Asp Ile Ile Pro Asn Pro Gln Glu Arg Thr Leu Thr Leu Val Asp Thr
       75                  80                  85
   ggc att ggc atg acc aaa gct gat ctc ata aat aat ttg gga acc att        399
   Gly Ile Gly Met Thr Lys Ala Asp Leu Ile Asn Asn Leu Gly Thr Ile
   90                  95                  100                 105
   gcc aag tct ggt act aaa gca ttc atg gag gct ctt cag gct ggt gca        447
   Ala Lys Ser Gly Thr Lys Ala Phe Met Glu Ala Leu Gln Ala Gly Ala
                   110                 115                 120
   gac atc tcc atg att ggg cag ttt ggt gtt ggc ttt tat tct gcc tac        495
   Asp Ile Ser Met Ile Gly Gln Phe Gly Val Gly Phe Tyr Ser Ala Tyr
               125                 130                 135
   ttg gtg gca gag aaa gtg gtt gtg atc aca aag cac aac gat gat gaa        543
   Leu Val Ala Glu Lys Val Val Val Ile Thr Lys His Asn Asp Asp Glu
           140                 145                 150
   cag tat gct tgg gag tct tct gct gga ggt tcc ttc act gtg cgt gct        591
   Gln Tyr Ala Trp Glu Ser Ser Ala Gly Gly Ser Phe Thr Val Arg Ala
       155                 160                 165
   gac cat ggt gag ccc att ggc agg ggt acc aaa gtg atc ctc cat ctt        639
   Asp His Gly Glu Pro Ile Gly Arg Gly Thr Lys Val Ile Leu His Leu
   170                 175                 180                 185
   aaa gaa gat cag aca gag tac cta gaa gag agg cgg gtc aaa gaa gta        687
   Lys Glu Asp Gln Thr Glu Tyr Leu Glu Glu Arg Arg Val Lys Glu Val
                   190                 195                 200
   gtg aag aag cat tct cag ttc ata ggc tat ccc atc acc ctt tat ttg        735
   Val Lys Lys His Ser Gln Phe Ile Gly Tyr Pro Ile Thr Leu Tyr Leu
               205                 210                 215
   gag aag gaa cga gag aag gaa att agt gat gat gag gca gag gaa gag       783
   Glu Lys Glu Arg Glu Lys Glu Ile Ser Asp Asp Glu Ala Glu Glu Glu
           220                 225                 230
   aaa ggt gag aaa gaa gag gaa gat aaa gat gat gaa gaa aaa ccc aag       831
   Lys Gly Glu Lys Glu Glu Glu Asp Lys Asp Asp Glu Glu Lys Pro Lys
       235                 240                 245
   atc gaa gat gtg ggt tca gat gag gag gat gac agc ggt aag gat aag       879
   Ile Glu Asp Val Gly Ser Asp Glu Glu Asp Asp Ser Gly Lys Asp Lys
   250                 255                 260                 265
   aag aag aaa act aag aag atc aaa gag aaa tac att gat cag gaa gaa       927
   Lys Lys Lys Thr Lys Lys Ile Lys Glu Lys Tyr Ile Asp Gln Glu Glu
                   270                 275                 280
   cta aac aag acc aag cct att tgg acc aga aac cct gat gac atc acc       975
   Leu Asn Lys Thr Lys Pro Ile Trp Thr Arg Asn Pro Asp Asp Ile Thr
               285                 290                 295
   caa gag gag tat gga gaa ttc tac aag agc ctc act aat gac tgg gaa      1023
   Gln Glu Glu Tyr Gly Glu Phe Tyr Lys Ser Leu Thr Asn Asp Trp Glu
           300                 305                 310
   gac cac ttg gca gtc aag cac ttt tct gta gaa ggt cag ttg gaa ttc      1071
   Asp His Leu Ala Val Lys His Phe Ser Val Glu Gly Gln Leu Glu Phe
       315                 320                 325
   agg gca ttg cta ttt att cct cgt cgg gct ccc ttt gac ctt ttt gag      1119
   Arg Ala Leu Leu Phe Ile Pro Arg Arg Ala Pro Phe Asp Leu Phe Glu
   330                 335                 340                 345
   aac aag aag aaa aag aac aac atc aaa ctc tat gtc cgc cgt gtg ttc      1167
   Asn Lys Lys Lys Lys Asn Asn Ile Lys Leu Tyr Val Arg Arg Val Phe
                   350                 355                 360
   atc atg gac agc tgt gat gag ttg ata cca gag tat ctc aat ttt atc      1215
   Ile Met Asp Ser Cys Asp Glu Leu Ile Pro Glu Tyr Leu Asn Phe Ile
               365                 370                 375
   cgt ggt gtg gtt gac tct gag gat ctg ccc ctg aac atc tcc cga gaa      1263
   Arg Gly Val Val Asp Ser Glu Asp Leu Pro Leu Asn Ile Ser Arg Glu
           380                 385                 390
   atg ctc cag cag agc aaa atc ttg aaa gtc att cgc aaa aac att gtt      1311
   Met Leu Gln Gln Ser Lys Ile Leu Lys Val Ile Arg Lys Asn Ile Val
       395                 400                 405
   aag aag tgc ctt gag ctc ttc tct gag ctg gca gaa gac aag gag aat      1359
   Lys Lys Cys Leu Glu Leu Phe Ser Glu Leu Ala Glu Asp Lys Glu Asn
   410                 415                 420                 425
   tac aag aaa ttc tat gag gca ttc tct aaa aat ctc aag ctt gga atc      1407
   Tyr Lys Lys Phe Tyr Glu Ala Phe Ser Lys Asn Leu Lys Leu Gly Ile
                   430                 435                 440
   cac gaa gac tcc act aac cgc cgc cgc ctg tct gag ctg ctg cgc tat      1455
   His Glu Asp Ser Thr Asn Arg Arg Arg Leu Ser Glu Leu Leu Arg Tyr
               445                 450                 455
   cat acc tcc cag tct gga gat gag atg aca tct ctg tca gag tat gtt      1503
   His Thr Ser Gln Ser Gly Asp Glu Met Thr Ser Leu Ser Glu Tyr Val
           460                 465                 470
   tct cgc atg aag gag aca cag aag tcc atc tat tac atc act ggt gag         1551
   Ser Arg Met Lys Glu Thr Gln Lys Ser Ile Tyr Tyr Ile Thr Gly Glu
       475                 480                 485
   agc aaa gag cag gtg gcc aac tca gct ttt gtg gag cga gtg cgg aaa         1599
   Ser Lys Glu Gln Val Ala Asn Ser Ala Phe Val Glu Arg Val Arg Lys
   490                 495                 500                 505
   cgg ggc ttc gag gtg gta tat atg acc gag ccc att gac gag tac tgt         1647
   Arg Gly Phe Glu Val Val Tyr Met Thr Glu Pro Ile Asp Glu Tyr Cys
                   510                 515                 520
   gtg cag cag ctc aag gaa ttt gat ggg aag agc ctg gtc tca gtt acc         1695
   Val Gln Gln Leu Lys Glu Phe Asp Gly Lys Ser Leu Val Ser Val Thr
               525                 530                 535
   aag gag ggt ctg gag ctg cct gag gat gag gag gag aag aag aag atg         1743
   Lys Glu Gly Leu Glu Leu Pro Glu Asp Glu Glu Glu Lys Lys Lys Met
           540                 545                 550
   gaa gag agc aag gca aag ttt gag aac ctc tgc aag ctc atg aaa gaa         1791
   Glu Glu Ser Lys Ala Lys Phe Glu Asn Leu Cys Lys Leu Met Lys Glu
       555                 560                 565
   atc tta gat aag aag gtt gag aag gtg aca atc tcc aat aga ctt gtg         1839
   Ile Leu Asp Lys Lys Val Glu Lys Val Thr Ile Ser Asn Arg Leu Val
   570                 575                 580                 585
   tct tca cct tgc tgc att gtg acc agc acc tac ggc tgg aca gcc aat         1887
   Ser Ser Pro Cys Cys Ile Val Thr Ser Thr Tyr Gly Trp Thr Ala Asn
                   590                 595                 600
   atg gag cgg atc atg aaa gcc cag gca ctt cgg gac aac tcc acc atg         1935
   Met Glu Arg Ile Met Lys Ala Gln Ala Leu Arg Asp Asn Ser Thr Met
               605                 610                 615
   ggc tat atg atg gcc aaa aag cac ctg gag atc aac cct gac cac ccc         1983
   Gly Tyr Met Met Ala Lys Lys His Leu Glu Ile Asn Pro Asp His Pro
           620                 625                 630
   att gtg gag acg ctg cgg cag aag gct gag gcc gac aag aat gat aag         2031
   Ile Val Glu Thr Leu Arg Gln Lys Ala Glu Ala Asp Lys Asn Asp Lys
       635                 640                 645
   gca gtt aag gac ctg gtg gtg ctg ctg ttt gaa acc gcc ctg cta tct         2079
   Ala Val Lys Asp Leu Val Val Leu Leu Phe Glu Thr Ala Leu Leu Ser
   650                 655                 660                 665
   tct ggc ttt tcc ctt gag gat ccc cag acc cac tcc aac cgc atc tat         2127
   Ser Gly Phe Ser Leu Glu Asp Pro Gln Thr His Ser Asn Arg Ile Tyr
                   670                 675                 680
   cgc atg atc aag cta ggt cta ggt att gat gaa gat gaa gtg gca gca         2175
   Arg Met Ile Lys Leu Gly Leu Gly Ile Asp Glu Asp Glu Val Ala Ala
               685                 690                 695
   gag gaa ccc aat gct gca gtt cct gat gag atc ccc cct ctc gag ggc         2223
   Glu Glu Pro Asn Ala Ala Val Pro Asp Glu Ile Pro Pro Leu Glu Gly
           700                 705                 710
   gat gag gat gcg tct cgc atg gaa gaa gtc gat tag gttaggagtt              2269
   Asp Glu Asp Ala Ser Arg Met Glu Glu Val Asp
       715                 720
   catagttgga aaacttgtgc ccttgtatag tgtccccatg ggctcccact gcagcctcga    2329
   gtgcccctgt cccacctggc tccccctgct ggtgtctagt gtttttttcc ctctcctgtc    2389
   cttgtgttga aggcagtaaa ctaagggtgt caagccccat tccctctcta ctcttgacag    2449
   caggattgga tgttgtgtat tgtggtttat tttattttct tcattttgtt ctgaaattaa    2509
   agtatgcaaa ataaagaata tgccgtttta aaaaaaaaaa aaaaaaaaaa aaaaaaaa      2567
<210> 65
   <211> 724
   <212> PRT
   <213> Homo sapiens
<400> 65
   Met Pro Glu Glu Val His His Gly Glu Glu Glu Val Glu Thr Phe Ala
   1               5                   10                  15
   Phe Gln Ala Glu Ile Ala Gln Leu Met Ser Leu Ile Ile Asn Thr Phe
               20                  25                  30
   Tyr Ser Asn Lys Glu Ile Phe Leu Arg Glu Leu Ile Ser Asn Ala Ser
           35                  40                  45
   Asp Ala Leu Asp Lys Ile Arg Tyr Glu Ser Leu Thr Asp Pro Ser Lys
       50                  55                  60
   Leu Asp Ser Gly Lys Glu Leu Lys Ile Asp Ile Ile Pro Asn Pro Gln
   65                  70                  75                  80
   Glu Arg Thr Leu Thr Leu Val Asp Thr Gly Ile Gly Met Thr Lys Ala
                   85                  90                  95
   Asp Leu Ile Asn Asn Leu Gly Thr Ile Ala Lys Ser Gly Thr Lys Ala
               100                 105                 110
   Phe Met Glu Ala Leu Gln Ala Gly Ala Asp Ile Ser Met Ile Gly Gln
           115                 120                 125
   Phe Gly Val Gly Phe Tyr Ser Ala Tyr Leu Val Ala Glu Lys Val Val
       130                 135                 140
   Val Ile Thr Lys His Asn Asp Asp Glu Gln Tyr Ala Trp Glu Ser Ser
   145                 150                 155                 160
   Ala Gly Gly Ser Phe Thr Val Arg Ala Asp His Gly Glu Pro Ile Gly
                   165                 170                 175
   Arg Gly Thr Lys Val Ile Leu His Leu Lys Glu Asp Gln Thr Glu Tyr
               180                 185                 190
   Leu Glu Glu Arg Arg Val Lys Glu Val Val Lys Lys His Ser Gln Phe
           195                 200                 205
   Ile Gly Tyr Pro Ile Thr Leu Tyr Leu Glu Lys Glu Arg Glu Lys Glu
       210                 215                 220
   Ile Ser Asp Asp Glu Ala Glu Glu Glu Lys Gly Glu Lys Glu Glu Glu
   225                 230                 235                 240
   Asp Lys Asp Asp Glu Glu Lys Pro Lys Ile Glu Asp Val Gly Ser Asp
                   245                 250                 255
   Glu Glu Asp Asp Ser Gly Lys Asp Lys Lys Lys Lys Thr Lys Lys Ile
               260                 265                 270
   Lys Glu Lys Tyr Ile Asp Gln Glu Glu Leu Asn Lys Thr Lys Pro Ile
           275                 280                 285
   Trp Thr Arg Asn Pro Asp Asp Ile Thr Gln Glu Glu Tyr Gly Glu Phe
       290                 295                 300
   Tyr Lys Ser Leu Thr Asn Asp Trp Glu Asp His Leu Ala Val Lys His
   305                 310                 315                 320
   Phe Ser Val Glu Gly Gln Leu Glu Phe Arg Ala Leu Leu Phe Ile Pro
                   325                 330                 335
   Arg Arg Ala Pro Phe Asp Leu Phe Glu Asn Lys Lys Lys Lys Asn Asn
               340                 345                 350
   Ile Lys Leu Tyr Val Arg Arg Val Phe Ile Met Asp Ser Cys Asp Glu
           355                 360                 365
   Leu Ile Pro Glu Tyr Leu Asn Phe Ile Arg Gly Val Val Asp Ser Glu
       370                 375                 380
   Asp Leu Pro Leu Asn Ile Ser Arg Glu Met Leu Gln Gln Ser Lys Ile
   385                 390                 395                 400
   Leu Lys Val Ile Arg Lys Asn Ile Val Lys Lys Cys Leu Glu Leu Phe
                   405                 410                 415
   Ser Glu Leu Ala Glu Asp Lys Glu Asn Tyr Lys Lys Phe Tyr Glu Ala
               420                 425                 430
   Phe Ser Lys Asn Leu Lys Leu Gly Ile His Glu Asp Ser Thr Asn Arg
   435 440 445
   Arg Arg Leu Ser Glu Leu Leu Arg Tyr His Thr Ser Gln Ser Gly Asp
   450 455 460
   Glu Met Thr Ser Leu Ser Glu Tyr Val Ser Arg Met Lys Glu Thr Gln
   465 470 475 480
   Lys Ser Ile Tyr Tyr Ile Thr Gly Glu Ser Lys Glu Gln Val Ala Asn
   485 490 495
   Ser Ala Phe Val Glu Arg Val Arg Lys Arg Gly Phe Glu Val Val Tyr
   500 505 510
   Met Thr Glu Pro Ile Asp Glu Tyr Cys Val Gln Gln Leu Lys Glu Phe
   515 520 525
   Asp Gly Lys Ser Leu Val Ser Val Thr Lys Glu Gly Leu Glu Leu Pro
   530 535 540
   Glu Asp Glu Glu Glu Lys Lys Lys Met Glu Glu Ser Lys Ala Lys Phe
   545 550 555 560
   Glu Asn Leu Cys Lys Leu Met Lys Glu Ile Leu Asp Lys Lys Val Glu
   565 570 575
   Lys Val Thr Ile Ser Asn Arg Leu Val Ser Ser Pro Cys Cys Ile Val
   580 585 590
   Thr Ser Thr Tyr Gly Trp Thr Ala Asn Met Glu Arg Ile Met Lys Ala
   595 600 605
   Gln Ala Leu Arg Asp Asn Ser Thr Met Gly Tyr Met Met Ala Lys Lys
   610 615 620
   His Leu Glu Ile Asn Pro Asp His Pro Ile Val Glu Thr Leu Arg Gln
   625 630 635 640
   Lys Ala Glu Ala Asp Lys Asn Asp Lys Ala Val Lys Asp Leu Val Val
   645 650 655
   Leu Leu Phe Glu Thr Ala Leu Leu Ser Ser Gly Phe Ser Leu Glu Asp
   660 665 670
   Pro Gln Thr His Ser Asn Arg Ile Tyr Arg Met Ile Lys Leu Gly Leu
                   675                 680                 685
   Gly Ile Asp Glu Asp Glu Val Ala Ala Glu Glu Pro Asn Ala Ala Val
       690                 695                 700
   Pro Asp Glu Ile Pro Pro Leu Glu Gly Asp Glu Asp Ala Ser Arg Met
   705                 710                 715                 720
   Glu Glu Val Asp
<210> 66
   <211> 21
   <212> PRT
   <213> Artificial Sequence
<220>
   <223> An artificially synthesized peptide
<400> 66
   Ser Gly Arg Gly Lys Gly Gly Lys Gly Leu Gly Lys Gly Gly Ala Lys
   1               5                   10                  15
   Arg His Arg Lys Val
               20
<210> 67
   <211> 4772
   <212> DNA
   <213> Homo sapiens
<220>
   <221> CDS
   <222> (167)..(2953)
<400> 67
gctcagttgc cgggcggggg agggcgcgtc cggtttttct caggggacgt tgaaattatt        60
   tttgtaacgg gagtcgggag aggacggggc gtgccccgac gtgcgcgcgc gtcgtcctcc       120
   ccggcgctcc tccacagctc gctggctccc gccgcggaaa ggcgtc atg ccg ccc          175
   aaa acc ccc cga aaa acg gcc gcc acc gcc gcc gct gcc gcc gcg gaa         223
   ccc ccg gca ccg ccg ccg ccg ccc cct cct gag gag gac cca gag cag         271
   gac agc ggc ccg gag gac ctg cct ctc gtc agg ctt gag ttt gaa gaa         319
   aca gaa gaa cct gat ttt act gca tta tgt cag aaa tta aag ata cca        367
   gat cat gtc aga gag aga gct tgg tta act tgg gag aaa gtt tca tct        415
   gtg gat gga gta ttg gga ggt tat att caa aag aaa aag gaa ctg tgg        463
   gga atc tgt atc ttt att gca gca gtt gac cta gat gag atg tcg ttc        511
   act ttt act gag cta cag aaa aac ata gaa atc agt gtc cat aaa ttc        559
   ttt aac tta cta aaa gaa att gat acc agt acc aaa gtt gat aat gct        607
   atg tca aga ctg ttg aag aag tat gat gta ttg ttt gca ctc ttc agc        655
   aaa ttg gaa agg aca tgt gaa ctt ata tat ttg aca caa ccc agc agt        703
   tcg ata tct act gaa ata aat tct gca ttg gtg cta aaa gtt tct tgg        751
   atc aca ttt tta tta gct aaa ggg gaa gta tta caa atg gaa gat gat        799
   ctg gtg att tca ttt cag tta atg cta tgt gtc ctt gac tat ttt att        847
   aaa ctc tca cct ccc atg ttg ctc aaa gaa cca tat aaa aca gct gtt        895
   ata ccc att aat ggt tca cct cga aca ccc agg cga ggt cag aac agg        943
   agt gca cgg ata gca aaa caa cta gaa aat gat aca aga att att gaa        991
   gtt ctc tgt aaa gaa cat gaa tgt aat ata gat gag gtg aaa aat gtt       1039
   tat ttc aaa aat ttt ata cct ttt atg aat tct ctt gga ctt gta aca       1087
   tct aat gga ctt cca gag gtt gaa aat ctt tct aaa cga tac gaa gaa      1135
   att tat ctt aaa aat aaa gat cta gat gca aga tta ttt ttg gat cat      1183
   gat aaa act ctt cag act gat tct ata gac agt ttt gaa aca cag aga      1231
   aca cca cga aaa agt aac ctt gat gaa gag gtg aat gta att cct cca      1279
   cac act cca gtt agg act gtt atg aac act atc caa caa tta atg atg      1327
   att tta aat tca gca agt gat caa cct tca gaa aat ctg att tcc tat      1375
   ttt aac aac tgc aca gtg aat cca aaa gaa agt ata ctg aaa aga gtg      1423
   aag gat ata gga tac atc ttt aaa gag aaa ttt gct aaa gct gtg gga      1471
   cag ggt tgt gtc gaa att gga tca cag cga tac aaa ctt gga gtt cgc      1519
   ttg tat tac cga gta atg gaa tcc atg ctt aaa tca gaa gaa gaa cga      1567
   tta tcc att caa aat ttt agc aaa ctt ctg aat gac aac att ttt cat      1615
   atg tct tta ttg gcg tgc gct ctt gag gtt gta atg gcc aca tat agc      1663
   aga agt aca tct cag aat ctt gat tct gga aca gat ttg tct ttc cca      1711
   tgg att ctg aat gtg ctt aat tta aaa gcc ttt gat ttt tac aaa gtg      1759
   atc gaa agt ttt atc aaa gca gaa ggc aac ttg aca aga gaa atg ata      1807
   aaa cat tta gaa cga tgt gaa cat cga atc atg gaa tcc ctt gca tgg      1855
   ctc tca gat tca cct tta ttt gat ctt att aaa caa tca aag gac cga        1903
   gaa gga cca act gat cac ctt gaa tct gct tgt cct ctt aat ctt cct        1951
   ctc cag aat aat cac act gca gca gat atg tat ctt tct cct gta aga        1999
   tct cca aag aaa aaa ggt tca act acg cgt gta aat tct act gca aat        2047
   gca gag aca caa gca acc tca gcc ttc cag acc cag aag cca ttg aaa        2095
   tct acc tct ctt tca ctg ttt tat aaa aaa gtg tat cgg cta gcc tat        2143
   ctc cgg cta aat aca ctt tgt gaa cgc ctt ctg tct gag cac cca gaa        2191
   tta gaa cat atc atc tgg acc ctt ttc cag cac acc ctg cag aat gag        2239
   tat gaa ctc atg aga gac agg cat ttg gac caa att atg atg tgt tcc        2287
   atg tat ggc ata tgc aaa gtg aag aat ata gac ctt aaa ttc aaa atc        2335
   att gta aca gca tac aag gat ctt cct cat gct gtt cag gag aca ttc        2383
   aaa cgt gtt ttg atc aaa gaa gag gag tat gat tct att ata gta ttc        2431
   tat aac tcg gtc ttc atg cag aga ctg aaa aca aat att ttg cag tat        2479
   gct tcc acc agg ccc cct acc ttg tca cca ata cct cac att cct cga        2527
   agc cct tac aag ttt cct agt tca ccc tta cgg att cct gga ggg aac        2575
   atc tat att tca ccc ctg aag agt cca tat aaa att tca gaa ggt ctg        2623
   cca aca cca aca aaa atg act cca aga tca aga atc tta gta tca att        2671
   ggt gaa tca ttc ggg act tct gag aag ttc cag aaa ata aat cag atg        2719
   gta tgt aac agc gac cgt gtg ctc aaa aga agt gct gaa gga agc aac        2767
   cct cct aaa cca ctg aaa aaa cta cgc ttt gat att gaa gga tca gat        2815
   gaa gca gat gga agt aaa cat ctc cca gga gag tcc aaa ttt cag cag        2863
   aaa ctg gca gaa atg act tct act cga aca cga atg caa aag cag aaa        2911
   atg aat gat agc atg gat acc tca aac aag gaa gag aaa tga                2953
   ggatctcagg accttggtgg acactgtgta cacctctgga ttcattgtct ctcacagatg     3013
   tgactgtata actttcccag gttctgttta tggccacatt taatatcttc agctcttttt     3073
   gtggatataa aatgtgcaga tgcaattgtt tgggtgattc ctaagccact tgaaatgtta     3133
   gtcattgtta tttatacaag attgaaaatc ttgtgtaaat cctgccattt aaaaagttgt     3193
   agcagattgt ttcctcttcc aaagtaaaat tgctgtgctt tatggatagt aagaatggcc     3253
   ctagagtggg agtcctgata acccaggcct gtctgactac tttgccttct tttgtagcat     3313
   ataggtgatg tttgctcttg tttttattaa tttatatgta tattttttta atttaacatg     3373
   aacaccctta gaaaatgtgt cctatctatc ttccaaatgc aatttgattg actgcccatt     3433
   caccaaaatt atcctgaact cttctgcaaa aatggatatt attagaaatt agaaaaaaat     3493
   tactaatttt acacattaga ttttatttta ctattggaat ctgatatact gtgtgcttgt     3553
   tttataaaat tttgctttta attaaataaa agctggaagc aaagtataac catatgatac     3613
   tatcatacta ctgaaacaga tttcatacct cagaatgtaa aagaacttac tgattatttt     3673
   cttcatccaa cttatgtttt taaatgagga ttattgatag tactcttggt ttttatacca     3733
   ttcagatcac tgaatttata aagtacccat ctagtacttg aaaaagtaaa gtgttctgcc     3793
   agatcttagg tatagaggac cctaacacag tatatcccaa gtgcactttc taatgtttct     3853
   gggtcctgaa gaattaagat acaaattaat tttactccat aaacagactg ttaattatag     3913
   gagccttaat ttttttttca tagagatttg tctaattgca tctcaaaatt attctgccct 3973
   ccttaatttg ggaaggtttg tgttttctct ggaatggtac atgtcttcca tgtatctttt 4033
   gaactggcaa ttgtctattt atcttttatt tttttaagtc agtatggtct aacactggca 4093
   tgttcaaagc cacattattt ctagtccaaa attacaagta atcaagggtc attatgggtt 4153
   aggcattaat gtttctatct gattttgtgc aaaagcttca aattaaaaca gctgcattag 4213
   aaaaagaggc gcttctcccc tcccctacac ctaaaggtgt atttaaacta tcttgtgtga 4273
   ttaacttatt tagagatgct gtaacttaaa ataggggata tttaaggtag cttcagctag 4333
   cttttaggaa aatcactttg tctaactcag aattattttt aaaaagaaat ctggtcttgt 4393
   tagaaaacaa aattttattt tgtgctcatt taagtttcaa acttactatt ttgacagtta 4453
   ttttgataac aatgacacta gaaaacttga ctccatttca tcattgtttc tgcatgaata 4513
   tcatacaaat cagttagttt ttaggtcaag ggcttactat ttctgggtct tttgctacta 4573
   agttcacatt agaattagtg ccagaatttt aggaacttca gagatcgtgt attgagattt 4633
   cttaaataat gcttcagata ttattgcttt attgcttttt tgtattggtt aaaactgtac 4693
   atttaaaatt gctatgttac tattttctac aattaatagt ttgtctattt taaaataaat 4753
   tagttgttaa gagtcttaa 4772
<210> 68
   <211> 928
   <212> PRT
   <213> Homo sapiens
<400> 68
<210> 69
   <211> 19
   <212> PRT
   <213> Artificial Sequence
<220>
   <223> An artifically synthesized peptide
<400> 69
   Cys Gly Gly Asn Ile Tyr Ile Ser Pro Leu Lys Ser Pro Tyr Lys Ile
   1 5 10 15
   Ser Glu Gly
<210> 70
   <211> 19
   <212> PRT
   <213> Artificial Sequence
<220>
   <223> An artificially synthesized peptide
<220>
   <221> MOD_RES
   <222> (11)..(11)
   <223> METHYLATION
<400> 70
   Cys Gly Gly Asn Ile Tyr Ile Ser Pro Leu Lys Ser Pro Tyr Lys Ile
   1 5 10 15
   Ser Glu Gly
<210> 71
   <211> 20
   <212> PRT
   <213> Homo sapiens
<400> 71
   Gln Leu Lys Glu Phe Asp Gly Lys Ser Leu Val Ser Val Thr Lys Glu
   1               5                   10                  15
   Gly Leu Glu Leu
               20
<210> 72
   <211> 20
   <212> PRT
   <213> Oryctolagus cuniculus
<400> 72
   Gln Leu Lys Glu Phe Asp Gly Lys Ser Leu Val Ser Val Thr Lys Glu
   1               5                   10                  15
   Gly Leu Glu Leu
               20
<210> 73
   <211> 20
   <212> PRT
   <213> Rattus norvegicus
<400> 73
   Gln Leu Lys Glu Phe Asp Gly Lys Ser Leu Val Ser Val Thr Lys Glu
   1               5                   10                  15
   Gly Leu Glu Leu
               20
<210> 74
   <211> 20
   <212> PRT
   <213> Mus musculus
<400> 74
   Gln Leu Lys Glu Phe Asp Gly Lys Ser Leu Val Ser Val Thr Lys Glu
   1               5                   10                  15
   Gly Leu Glu Leu
               20
<210> 75
   <211> 20
   <212> PRT
   <213> Xenopus laevis
<400> 75
   Gln Leu Lys Glu Phe Asp Gly Lys Thr Leu Val Ser Val Thr Lys Glu
   1               5                   10                  15
   Gly Leu Glu Leu
               20
<210> 76
   <211> 20
   <212> PRT
   <213> Danio rerio
<400> 76
   Gln Leu Lys Asp Phe Asp Gly Lys Ser Leu Val Ser Val Thr Lys Glu
   1               5                   10                  15
   Gly Leu Glu Leu
               20
<210> 77
   <211> 17
   <212> PRT
   <213> Homo sapiens
<400> 77
   Met Lys Glu Ile Leu Asp Lys Lys Val Thr Ile Ser Asn Arg Leu Val
   1               5                   10                  15
   Ser
<210> 78
   <211> 17
   <212> PRT
   <213> Oryctolagus cuniculus
<400> 78
   Met Lys Glu Ile Leu Asp Lys Lys Val Thr Ile Ser Asn Arg Leu Val
   1               5                   10                  15
   Ser
<210> 79
   <211> 17
   <212> PRT
   <213> Rattus norvegicus
<400> 79
   Met Lys Glu Ile Leu Asp Lys Lys Val Thr Ile Ser Asn Arg Leu Val
   1 5 10 15
   Ser
<210> 80
   <211> 17
   <212> PRT
   <213> Mus musculus
<400> 80
   Met Lys Glu Ile Leu Asp Lys Lys Val Thr Ile Ser Asn Arg Leu Val
   1 5 10 15
   Ser
<210> 81
   <211> 17
   <212> PRT
   <213> Xenopus laevis
<400> 81
   Met Lys Glu Ile Leu Asp Lys Lys Val Thr Val Ser Asn Arg Leu Val
   1 5 10 15
   Ser
<210> 82
   <211> 17
   <212> PRT
   <213> Danio rerio
<400> 82
   Met Lys Glu Ile Leu Asp Lys Lys Val Thr Val Ser Asn Arg Leu Val
   1 5 10 15
   Ser
<210> 83
   <211> 23
   <212> PRT
   <213> Homo sapiens
<400> 83
   Phe Pro Ser Ser Pro Leu Arg Ile Pro Gly Gly Asn Ile Tyr Ile Ser
   1 5 10 15
   Pro Leu Lys Ser Pro Tyr Lys
   20

## Description

### Technical Field

The present invention relates to the field of biological science, more specifically to the field of cancer research, cancer diagnosis and cancer therapy. In particular, the present invention relates to methods for detecting and diagnosing the presence and/or predisposition for developing cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. The present invention also relates to methods for treating and preventing cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. The present invention further relates to methods of screening for a candidate substance effective for the treatment and/or prevention of cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

### PRIORITY

The present application claims the benefit of U.S. Provisional Applications No. 61/566,193, filed on December 2, 2011.

### Background Art

Heat shock protein 90 (HSP90) is an evolutionarily conserved molecular chaperone that participates in stabilizing and activating more than 200 proteins referred to as HSP90 "clients"; many of which are essential for constitutive cell signaling and adaptive responses to stress [NPL1.2]. To accomplish this task, HSP90 and additional proteins termed "co-chaperones" form the dynamic complex known as the HSP90 chaperone machine [NPL3]. Cancer cells use the HSP90 chaperone machinery to protect an array of mutated and over-expressed oncoproteins from misfolding and degradation. Therefore, HSP90 is recognized as a crucial facilitator of oncogene addiction and cancer survival [NPL4].

HSP90 function is regulated by various post-translational modifications such as acetylation, phosphorylation and nitrosylation. Phosphorylation of the charged linker on HSP90beta regulates the interaction with the aryl hydrocarbon receptor (AHR) client. Mutation of phosphorylated Ser 225 and Ser 254 to Ala in the HSP90 middle domain increases HSP90 binding to AHR, suggesting that phosphorylation negatively regulates the complex [NPL5]. In other cases, HSP90 phosphorylation facilitates client maturation. For example, SRC-dependent phosphorylation of HSP90AB1 (on Tyr 300) increases the chaperone's association with the client endothelial nitric oxide synthase (eNOS) on activation of vascular endothelial growth signaling [NPL6]. Meanwhile, acetylation of Lys 294 in the middle domain by an unknown acetylase inhibits both client protein maturation and co-chaperone binding [NPL7, 8], histone deacetylase 6 deacetylates this residue in vivo. Moreover, nitrosylation of Cys 597 in the HSP90AB 1 CTD inhibits eNOS activation in vivo [NPL9, 10], and in vitro S-nitrosylation inhibits HSP90 ATPase activity and shifts the conformational equilibrium of the chaperone cycle. Other post-translational modifications have been reported for HSP90. However, the physiological significance of methylation has yet to be elucidated.

The retinoblastoma tumor suppressor protein (RB) has a central role in cell cycle regulation and is mutated in several types of cancer [NPL23-25]. RB can interact with the E2F transcription factor and regulate genes related to S phase entry. In its hypo-phosphorylated state, RB binds to E2F and represses the expression of E2F target genes. When RB is hyper-phosphorylated by the Cyclin/CDK complexes, E2F is separated from RB and transactivates its target genes that drive cell cycle progression [NPL23,24,26,27]. Although RB has been reported to be inactivated in more than 90% of human small-cell lung carcinomas (SCLC) [NPL23], the majority of human cancers express a wild-type RB that is predominantly at a phosphorylated state due to the deregulation of CDKs. Thereby, most human cancers appear to have lost the G₁ checkpoint control through the deregulation of RB functions, and thus phosphorylation of RB is the key regulatory step in the pathway controlling proliferation of cancer cells [NPL24]. In addition to phosphorylation, the RB protein has been known to be acetylated [NPL28,29]. During keratinocyte differentiation, RB is acetylated by the acetyltransferase P-CAF, and the acetylation of two major lysine residues (lysines 873 and 874) that are located within the nuclear localization signal is likely to play a crucial role in differentiation through the retention of the RB protein in the nucleus [NPL30]. However, the significance of other posttranslational modifications (PTMs), including lysine methylation for regulation of RB functions, still remains unclear.

The present inventors have demonstrated that certain histone methyltransferases (HMTs) play a vital role in human cancer pathogenesis, in addition to normal cellular biology [PL1-2, NPL11-13]. HMTs have also been suggested to be involved malignant alterations of human cells [NPL14-16].

SMYD2 was first identified as one of the SMYD family members that contain a SET domain and a MYND domain [NPL17]. SMYD2 has been shown to methylate H3K36 mark and function as a transcriptional repressor in Cooperation with the Sin3A and HDACl histone deacetylase complex [NPL17]. In addition to the histone methylation process, SMYD2 also methylates p53 and retinoblastoma (RB) proteins, and thereby alters their functions [NPL18,19]. In Xenopurs laevis, smyd2 is expressed in muscle tissues and thus maybe associated with muscle cells differentiation [NPL20]. Smyd2 has also been shown to be expressed in various types of neonatal mouse tissues, especially neonatal heart [NPL21], though unlike SMYD1 which is indispensable for cardiomyocyte differentiation and cardiac morphogenesis, SMYD2 is not critical for mouse heart development [NPL22].
[NPL31] discloses a method of screening for an inhibitor of the p53 methylation activity of SMYD2.

Thus, while certain properties have been clarified, the significance of SMYD2 in both normal cellular biology and diseases like Cancer remains largely unclear.

### Citation List

### Patent Literature

[PTL 1] WO2005/071102
[PTL 2] WO2003/027143

### Non Patent Literature

[NPL 1] Trepel, J., Mollapour, M., Giaccone, G. & Neckers, L. Nat Rev Cancer 10, 537-549 (2010).
[NPL 2] Wandinger, S.K., Richter, K. & Buchner, J. J Biol Chem 283, 18473-18477 (2008).
[NPL 3] Pratt, W.B. & Toft, D.O. Exp Biol Med (Maywood) 228, 111-133 (2003).
[NPL 4] Whitesell, L. & Lindquist, S.L. Nat Rev Cancer 5, 761-772 (2005).
[NPL 5] Ogiso, H. et al. Biochemistry 43, 15510-15519 (2004).
[NPL 6] Duval, M., Le Boeuf, F., Huot, J. & Gratton, J.P. Mol Biol Cell 18, 4659-4668 (2007).
[NPL 7] Scroggins, B.T. et al. Mol Cell 25, 151-159 (2007).
[NPL 8] Kovacs, JJ. et al. Mol Cell 18, 601-607 (2005).
[NPL 9] Retzlaff, M. et al. EMBO Rep 10, 1147-1153 (2009).
[NPL10] Martinez-Ruiz, A. et al. Proc Natl Acad Sei U S A 102, 8525-8530 (2005).
[NPL11] Hamamoto R et al. Nat Cell Biol 6,731-40 (2004)
[NPL 12] Takawa, M. et al. Cancer Sei (2011).
[NPL 13] Yoshimatsu, M. et al. Int J Cancer 128, 562-573 (2011).
[NPL 14] Portela, A. & Esteller, M. 1 28, 1057-1068 (2010).
[NPL15] Schneider, R., Bannister, A.J. & Kouzarides, T. Trends Biochem Sei 27, 396-402 (2002).
[NPL 16] Sparmann, A. & van Lohuizen, M. Nat Rev Cancer 6, 846-856 (2006).
[NPL 17] Brown, M.A., Sims, R.J., 3rd, Gottlieb, P.D. & Tucker, P.W.Mol Cancer 5, 26 (2006).
[NPL18] Huang, J. et al. Nature 444, 629-632 (2006).
[NPL19] Saddic, L.A. et al. J Biol Chem 285, 37733-37740 (2010).
[NPL20] Kawamura, S., Yoshigai, E., Kuhara, S. & Tashiro, K. Cytotechnology 57, 161-168 (2008).
[NPL21] Diehl, F. et al. PLoS One 5, e9748 (2010).
[NPL22] Gottlieb, P.D. et al. Nat Genet 31, 25-32 (2002).
[NPL23] Burkhart, DL. et al. Nat Rev Cancer 8, 671-682 (2008).
[NPL24] Knudsen, ES. et al. Nat Rev Cancer 8, 714-724 (2008).
[NPL25] Weinberg, RA. et al. Cell 81, 323-330 (1995).
[NPL26] SherrCJ. Science 274, 1672-1677 (1996).
[NPL27] Sherr, CJ. et al. Cancer Cell 2, 103-112 (2002).
[NPL28] Chan, HM. et al. Nat Cell Biol 3, 667-674 (2001).
[NPL29] Nguyen, DX. et al. EMBO J 23, 1609-1618 (2004).
[NPL30] Pickard, A. et al. J Cell Sci 123, 3718-3726 (2010).
[NPL31] Andrew D. Ferguson, et al. Structure, Current Biology Ltd., vol. 19, no.9, 1262-1273 (2011) Ferguson et al. teaches a method of screening for an inhibitor of the p53 methylation activity of SMYD2.

### Summary of Invention

The present inventors relates to the SMYD2 (SET and MYND domain containing 2) gene and the crucial role it plays in cancer cell growth. As such, the present invention relates to novel compositions and methods for detecting, diagnosing, treating and/or preventing cancer as well as over-express methods of screening for candidate substances useful for either or both of cancer prevention and treatment.

In the course of the present invention, the present inventors confirmed that the SMYD2 gene is over-expressed in various cancers, including, for example, bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. The present inventors also confirmed the methyltransferase activities of SMYD2 protein for HSP90AB1 protein and RB1 protein.

In view of these findings, it was postulated that the oncogenic activity of the SMYD2 protein is brought out through the interaction with the histone protein, HSP90AB1 protein or RBI protein, so as to play an important role in cancer cells. In the course of the present invention, it was discovered that SMYD2 protein promotes cancer cell proliferation through methylation of histone protein, HSP90AB1 protein and/or RB 1 protein.

Using a conventional in vitro methyltransferase assay, the present inventors demonstrated that SMYD2 protein methylates HSP90AB1 protein in a dose-dependent manner. Through mass spectrometric analysis, the present inventors identified lysine 531 and/or lysine 574 of HSP90AB1 protein as the primary target(s) of SMYD2-dependent methylation. The present inventors further confirmed that mono-methylation at lysine 574 of HSP90AB1 protein promoted the dimerization and chaperonin complex formation. Additionally, methylated HSP90AB1 protein accelerated the proliferation of cancer cells.

The present inventors also demonstrated that SMYD2 protein methylates RB1 protein. Mass spectrometric analysis revealed that lysine 810 of RB1 was methylated by SMYD2 protein. This methylation enhanced serine 807 and/or serine 811 phosphorylation of RB 1 protein both in vitro and in vivo. Furthermore, the present inventors demonstrated that methylated RB 1 protein accelerates E2F transcriptional activity and promotes cell cycle progression. These results indicates that SMYD2 protein is an important oncoprotein in various types of cancer, and SMYD2-dependent RB 1 methylation at lysine 810 promotes cell cycle progression of cancer cells.

Taken together, this data suggests that targeting the SMYD2 molecule may hold promise for the development of a new diagnostic and therapeutic strategy in the clinical management of cancers.

It is therefore an object of the present invention to provide a method of diagnosing or determining the presence or predisposition for developing cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer in a subject using the expression level of SMYD2 gene in a subject derived biological sample as an index of disease. An increase in the expression level of the SMYD2 gene as compared to a normal control level of the gene indicates that the subject suffers from or is at risk of developing cancer, particularly bladder cancer, lung cancer, breast cancer, cervix cancer, colon cancer, kidney cancer, liver cancer, head and neck cancer, seminoma, cutaneous cancer and/or pancreatic cancer.

It is a further object of the present invention to provide a method of screening for a candidate substance effective for the treatment and/or prevention of cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. Such a substance would bind with the SMYD2 polypeptide or reduce the biological activity of the SMYD2 polypeptide or the expression of the SMYD2 gene or reporter gene surrogating the SMYD2 gene. Alternatively, such substance would inhibit the binding between the SMYD2 polypeptide and the HSP90AB1 polypeptide or the RB 1 polypeptide, or inhibit the methyltransferase activity of the SMYD2 polypeptide.

More specifically, the present invention provides the following [1] to [33]:
[1] A method for detecting or diagnosing cancer or detecting a predisposition for developing the cancer in a subject, said method comprising a step of determining an expression level of an SMYD2 gene in a subject-derived biological sample, wherein an increase in said expression level as compared to a normal control level of said gene indicates that said subject suffers from or is at a risk of developing cancer, wherein said expression level is determined by any method selected from a group consisting of:
   (a) detecting an mRNA of the SMYD2 gene;
   (b) detecting a protein encoded by the SMYD2 gene; and
   (c) detecting a biological activity of a protein encoded by the SMYD2 gene,
[2] The method of [1], wherein said increase is at least 10% greater than said normal control level,
[3] The method of [1], wherein the subject-derived biological sample comprises a biopsy specimen, saliva, sputum, blood, serum, plasma, pleural effusion or urine sample,
[4] A kit for detecting or diagnosing the presence of or a predisposition for developing cancer in a subject, which comprises a reagent selected from the group consisting of:
   (a) a reagent for detecting an mRNA of an SMYD2 gene;
   (b) a reagent for detecting the protein encoded by an SMYD2 gene and
   (c) a reagent for detecting the biological activity of the protein encoded by an SMYD2 gene,
[5] The kit of [4], wherein the reagent is a probe or primer set that bind to the mRNA of the SMYD2 gene,
[6] The kit of [4], wherein the reagent is an antibody against the protein encoded by the SMYD2 gene or a fragment thereof,
[7] The method of any one of [1] to [3], or the kit of any one of [4] to [6], wherein the biological activity is cell-proliferation promoting activity or methyltransferase activity,
[8] A method of screening for a candidate substance for either or both of treating and preventing cancer or inhibiting cancer cell growth, said method comprising the steps of:
   (a) contacting a test substance with an SMYD2 polypeptide or functional equivalent thereof;
   (b) detecting the binding activity between the polypeptide or functional equivalent thereof and the test substance; and
   (c) selecting the test substance that binds to the polypeptide or functional equivalent thereof,
[9] A method of screening for a candidate substance for either or both of treating and preventing cancer or inhibiting cancer cell growth, said method comprising the steps of:
   (a) contacting a test substance with a cell expressing an SMYD2 gene; and
   (b) selecting the test substance that reduces the expression level of the SMYD2 gene in comparison with the expression level in the absence of the test substance,
[10] A method of screening for a candidate substance for either or both of treating and preventing cancer or inhibiting cancer cell growth, said method comprising the steps of:
   (a) contacting a test substance with an SMYD2 polypeptide or functional equivalent thereof;
   (b) detecting a biological activity of the polypeptide or functional equivalent thereof of step (a); and
   (c) selecting the test substance that suppresses the biological activity of the polypeptide or functional equivalent thereof in comparison with the biological activity detected in the absence of the test substance,
[11] The method of [10], wherein the biological activity is cell-proliferation promoting activity or methyltransferase activity,
[12] A method of screening for a candidate substance for either or both of treating and preventing cancer or inhibiting cancer cell growth, said method comprising the steps of:
   (a) contacting a test substance with a cell into which a vector comprising the transcriptional regulatory region of an SMYD2 gene and a reporter gene that is expressed under the control of the transcriptional regulatory region has been introduced,
   (b) measuring the expression or activity level of said reporter gene; and
   (c) selecting the test substance that reduces the expression or activity level of said reporter gene, as compared to a level in the absence of the test substance,
[13] A method of screening for a candidate substance for either or both of treating and preventing cancer or inhibiting cancer cell growth, said method comprising the steps of:
   (a) contacting an SMYD2 polypeptide or functional equivalent thereof with a substrate to be methylated in the presence of a test substance under the condition capable of methylation of the substrate;
   (b) detecting the methylation level of the substrate; and
   (c) selecting the test substance that decreases the methylation level of the substrate as compared to the methylation level detected in the absence of the test substance,
[14] The method of [13], wherein the substrate is a histone protein or a fragment thereof that comprises at least one methylation site,
[15] The method of [14], wherein the histone is histone H4 or histone H3,
[16] The method of [13], wherein the substrate is an HSP90AB1 polypeptide or a fragment thereof that comprises at least one methylation site,
[17] The method of [16], wherein the methylation site is the lysine 531 and/or lysine 574 of SEQ ID NO: 65,
[18] The method of [13], wherein the substrate is an RB1 polypeptide or a fragment thereof that comprises at least one methylation site,
[19] The method of [18], wherein the methylation site is the lysine 810 of SEQ ID NO: 68,
[20] A method of screening for a candidate substance for treating or preventing cancer, or inhibiting cancer cell growth, said method comprising the steps of:
   (a) contacting a polypeptide comprising an HSP90AB1-binding domain of an SMYD2 polypeptide with a polypeptide comprising an SMYD2-binding domain of an HSP90AB1 polypeptide in the presence of a test substance;
   (b) detecting a binding between the polypeptides;
   (c) comparing the binding level detected in the step (b) with that detected in the absence of the test substance; and
   (d) selecting the test substance that inhibits the binding between the polypeptides as a candidate substance for either or both of treating and preventing cancer or inhibiting cancer cell growth,
[21] The method of [20], wherein the polypeptide comprising the HSP90AB1-binding domain comprises amino acid residues 100-247 of SEQ ID NO:63,
[22] The method of [20], wherein the polypeptide comprising the SMYD2-binding domain comprises amino acid residues 500-724 of SEQ ID NO: 65,
[23] A method of screening for a candidate substance for either or both of treating and preventing cancer, or inhibiting cancer cell growth, said method comprising the steps of:
   (a) contacting a polypeptide comprising an RB1-binding domain of an SMYD2 polypeptide with a polypeptide comprising an SMYD2-binding domain of an RB 1 polypeptide in the presence of a test substance;
   (b) detecting a binding between the polypeptides; and
   (c) comparing the binding level detected in the step (b) with that detected in the absence of the test substance; and
   (d) selecting the test substance that inhibits the binding between the polypeptides as a candidate substance for either or both of treating and preventing cancer or inhibiting cancer cell growth,
[24] The method of [23], wherein the polypeptide comprising the RB1-binding domain comprises amino acid residues 330-433 of SEQ ID NO:63,
[25] The method of [23], wherein the polypeptide comprising the SMYD2-binding domain comprises amino acid residues 773-813 of SEQ ID NO: 68,
[26] A method of screening for a candidate substance for treating or preventing cancer, or inhibiting cancer cell growth, said method comprising the steps of:
   (a) contacting a test substance with a cell expressing an SMYD2 gene and an RB1 gene;
   (b) detecting the phosphorylation level of the RB 1 polypeptide or functional equivalent thereof of step (a); and;
   (c) selecting the test substance that reduces the phosphorylation level of the polypeptide or functional equivalent thereof in comparison with the phosphorylation level detected in the absence of the test substance,
[27] The method of [26], the phosphorylation level of the RB1 polypeptide is detected by an antibody against phosphorylated RB 1 at the serine 807 and/or serine 811 of SEQ ID NO: 68,
[28] A kit for screening for a candidate substance for either or both of treating and preventing cancer, or inhibiting cancer cell growth, wherein said kit comprises the following components:
   (a) an SMYD2 polypeptide or a functional equivalent thereof;
   (b) a component selected from the group consisting of (i) to (iii)
      (i) a histone protein or a fragment thereof that comprises at least one methylation site,
      (ii) an HSP90AB1 polypeptide or a functional equivalent thereof;
      (iii) an RB1 polypeptide or a functional equivalent thereof;
   (c) a reagent selected from the group consisting of (i) to (iii);
      (i) a reagent for detecting the methylation level of the histone protein or the functional equivalent thereof;
      (ii) a reagent for detecting the methylation level of the HSP90AB1 polypeptide or a functional equivalent thereof;
      (iii) a reagent for detecting the methylation level of the RB 1 polypeptide or a functional equivalent thereof; and
   (d) a methyl donor,
[29] The kit of [28], wherein the histone protein is a histone H4 or a histone H3,
[30] The kit of [28], wherein the reagent in the step (c) (i) is an antibody against the methylated histone H4 protein or the methylated histone H3 protein,
[31] The kit of [28], wherein the reagent in the step (c) (ii) is an antibody against an HSP90AB1 polypeptide methylated at the lysine 531 and/or lysine 574 of SEQ ID NO: 65,
[32] The kit of [28], wherein the reagent in the step (c) (iii) is an antibody against an RB1 polypeptide methylated at the lysine 810 of SEQ ID NO: 68, and
[33] The kit of any one of [28] to [32], wherein the methyl donor is S-adenosyl methionine.

It will be understood by those skilled in the art that one or more aspects of this invention can meet certain objectives, while one or more other aspects can meet certain other objectives. Each objective may not apply equally, in ail its respects, to every aspect of this invention. As such, the preceding objects can be viewed in the alternative with respect to any one aspect of this invention.

These and other objects and features of the invention will become more fully apparent when the following detailed description is read in conjunction with the accompanying figures and examples. However, it is to be understood that both the foregoing summary of the present invention and the following detailed description are of an exemplified embodiment, and not restrictive of the present invention or other alternate embodiments of the present invention. Other objects and features of the invention will become more fully apparent when the following detailed description is read in conjunction with the accompanying figures and examples.

In particular, while the invention is described herein with reference to a number of specific embodiments, it will be appreciated that the description is illustrative of the invention and is not constructed as limiting of the invention. Various modifications and applications may occur to those who are skilled in the art, as described by the appended claims. Likewise, other objects, features, benefits and advantages of the present invention will be apparent from this summary and certain embodiments described below, and will be readily apparent to those skilled in the art. Such objects, features, benefits and ad-vantages will be apparent from the above in conjunction with the accompanying examples, data, figures and ail reasonable inferences to be drawn therefrom, alone or with consideration of the references referred herein.

### Brief Description of Drawings

Various aspects and applications of the present invention will become apparent to the skilled artisan upon consideration of the brief description of the figures and the detailed description of the present invention and its preferred embodiments that follows:

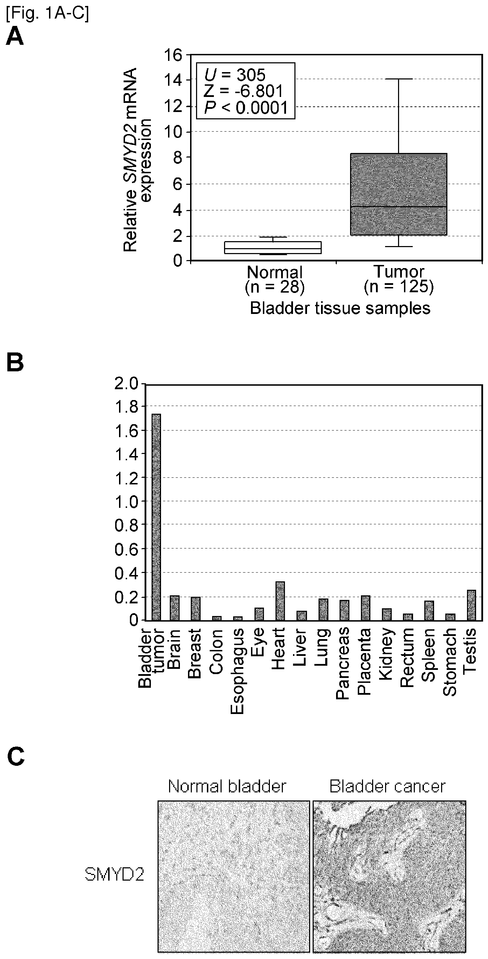

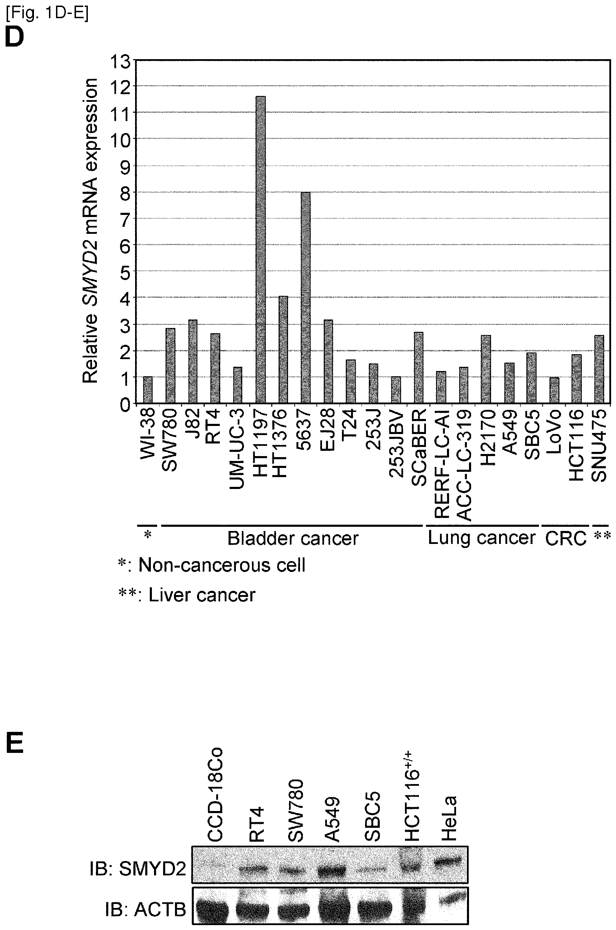

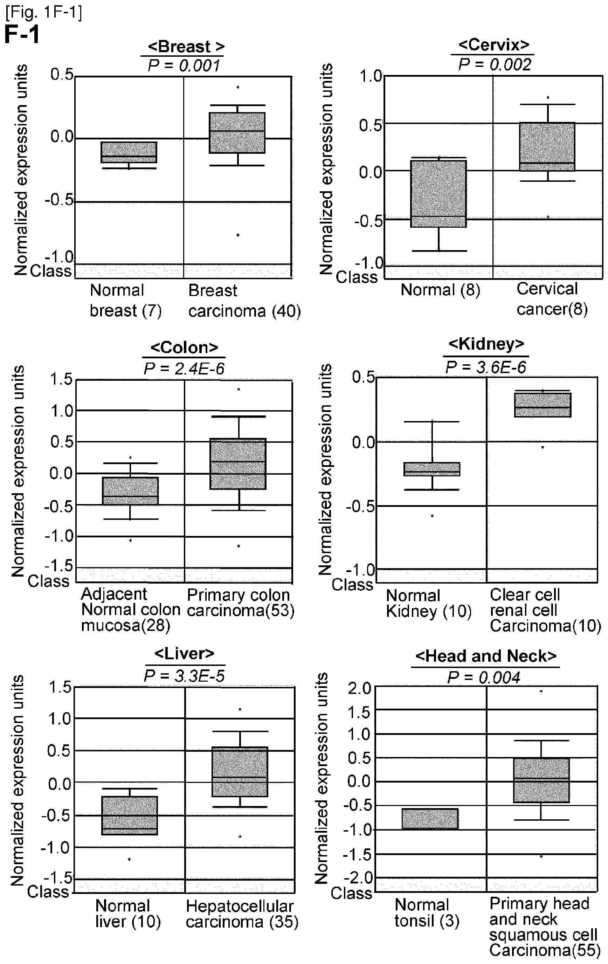

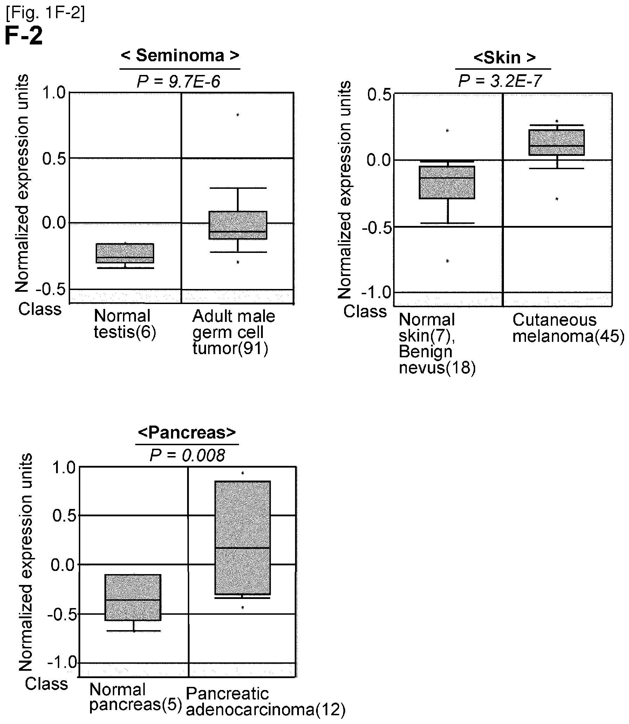

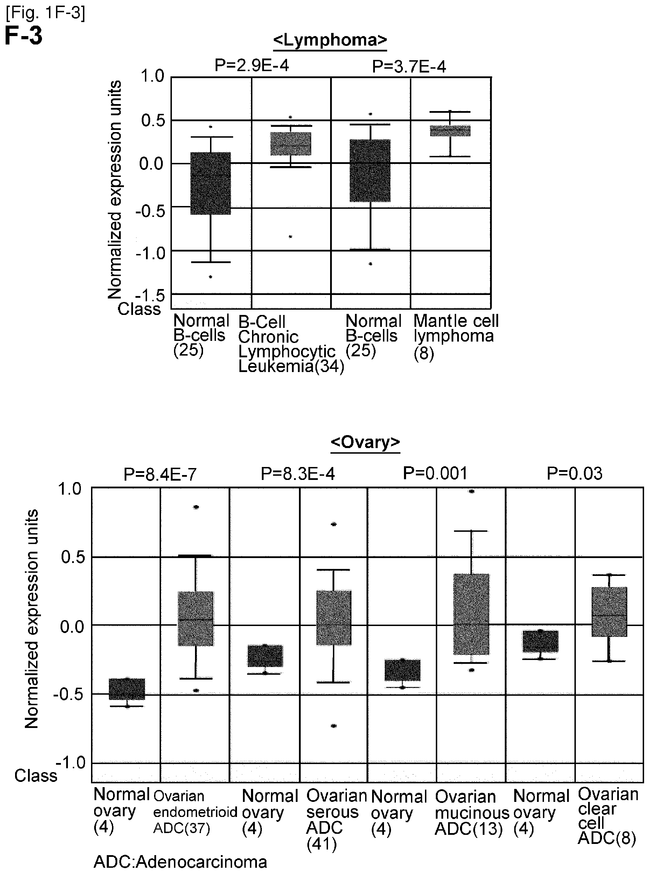

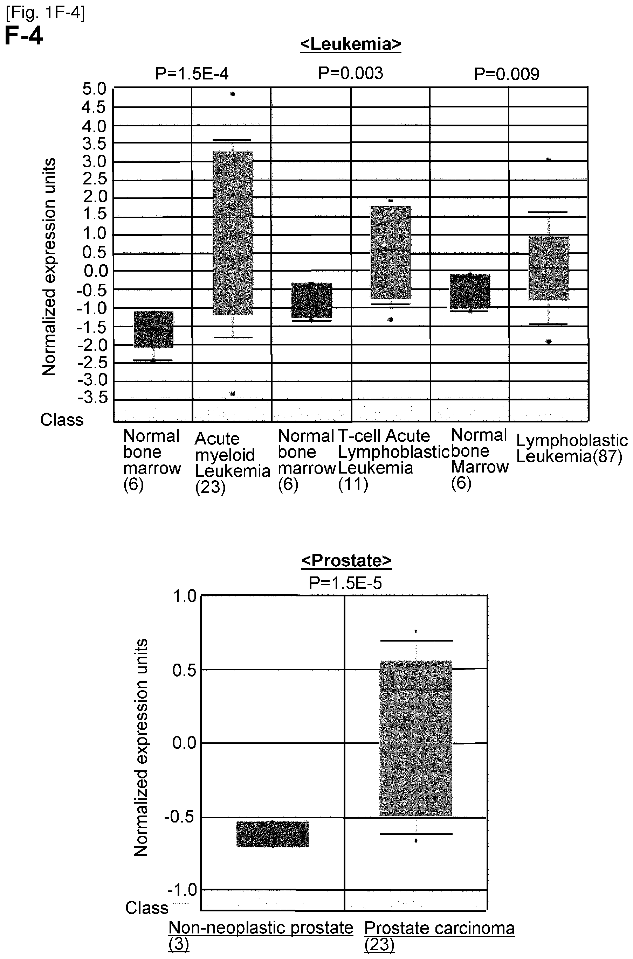

[fig.1A-C] Figure 1 demonstrates that SMYD2 is over-expressed in cancer tissues and cells. Part A depicts the expression analysis of SMYD2 at mRNA levels in 125 bladder cancer cases and 28 normal bladder cases by qRT-PCR. The result is shown by box-whisker plot. GAPDH and SDH were used as housekeeping genes. The Mann-Whitney U-test was used for statistical analysis (P < 0.0001). Part B depicts the comparison of mRNA levels of SMYD2 between bladder cancer samples and normal organ tissues. The normal organ tissues include brain, breast, colon, esophagus, eye, heart, liver, lung, pancreas, placenta, kidney, rectum, spleen, stomach and testis. Part C depicts the immunohistochemical analysis of bladder cancer and normal bladder tissues. All tissue samples were purchased from BioChain. Original magnification: x 200.

[fig.1D-E]Part D depicts the results of qRT-PCR analysis to examine expression levels of SMYD2 at the mRNA level in 1 non-cancerous cell lines (WI-38), 12 bladder cancer cell lines (SW780, J82, RT4, UMUC3, HT1197, HT1376, 5637, EJ28, T24, 253J, 253JBV and SCaBER), 5 lung cancer cell lines (RERF-LC-AI, LC319, H2170, A549 and SBC5), 2 colon cancer cell lines (LoVo and HCT116) and one liver cancer cell line (SNU475). Part E depicts the expression levels of SMYD2 at the protein level in various types of cell lines. Lysates from the colonic fibroblast cell line CCD-18Co, two bladder cancer cell lines (RT4 and SW780), two lung cancer cell lines (A549 and SBC5), one colon cancer cell line (HCT116) and one cervical cancer cell line (HeLa) were immunoblotted with anti-SMTD2 and anti-ACTB (an internal control) antibodies.

[fig.1F-1]Part F depicts the analysis of gene expression data in Oncomine. The thick bar in the boxes are average expression levels and the boxes represent 95% of the samples. The error bars are above or below the boxes, and the range of expression levels is enclosed by two dots.

[fig.1F-2]Fig. 1F-2 is a continuation of Fig. 1F-1.

[fig.1F-3]Fig. 1F-3 is a continuation of Fig. 1F-2.

[fig.1F-4]Fig. 1F-4 is a continuation of Fig. 1F-3.

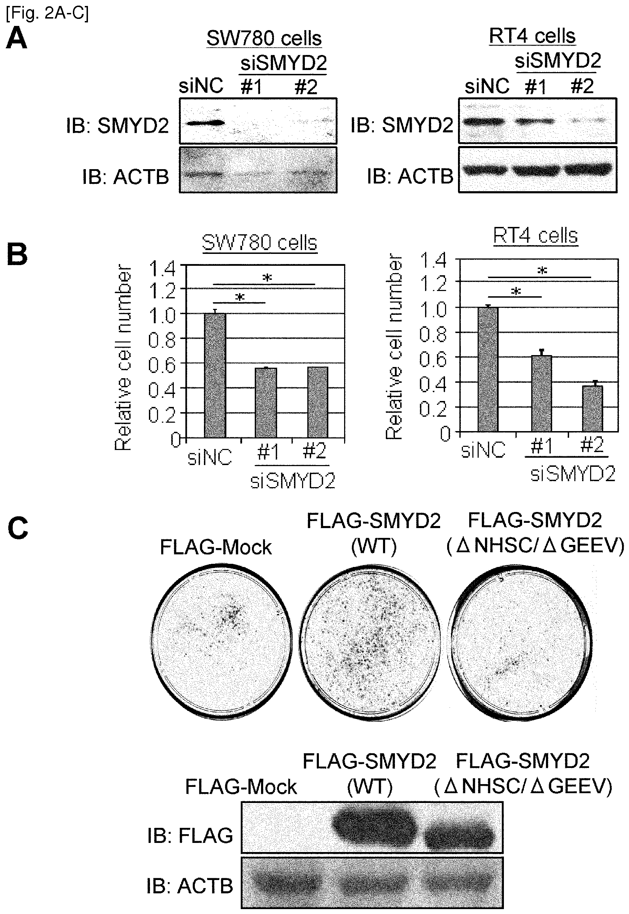

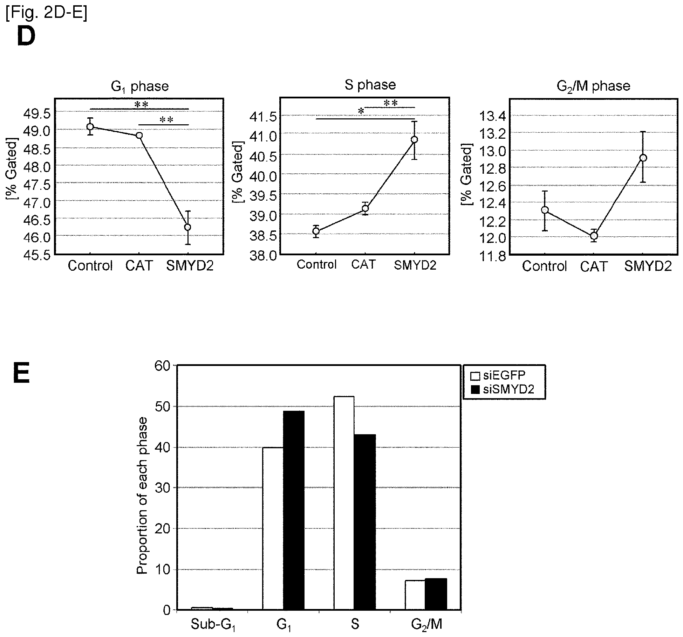

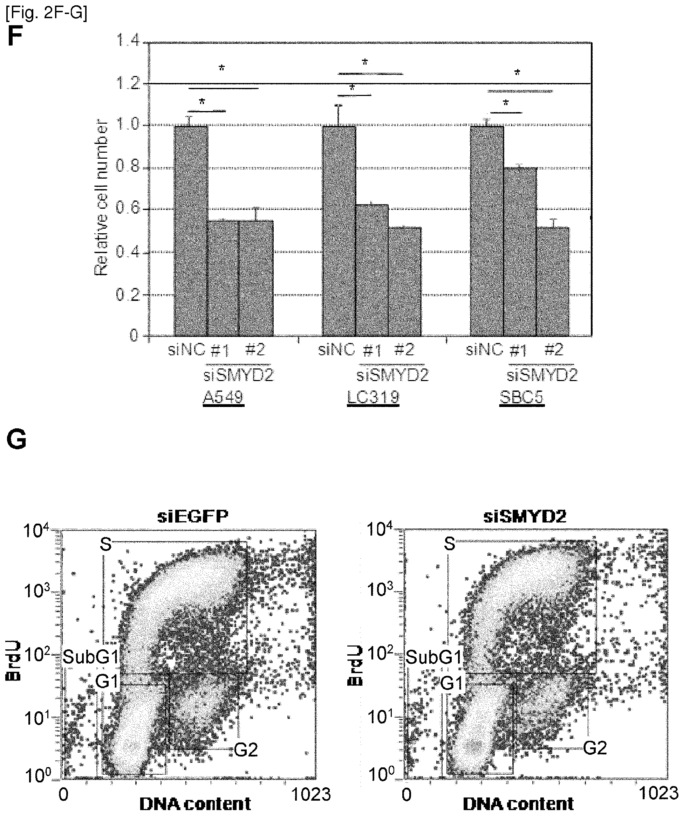

[fig.2A-C]Figure 2 demonstrates the involvement of SMYD2 in the growth of cancer cells. Part A depicts the validation of SMYD2 knockdown at the protein level. Lysates from SW780 and RT4 cells 72 hour after siRNA treatment were immunoblotted with anti-SMYD2 and anti-ACTB (an internal control) antibodies. Part B depicts the effects of SMYD2 knockdown on the proliferation of bladder cancer cell lines (SW780 and RT4) measured by Cell Counting Kit-8. Relative cell numbers are normalized to the number of siNC-treated cells (siNC = 1): results are the mean +/- SD (error bars)of three independent experiments. P-values were calculated using Student's t-test (*, P < 0.05). Part C depicts that a methylation activity of SMYD2 is critical for its growth promoting effect. COS7 cells transfected with FLAG-Mock, -SMYD2 (WT or delta-NHSC/delta-GEEV) and 10 days after transfection, Giemsa staining was performed. Expression of SMYD2 (WT or delta-NHSC/delta-GEEV) was confirmed by Western blot using anti-FLAG antibody. Expression of ACTB served as a control.

[fig.2D-E]Part D depicts that SMYD2 promotes the G₁/S transition of cell cycle. Numerical analysis of the FACS result, classifying cells by cell cycle status. The proportion of T-REx-SMYD2 cells in S phases is slightly higher than control cells (T-REx-Mock and T-REx-CAT). Mean +/- SD (error bars) of three independent experiments. Fisher's PLSD Post-Hoc test was used to calculate P-values (**, P < 0.01; *, P < 0.05). Part E depicts that cell cycle distribution analyzed by flow cytometry after coupled staining with fluorescein isothiocyanate (FITC)-conjugated anti-BrdU and 7-amino-actinomycin D (7-AAD) as described in Materials and Methods.

[fig.2F-G]Part F depicts the effects of SMYD2 knockdown on the proliferation of lung cancer cell lines. Relative cell numbers are measured by Cell Counting kit 8 and normalized to the number of siNC-treated cells (siNC = 1): results are the mean +/- SD of three independent experiments. P-values were calculated using Student's t-test (*, P<0.05). Part G depicts the effect of siSMYD2 on cell cycle kinetics in HeLa cells. Cell cycle distribution was analyzed by flow cytometry after coupled staining with fluorescein isothiocyanate (FITC)-conjugated anti-BrdU and 7-amino-actinomycin D (7-AAD) as described in Materials and Methods.

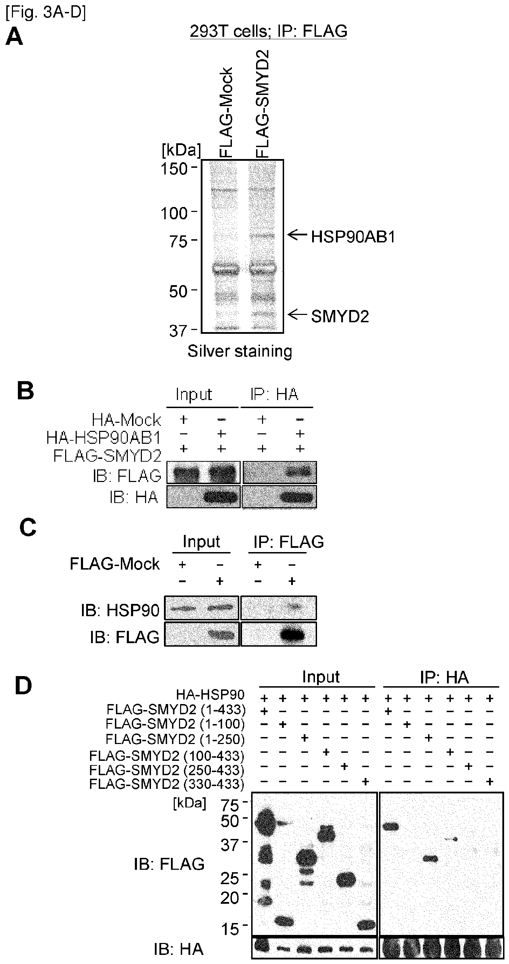

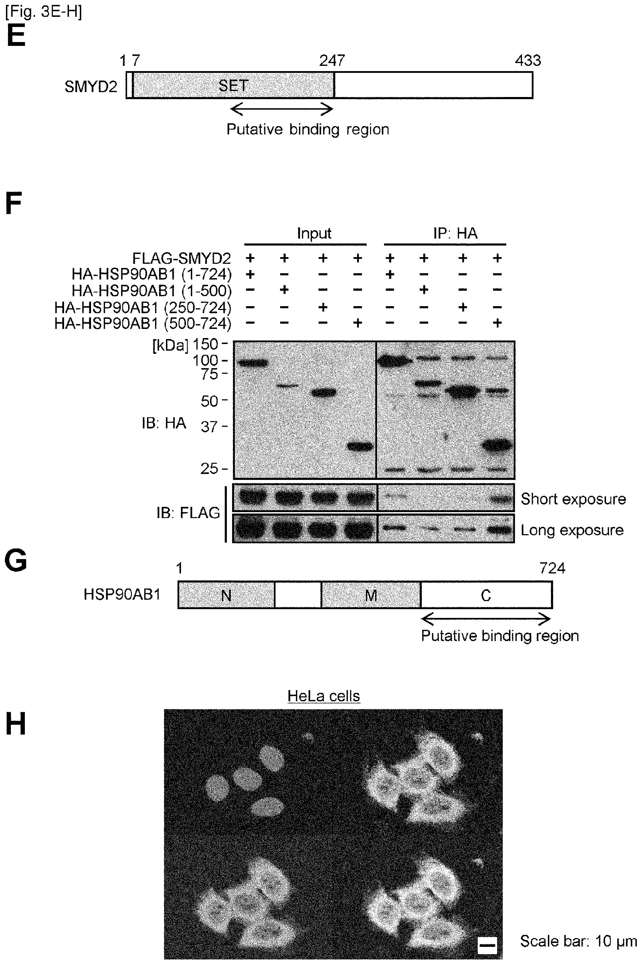

[fig.3A-D]Figure 3 demonstrates the SMYD2 forms a complex with HSP90AB1 in the cytoplasm. Part A depicts the silver staining of immunoprecipitates from FLAG-mock or FLAG-SMYD2 expressing cells. 293T cells were transfected with FLAG-Mock or FLAG-SMYD2 and immunoprecipitated using an anti-FLAG M2 agarose. Immunoprecipitates were subject to SDS-PAGE and silver staining, followed by mass spectrometry. Part B depicts that 293T cells were co-transfected with HA-HSP90AB1 or HA-Mock and FLAG-SMYD2 expression vectors, and HA-immunoprecipitates were immunoblotted with anti-FLAG and anti-HA antibodies. Part C depicts the interaction of FLAG-SMYD2 with endogenous HSP90. The interaction was confirmed by Western blot of FLAG-immunoprecipitates using anti-HSP90 and anti-FLAG antibodies. Part D depicts that the region included the SET domain of SMYD2 is required for interaction with HSP90AB 1. 293T cells were co-transfected with an HA-HSP90AB1 expression vector and six different lengths of FLAG-SMYD2 expression vectors ([1-433], [1-100], [1-250], [100-433], [250-433] and [330-433]). Immunoprecipitation was performed using anti-HA agarose beads, and samples were immunoblotted with anti-FLAG and anti-HA antibodies.

[fig.3E-H]Part E depicts the schematic representation of a binding region of SMYD2 to HSP90AB 1. Part F depicts that the C-terminal region of HSP90AB1 is required for binding to SMYD2. 293T cells were co-transfected with a FLAG-SMYD2 expression vector and four different lengths of HA-HSP90AB1 vectors ([1-724], [1-500], [250-724] and [500-724]). HA-immunoprecipitates were immunoblotted with anti-FLAG and anti-HA antibodies. Part G depicts the schematic representation of a binding region of HSP90AB1 to SMYD2. Part H depicts co-localization of SMYD2 and HSP90AB1 in HeLa cells. HeLa cells were stained with anti-SMYD2 (Alexa Fluor (registered trademark) 488) and anti-HSP90 (Alexa Fluor^{(registered trademark)} 594) antibodies, and DAPI. Scale bar denotes 10 micrometer.

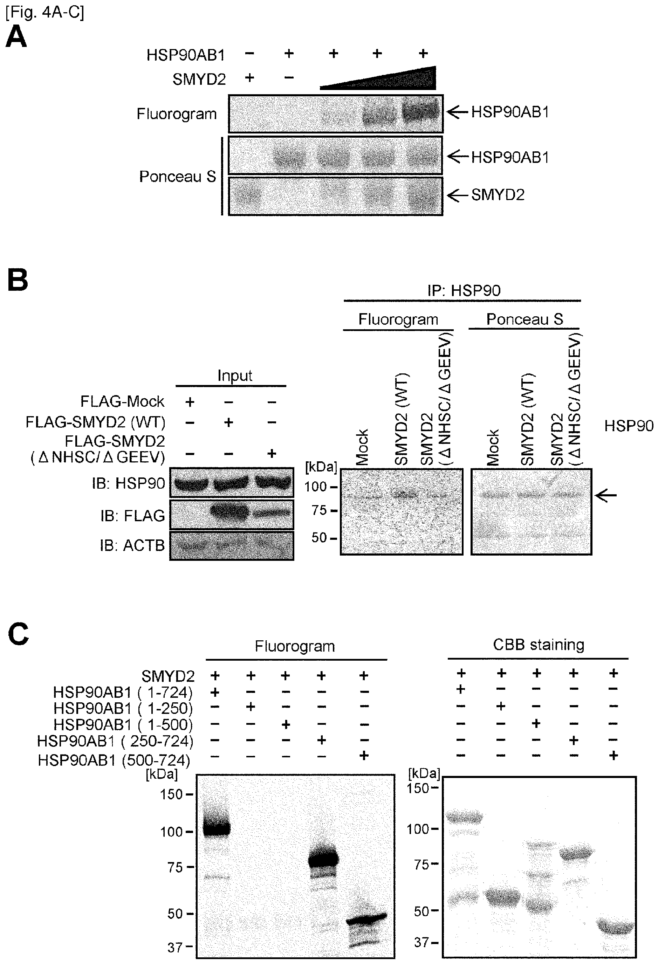

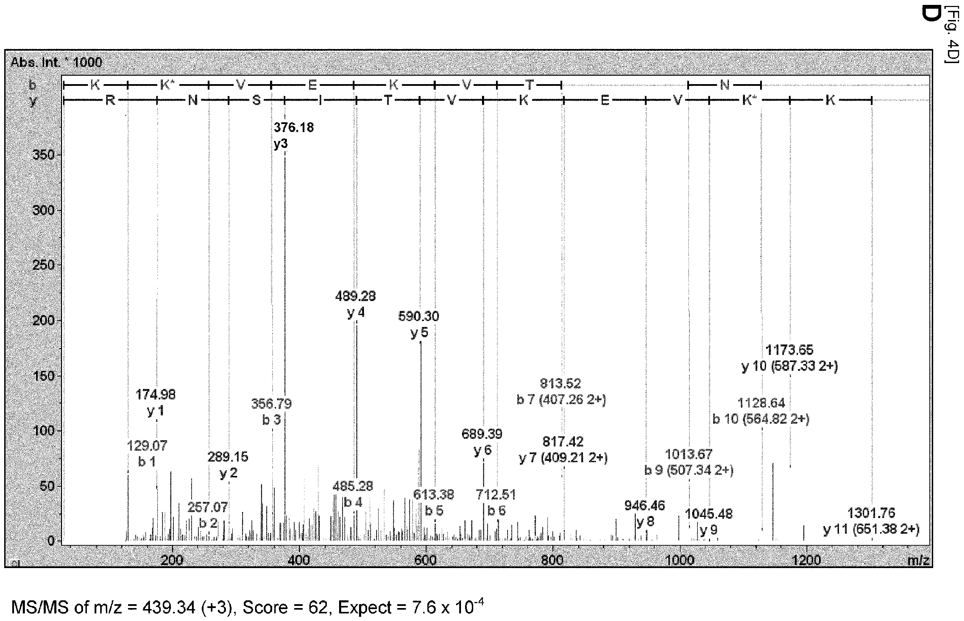

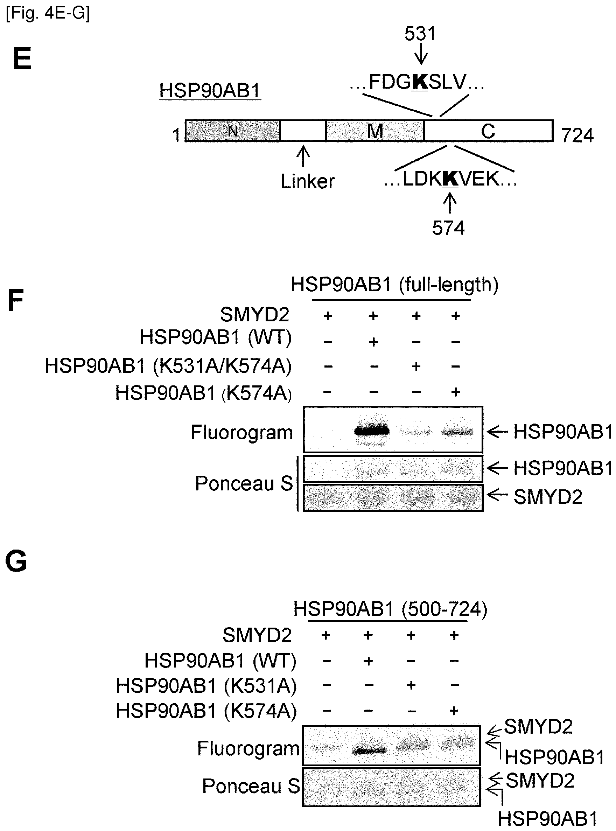

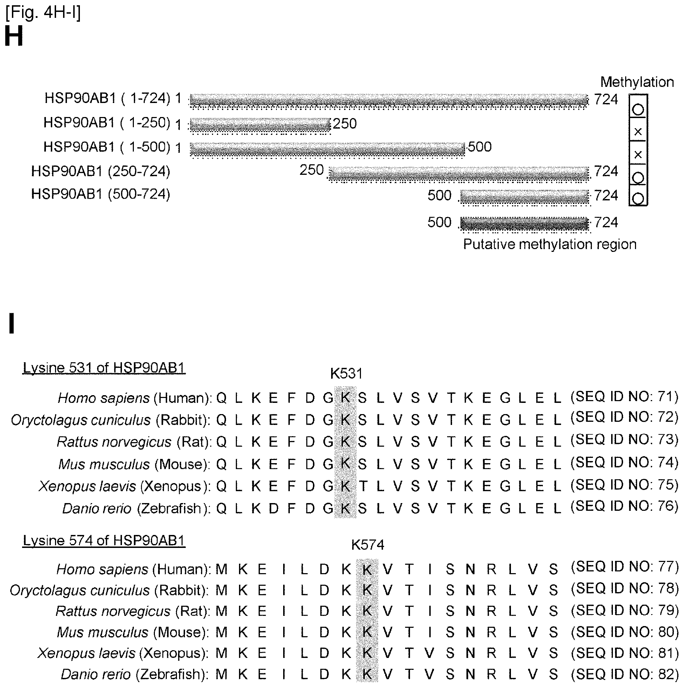

[fig.4A-C]Figure 4 demonstrates that SMYD2 methylates HSP90AB1 at K531 and K574. Part A depicts the methylation of HSP90AB1 by SMYD2 in a dose-dependent manner. In vitro methyltransferase reaction was performed using purified His-HSP90AB1 and His-SMYD2 recombinant proteins, and methylated HSP90AB1 was visualized with fluorography. Amounts of loading proteins were confirmed by staining the membrane with Ponceau S. Part B depicts the methylation of HSP90 in human cells by in vivo labelling experiment. 293T cells were transfected with FLAG-Mock, FLAG-SMYD2 (WT) or FLAG-SMYD2 (delta-NHSC/ delta-GEEV) expression vectors and treated with methionine-free medium, including cycloheximide and chloramphenicol. They were then labeled with L-[methyl-³H] methionine for 5 hours. Cell lysates were immunoprecipitated with an anti-HSP90 antibody, and methylated HSP90 was visualized by fluorography. The membrane was stained with Ponceau S and whole cell lysates were immunoblotted with anti-HSP90, anti-FLAG and anti-ACTB (an internal control) antibodies. Part C depicts that C-terminal region of HSP90AB 1 (500-724) was methylated by SMYD2. In vitro methyltransferase assay was performed using five different lengths of GST-HSP90AB1 and His-SMYD2. Methylation activity was visualized by fluorography and CBB staining was conducted to confirm amounts of proteins.

[fig.4D]Part D depicts the MS/MS spectrum corresponding to the mono-methylated HSP90AB1 peptide. The 14 Da increase of the Lys 594 residue was observed, demonstrating the mono-methylated Lys 594. Score and Expect show Mascot Ion Score and Expectation value in Mascot Database search results, respectively.

[fig.4E-G]Part E depicts the schematic representation of methylation sites of HSP90AB1. Part F depicts the results of in vitro methyltransferase assay using His-SMYD2 and full-length His-HSP90AB1 (WT, K531A/K574A and K574A). His-SMYD2 and full-length His-HSP90AB1 (WT, K531A/K574A and K574A) were reacted in the presence of S-adenosyl-_{L}-[methyl-³H] methionine, and the mixture was subjected to SDS-PAGE and visualized by fluorogram. The membrane was stained with Ponceau S. Part G depicts the results of in vitro methyltransferase assay using His-SMYD2 and partial His-HSP90AB1 [500-724] (WT, K531A and K574A). His-SMYD2 and partial His-HSP90AB1 [500-724] (WT, K531A and K574A) were reacted in the presence of S-adenosyl-_{L}-[methyl-³H] methionine, and the mixture was subjected to SDS-PAGE and visualized by fluorogram. The membrane was stained with Ponceau S.

[fig.4H-I]Part H depicts the schematic representation of HSP90AB1 methylated by SMYD2. 500-724 part of HSP90AB1 is a putative methylated region by SMYD2. Part I depicts the amino acid sequences of HSP90AB 1. Lysines 531 and 574 are conserved across various species, including human (Homo sapiens), rabbit (Oryctolagus cuniculus), rat (Rattus norvegicus), mouse (Mus musculus), xenopus (Xenopus laevis) and zebrafish (Danio retio).

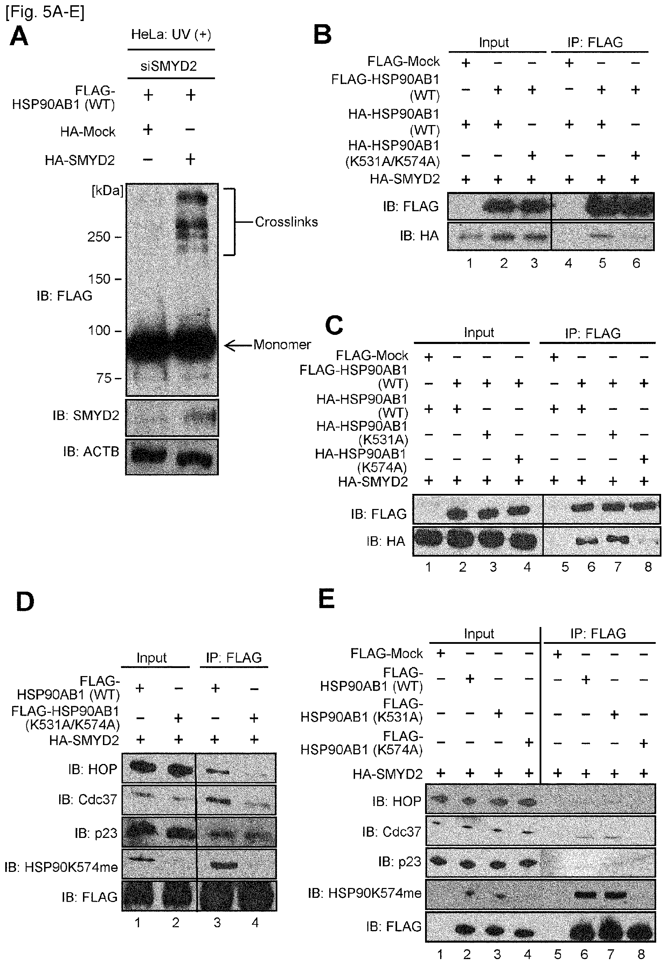

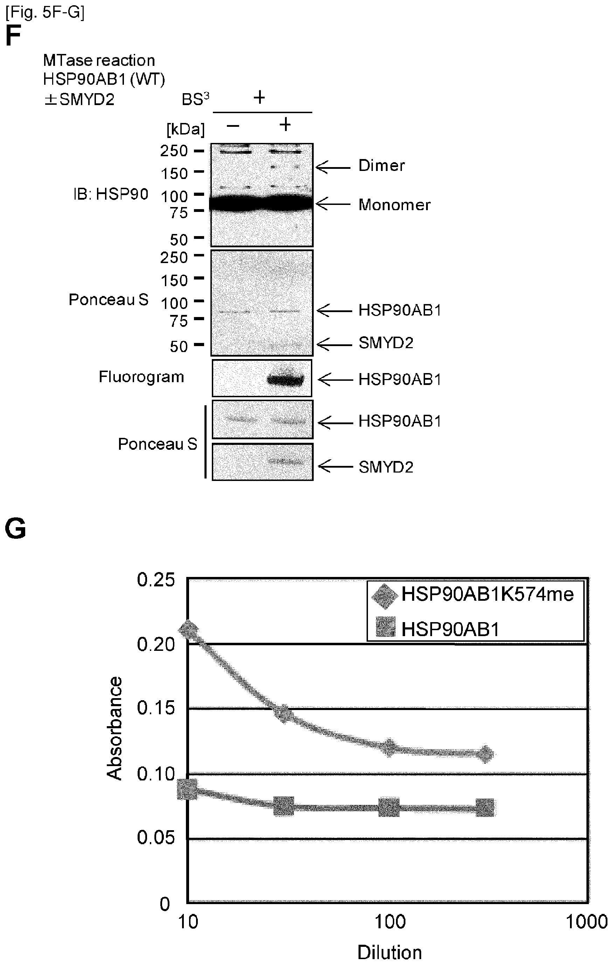

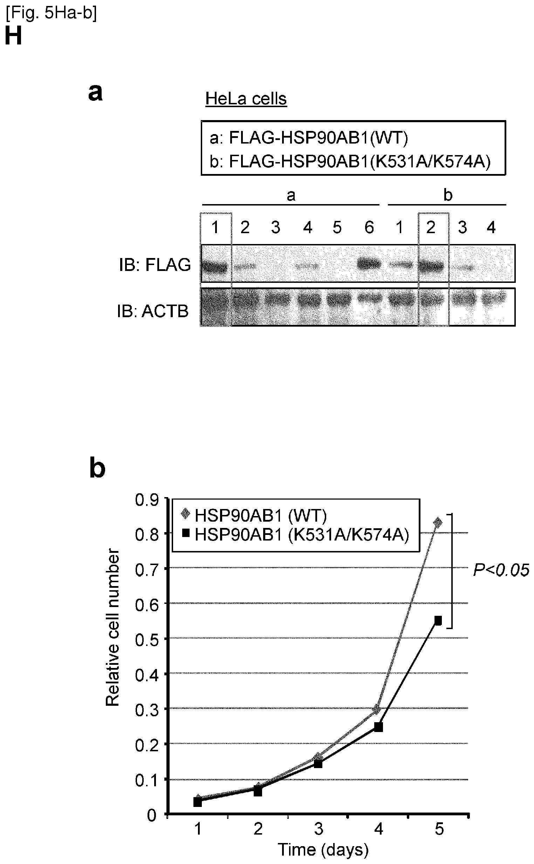

[fig.5A-E]Figure 5 demonstrates the methylation of K574 is vital for formation of the HSP90AB1 chaperonin complex. Part A depicts an in vivo cross-linking assay showing methylation-dependent cross-linking of HSP90AB1. HeLa cells were treated with siSMYD2#2 and 24 hours after siRNA treatment, the cells were transfected with FLAG-HSP90AB1 (WT) and HA-Mock or HA-SMYD2 expression vectors, followed by UV irradiation in the presence of DMEM-LM containing L-Photo-Leucine and L-Photo-Methionine. Then, cell lysates were immunoblotted with anti-FLAG, anti-SMYD2 and anti-ACTB (an internal control) antibodies. Part B depicts the results of immunoprecipitation analysis using 293T cells co-transfected with FLAG-Mock or FLAG-HSP90AB1 (WT) and HA-HSP90AB1 (WT or K531A/K574A) expression vectors in the presence of an HA-SMYD2 expression vector. Immunoprecipitation was performed using anti-FLAG^{(registered trademark)} M2 agarose beads, and samples were immunoblotted with anti-FLAG and anti-HA antibodies. Part C depicts the results of immunoprecipitation analysis using 293T cells co-transfected with FLAG-Mock or FLAG-HSP90AB1 (WT) and HA-HSP90AB1 (WT, K531A or K574A) expression vectors in the presence of an HA-SMYD2 expression vector. Immunoprecipitation was performed using anti-FLAG^{(registered trademark)} M2 agarose beads, and samples were immunoblotted with anti-FLAG and anti-HA antibodies. Part D and E depict that the methylation of K574 on HSP90AB1 is important for binding to some co-chaperones. 293T cells were transfected with FLAG-HSP90AB1 (WT) or FLAG-HSP90AB1 (K531A/K574A) (D), FLAG-Mock, FLAG-HSP90AB1 (WT), FLAG-HSP90AB1 (K531A) or FLAG-HSP90AB1 (K574A) (E) expression vectors, in the presence of an HA-SMYD2 expression vector. Immunoprecipitation was performed anti-FLAG^{(registered trademark)} M2 agarose beads and samples were immunoblotted with anti-HOP, anti-Cdc37, anti-p23, anti-HSP90meK574me1 and anti-FLAG antibodies.

[fig.5F-G]Part F depicts the promotion of HSP90AB1 dimerization by SMYD2-dependent methylation. After in vitro methyltransferase reaction of HSP90AB1 in the presence or absence of SMYD2, HSP90AB1 was cross-linked by BS, followed by SDS-PAGE and western blotting using an anti-HSP90 antibody. Methylation activity was validated by Fluorogram, and membranes were stained with Ponceau S to visualize amounts of loading proteins. Part G depicts the determination of the titer and specificity of an anti-mono-methylated HSP90AB1K574me antibody analyzed by ELISA.

[fig.5Ha-b]Part H depicts that the methylation of HSP90AB1 promotes cancer cell growth. Part Ha depicts the validation of HSP90AB1 (WT or K531A/K574A) expression in HeLa cells stably expressing FLAG-HSP90AB1 (WT or K531A/K574A). HeLa cells stably expressing FLAG-HSP90 (WT or K531A/K574A) was constructed and lysates were immunoblotted with anti-FLAG and anti-ACTB (an internal control) antibodies. Part Hb depicts that the result of the cell growth assay performed every 24 hours using Cell Counting kit 8. Relative cell numbers are normalized to the number of the cells expressing HSP90AB1 (WT): results are the mean +/- SD of three independent experiments. P values were calculated using Student's t-test (*, P < 0.05).

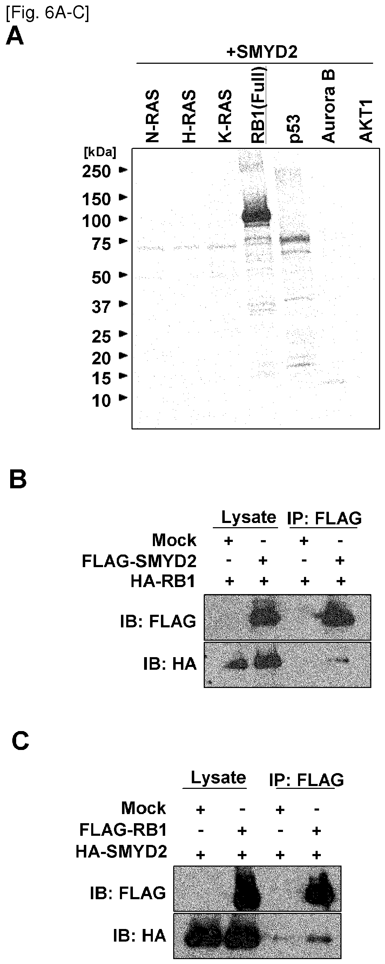

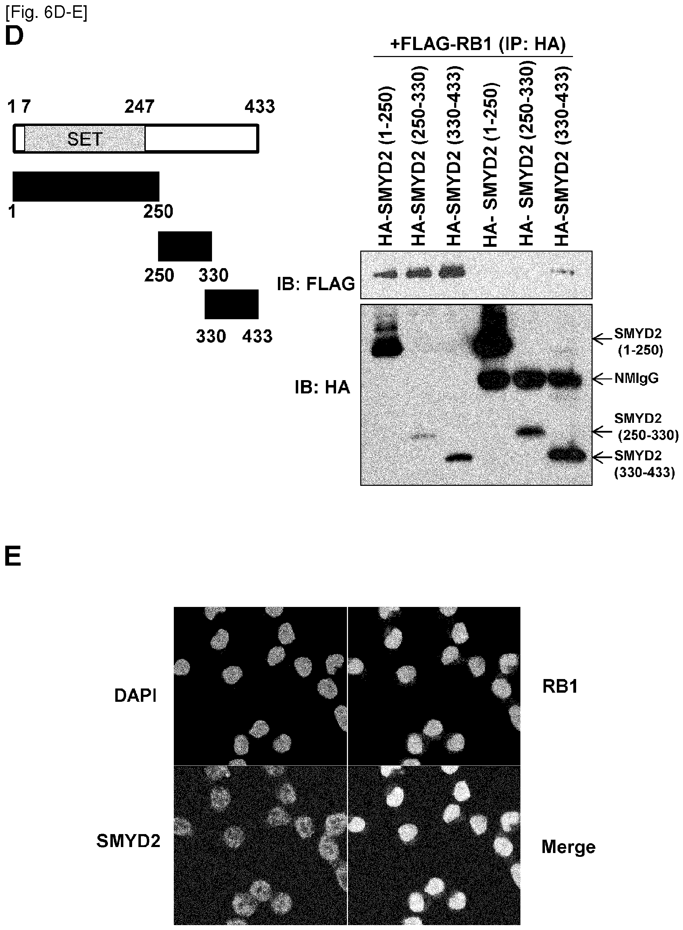

[fig.6A-C]Figure 6 demonstrates SMYD2 methylates RB1 and makes a complex through its C-terminal domain. Part A demonstrates that RB1 is methylated by SMYD2. In vitro methyltrasnferase reaction was performed using purified N-RAS, H-RAS, K-RAS, RB1, p53, Aurora B and AKT1 recombinant proteins. Methylated proteins were visualized with fluorography. Part B and C depict the results of Co-immunoprecipitation assays of SMYD2 and RB1 proteins. 293T cells were co-transfected with a SMYD2 expression vector and a RB 1 expression vector or a mock control vector. The interaction of FLAG-SMYD2 and HA-RB1 (B) or FLAG-RB1 and HA-SMYD2 (C) was examined by immunoprecipitation using anti-FLAG M2 agarose and immunoblotted with anti-FLAG and anti-HA antibodies.

[fig.6D-E]Part D demonstrates that the C-terminal region of SMYD2 is essential for the interaction with RB1. 293T cells were co-transfected with a FLAG-RB1 expression vector and three different regions of HA-SMYD2 vectors (amino acids 1-250, 250-330 and 320-433 in SMYD2 protein). Immunoprecipitation was performed using FLAG-M2 agarose and samples were immunoblotted with anti-FLAG and -HA antibodies. Part E demonstrates co-localization of SMYD2 and RB1 proteins in SBC5 cells. SBC5 cells were stained with anti-RB1 (Alexa Fluor^{(registered trademark)} 488) and anti-SMYD2 (Alexa Fluor^{(registered trademark)} 594) antibodies, and DAPI). Scale bar denotes 30 micrometers.

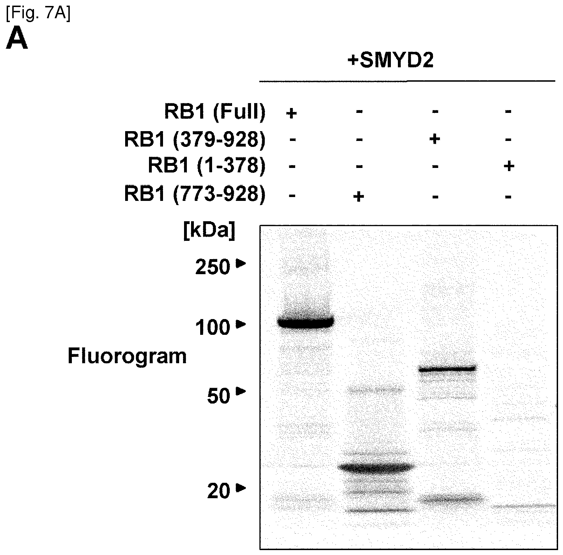

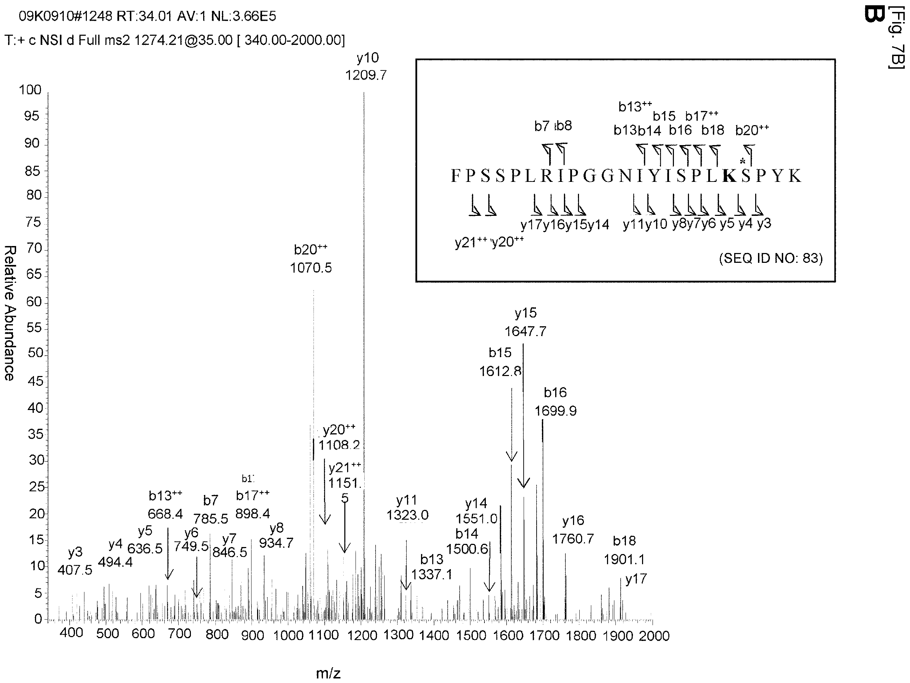

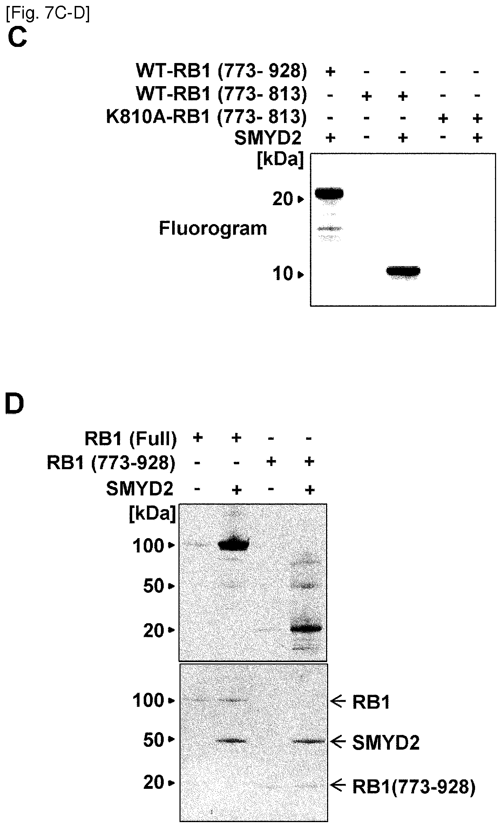

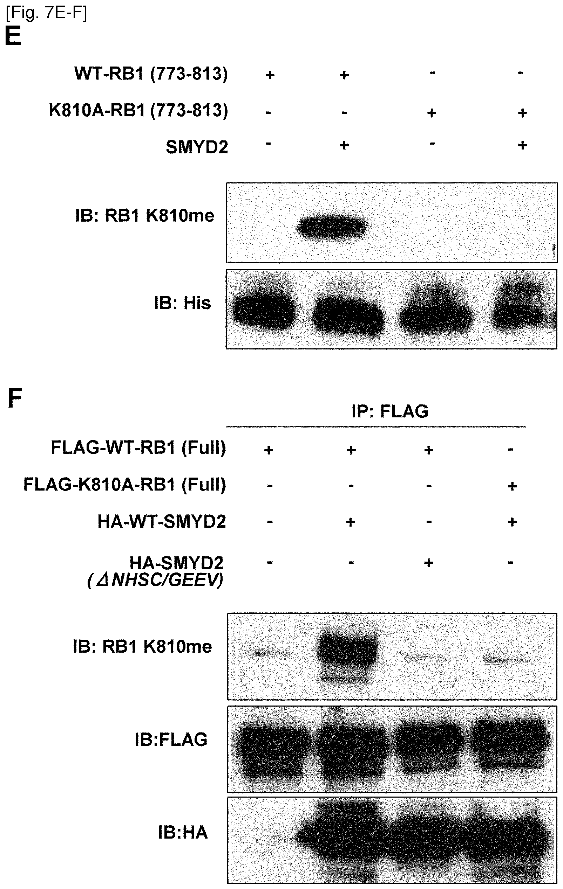

[fig.7A]Figure 7 demonstrates that SMYD2 methylates RB1 at K810. Part A demonstrates that the C-terminal region of RB 1 is methylated by SMYD2. In vitro methyltransferase assay was performed using purified RB 1 recombinant proteins [RB 1 (Full), RB1 (1-378), RB1 (379-928) and RB1 (773-928)], and methylated proteins were visualized with fluorography.

[fig.7B]Part B depicts the MS/MS spectrum of monomethyl-peptide of RB 1. RB1 protein (773-928) was treated with SMYD2 and then the mixture was subjected to SDS-PAGE. After CBB staining, a protein band of -25 kDa was digested with API and subjected to LC-MS/MS. A spectrum for the monomethylated RB1 is shown. The *K indicates monomethyl lysine.

[fig.7C-D]Part C demonstrates that K810A-RB1 is not methylated by SMYD2. In vitro methyltransferase assay was performed using RB1(773-928, 773-813), K810A-RB1(773-813). Part D and E depict the validation of the anti-K810me RB1 antibody. In vitro methyltransferase assay was conducted with RB 1 (Full) and RB 1 (773-928) (D) or RB1 (773-813) and K810A-RB1 (773-813) (E). The samples were immunoblotted with anti-RB1K810me and anti-His (internal control) antibodies.

[fig.7E-F]Part E depicts the validation of the anti-K810me RB1 antibody. In vitro methyltransferase assay was conducted with RB1 (773-813) and K810A-RB1 (773-813). Part F depicts that 293T cells were co-transfected with a FLAG-WT-RB1 vector or a FLAG-K810A-RB1 vector and an HA-WT-SMYD2 vector or an HA-SMYD2 (delta-NHSC/GEEV) vector. Immunoprecipitation was performed using anti-FLAG M2 agarose and the samples were immunoblotted with anti-RB1K810me, anti-FLAG and anti-HA antibodies.

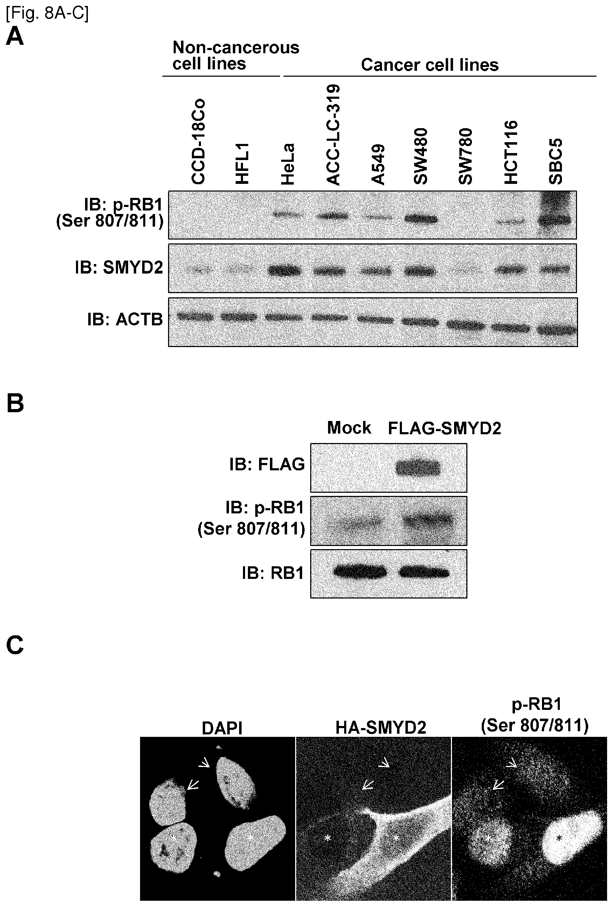

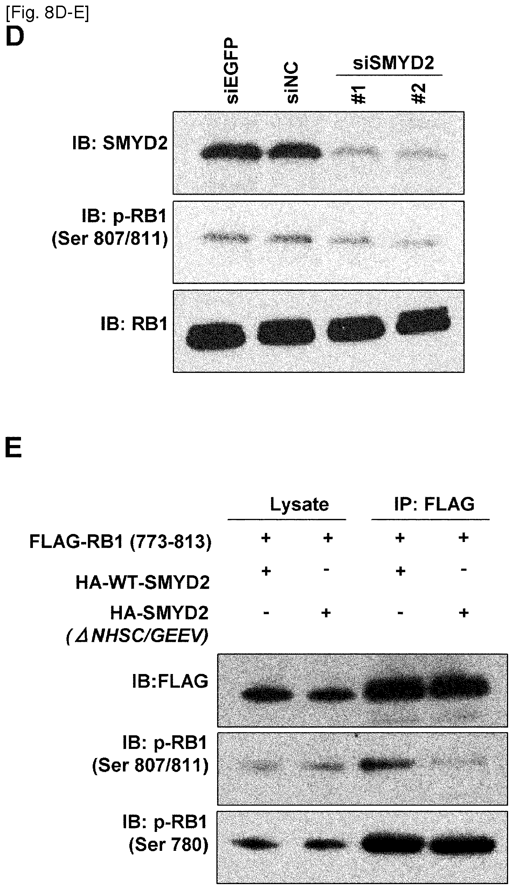

[fig.8A-C]Figure 8 demonstrates the enhancement of RB1 phosphorylation by SMYD2. Part A depicts the expression levels of SMYD2 correlate with phosphorylation levels of RB1 (Ser 807/811). Lysates from normal cell lines (CCD18Co and HFL1) and cancer cell lines (HeLa, ACC-LC-319, A549, SW480, SW780, HCT116 and SBC5) were immunoblotted with anti-p-RB1 (Ser 807/811), anti-SMYD2 and anti-ACTB (internal control) antibodies. Part B depicts that 293T cells were transfected with a FLAG-SMYD2 vector and a mock vector (negative control). Cells were lysed with RIPA like buffer containing complete protease inhibitor cocktail, and samples were immunoblotted with anti-FLAG, anti-phospho-RB1 (Ser 807/811) and anti-RB1 (internal control) antibodies. Part C depicts the results of immunocyto-chemical analysis in HeLa cells transfected with an HA-SMYD2 vector. After transfection with an HA-SMYD2 vector into HeLa cells, cells were fixed with 4% paraformaldehyde (PFA) and permeabilized with 0.5% Triton X-100. The fixed cells were stained with anti-phospho-RB1 (Ser 807/811) (Alexa Fluor^{(registered trademark)}488) and anti-HA (Alexa Fluor^{(registered trademark)} 594) antibodies, and DAPI.

[fig.8D-E]Part D depicts that knockdown of SMYD2 diminishes phosphorylation levels of RB1 (Ser 807/811). After knockdown of SMYD2 using SMYD2 specific siRNAs, cells were lysed with RIPA like buffer containing complete protease inhibitor cocktail. Immunoblot was performed with anti-SMYD2, anti-phospho-RB1(Ser 807/811) and anti-RB1(internal control) antibodies. Part E depicts the results of immunoprecipitation analysis using 293T cells transfected with a FLAG-RB1 (773-813) vector and an HA-WT-SMYD2 vector and an HA-SMYD2 (delta-NHSC/GEEV) vector. Immunoprecipitation was conducted with anti-FLAG M2 agarose. Anti-FLAG, anti-phospho-RB1 (Ser 807/811) and anti-phospho-RB1 (Ser 780) antibodies were used for immunoblot analysis.

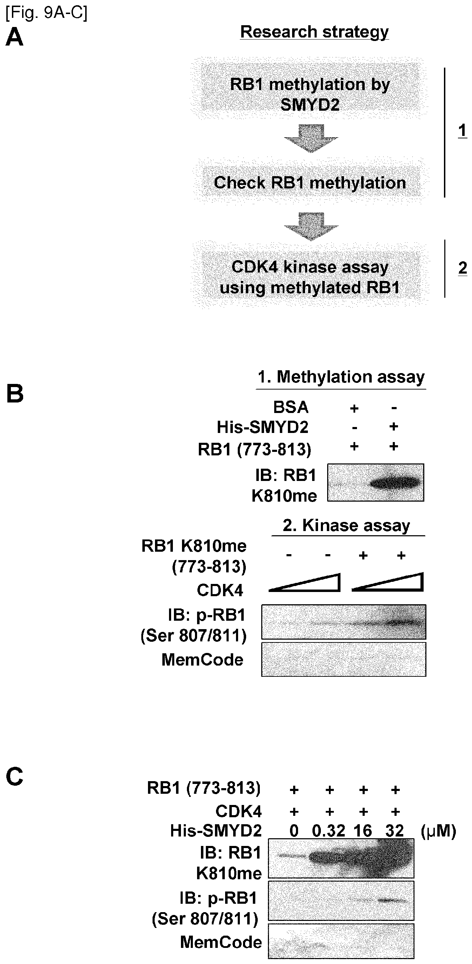

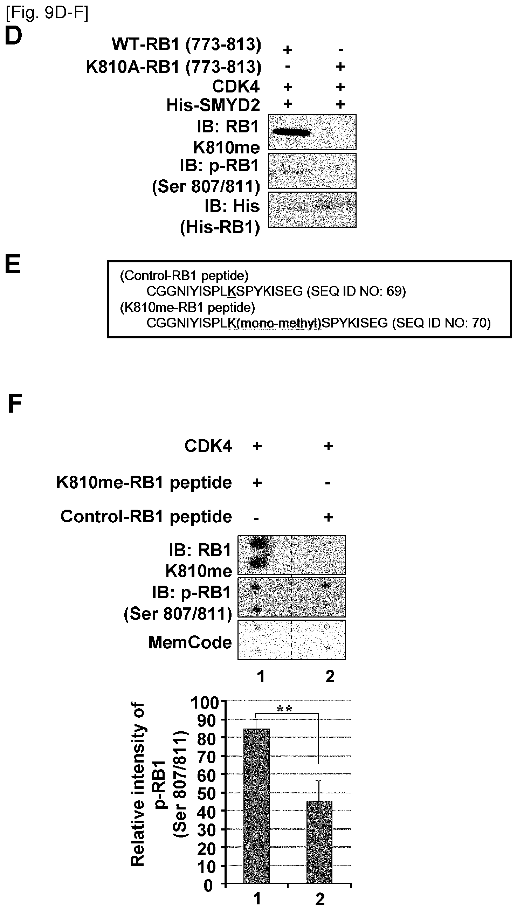

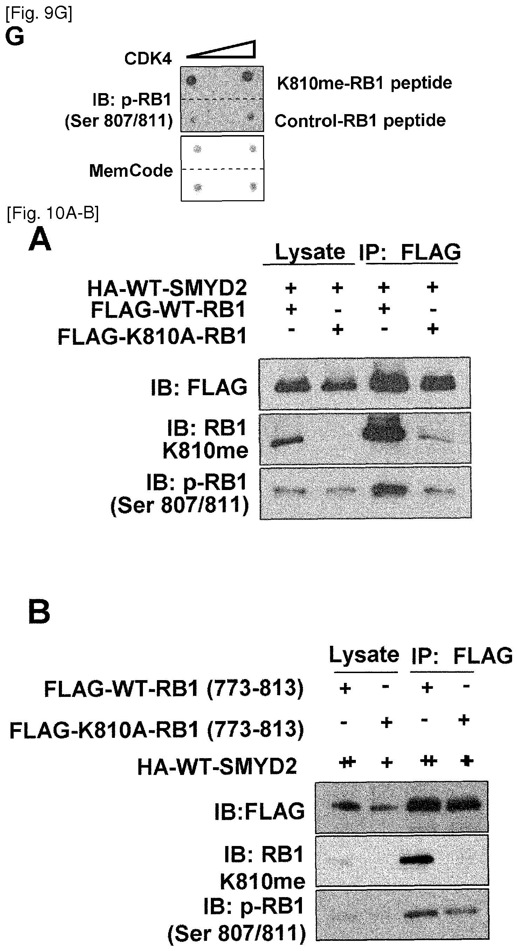

[fig.9A-C]Figure 9 demonstrates that SMYD2-dependent mono-methylation of RB1 at Lys 810 increases the phosphorylation of RB1 at Ser 807/811 in vitro. Part A depicts research strategy of sequential in vitro methylation and kinase assays. Part B depicts the results of in vitro methyltransferase assay performed using recombinant RB 1 (773-813) protein as a substrate reacted with BSA (negative control) or SMYD2 as an enzyme. After confirmation of RB 1 methylation by Western blot with anti-RB1K810me antibody, in vitro kinase assay was conducted using CDK4/Cyclin D1 complex as an enzyme. The samples were immunoblotted with an anti-phospho-RB 1 (Ser 807/811) antibody. Amounts of loading proteins and peptides were visualized by MemCode™ Reversible Protein Stain (Thermo Scientific). Part C depicts that methylation of RB1 at Lys 810 enhances phosphorylation levels of RB1 (Ser 807/811). After in vitro methyltransferase assay of RB1 treated with several different doses of SMYD2, in vitro kinase assay was performed with CDK4/Cyclin D1 complex as an enzyme. The samples were immunoblotted with anti-phopspho-RB1 (Ser 807/811) and anti-RB1 K810me antibodies. Amounts of loading proteins and peptides were visualized by MemCode™ Reversible Protein Stain (Thermo Scientific).

[fig.9D-F]Part D depicts the results of in vitro methyltransferase and kinase assays with WT-RB1 (773-813) and K810A-RB1 (773-813). Anti-RB1 K810me, anti-phospho-RB1 (Ser 807/811) and anti-His (internal control) antibodies were used for the immunoblot analysis. Part E demonstrates the sequences of methylated and unmethylated peptides of RB1. Part F depicts the results of in vitro kinase assay of K810 methylated or unmethylated RB1 peptides performed with CDK4/Cyclin D1 as an enzyme source. Anti-RB1 K810me and anti-phospho-RB 1 (Ser 807/811) antibodies were used for immunoblot analysis. Amounts of loading peptides were visualized by MemCode™ Reversible Protein Stain (Thermo Scientific). Mean +/- SD (error bars) of two independent experiments. P-values were calculated using Student's t-test (**, P < 0.01).

[fig.9G]Part G depicts the results of in vitro kinase assay of RB 1 peptides treated with two different doses of CDK4/Cyclin D1. Amounts of loading peptides were visualized by MemCode™ Reversible Protein Stain (Thermo Scientific).

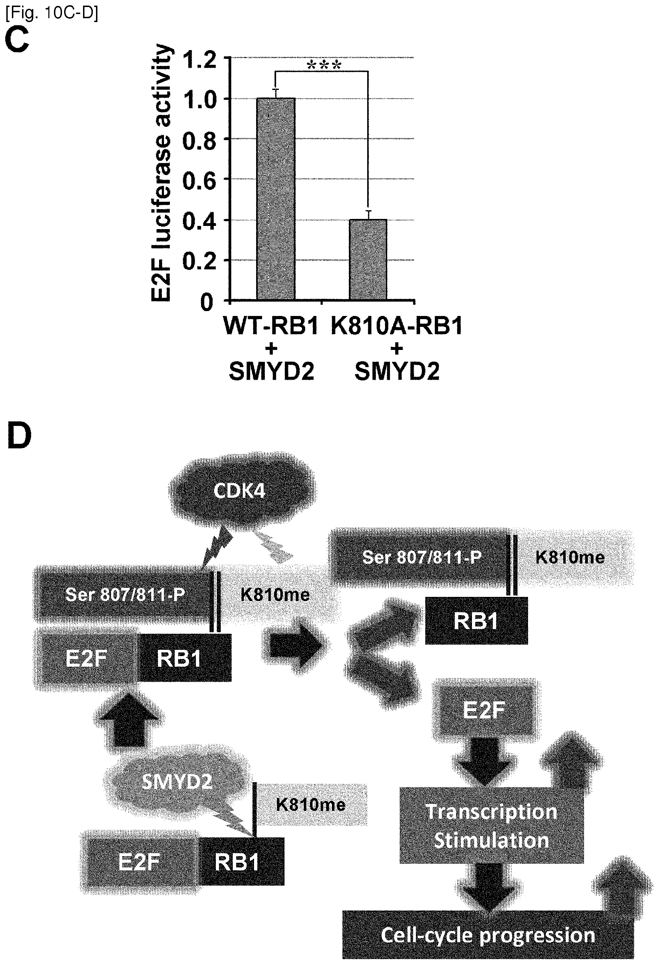

[fig.10A-B]Figure 10 demonstrates that Lys 810 methylation of RB 1 enhances the phosphorylation of RB 1 and E2F luciferase activity in vivo. Part A depicts the results of immunoprecipitation analysis using 293T cells transfected with a HA-WT-SMYD2 vector and a FLAG-WT-RB1 vector or a FLAG-K810A-RB vector. Immunoprecipitation was conducted with anti-FLAG M2 agarose. Anti-FLAG, anti-RB1 K810me and anti-phospho-RB1 (Ser 807/811) antibodies were used for immunoblot analysis. Part B depicts the results of immunoprecipitation analysis using 293T cells transfected with a FLAG-WT-RB1 (773-813) vector or a FLAG-K810A-RB1 (773-813) vector and a HA-WT-SMYD2 vector. Immunoprecipitation was conducted with anti-FLAG M2 agarose. Anti-FLAG, anti-RB1 K810me and anti-phospho-RB1 (Ser 807/811) antibodies were used for immunoblot analysis.

[fig.10C-D]Part C depicts the results of E2F reporter assay after over-expression of WT-RB1 and K810A-RB1 in 293T cells. Mean +/- SD (error bars) of three independent experiments. P-values were calculated using Student's t-test (***, P < 0.001). Part D depicts a schematic model for the dynamic regulation of RBI phosphorrylation through methylation of RBI by SMYD2.

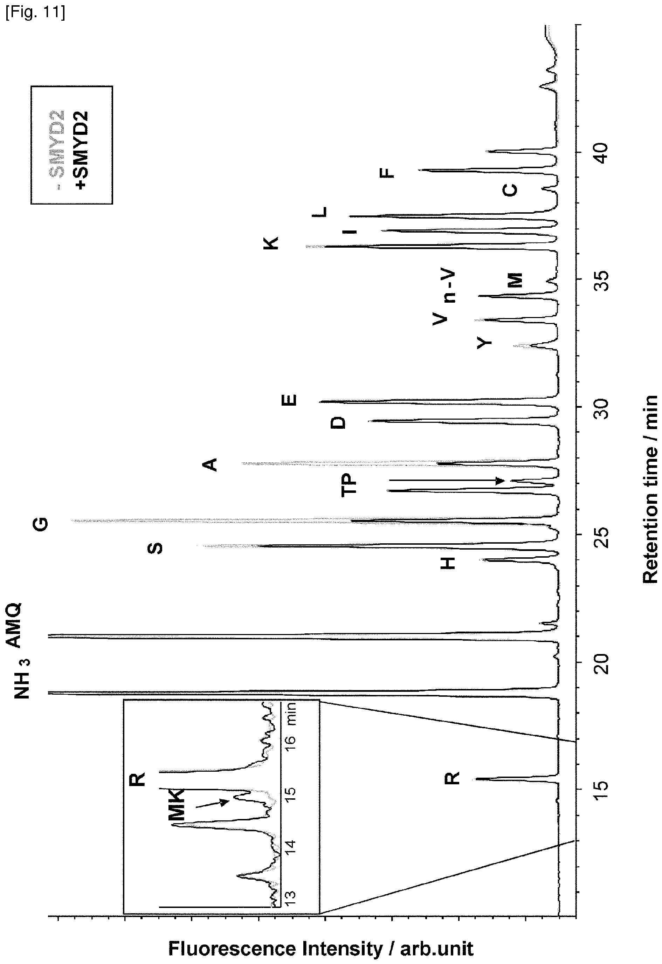

[fig. 11]Figurel 1 depicts the chromatogram of amino acids obtained by acid hydrolysis of RB1 after treatment with SMYD2 or without SMYD2. Inserted figure shows a magnified view of the region around the arginine. Except for mono-methylated lysine (MK) and norvaline (n-V), amino acid residues are annotated using their one-letter abbreviations. NH₃: ammonia, AMQ: 6-amino quinoline derived from hydrolysis of the derivatizing reagent for amino acids.

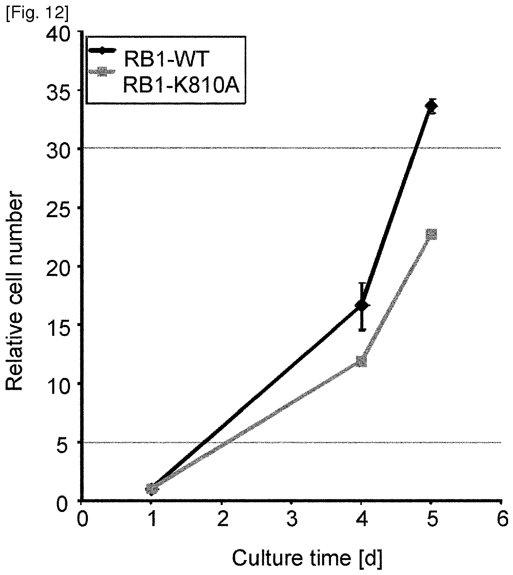

[fig. 12]Figure 12 depicts the results of the cell growth analysis of Flp-In T-REx 293 cell lines. The present inventors established stable cell lines, which can over-express wild-type RBI (RB1-WT) and K810-substituted RBI (RB1-K810A), using Flp-In™ T-REx™ system (Life technologies). Both wild-type and K810-substituted RBI proteins were induced by 1 microgram/ml doxycycline. The number of cells were calculated by Cell Counting Kit-8 (Dojindo), and the y-value shows the relative cell number to day 1 (dl = 1).

### Description of Embodiments

Although any methods and materials similar or equivalent to those described herein can be used in the practice or testing of embodiments of the present invention, the preferred methods, devices, and materials are now described. However, before the present materials and methods are described, it is to be understood that the present invention is not limited to the particular sizes, shapes, dimensions, materials, methodologies, protocols, etc. described herein, as these may vary in accordance with routine experimentation and optimization. It is also to be understood that the terminology used in the description is for the purpose of describing the particular versions or embodiments only, and is not intended to limit the scope of the present invention which will be limited only by the appended claims.

Nothing herein is to be construed as an admission that the invention is not entitled to antedate such disclosure by virtue of prior invention.

Unless otherwise defined, all technical and scientific terms used herein have the same meaning as commonly understood by one of ordinary skill in the art to which the present invention belongs. In case of conflict, the present specification, including definitions, will control. In addition, the materials, methods, and examples are illustrative only and not intended to be limiting.

### Definition

The words "a", "an", and "the" as used herein mean "at least one" unless otherwise specifically indicated.

The terms "gene", "polynucleotide", "oligonucleotide", "nucleic acid", and "nucleic acid molecule" are used interchangeably herein to refer to a polymer of nucleic acid residues and, unless otherwise specifically indicated are referred to by their commonly accepted single-letter codes. The terms apply to nucleic acid (nucleotide) polymers in which one or more nucleic acids are linked by ester bonding. The nucleic acid polymers may be composed of DNA, RNA or a combination thereof and encompass both naturally-occurring and non-naturally occurring nucleic acid polymers. The polynucleotide, oligonucleotide, nucleic acid, or nucleic acid molecule may be composed of DNA, RNA or a combination thereof.

The terms "polypeptide", "peptide", and "protein" are used interchangeably herein to refer to a polymer of amino acid residues. The terms refer to naturally occurring and synthetic amino acids, as well as amino acids analogs and amino acids mimetics amino acid polymers in which one or more amino acid residue is a modified residue, or a non-naturally occurring residue, such as an artificial chemical mimetic of a corresponding naturally occurring amino acid.

The term "amino acid" refers to naturally occurring and synthetic amino acids, as well as amino acid analogs and amino acid mimetics that similarly functions to the naturally occurring amino acids. Naturally occurring amino acids are those encoded by the genetic code, as well as those modified after translation in cells (e.g., hydroxyproline, gamma-carboxyglutamate, and O-phosphoserine). The phrase "amino acid analog" refers to compounds that have the same basic chemical structure (an alpha carbon bound to a hydrogen, a carboxy group, an amino group, and an R group) as a naturally occurring amino acid but have a modified R group or modified backbones (e.g., homoserine, norleucine, methionine, sulfoxide, methionine methyl sulfonium). The phrase "amino acid mimetic" refers to chemical compounds that have different structures but similar functions to general amino acids.

Amino acids may be referred to herein by their commonly known three letter symbols or the one-letter symbols recommended by the IUPAC-IUB Biochemical Nomenclature Commission.

In the context of the present invention, the phrase "SMYD2 gene", "HSP90AB 1 gene" or "RB1 gene" encompass polynucleotides that encode the human SMYD2 gene, HSP90AB1 gene or RB 1 gene or any of the functional equivalents of the human SMYD2 gene, HSP90AB1 gene or RB1 gene. The SMYD2 gene, HSP90AB1 gene or RB1 gene can be obtained from nature as naturally occurring polynucleotides via conventional cloning methods or through chemical synthesis based on the selected nucleotide sequence. Methods for cloning genes using cDNA libraries and such are well known in the art.

Unless otherwise defined, the term "cancer" refers to cancers over-expressing the SMYD2 gene. Examples of cancers over-expressing SMYD2 gene include, but are not limited to bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

Terms "isolated" and "purified" used in relation with a substance (e.g., polypeptide, antibody, polynucleotide, etc.) indicates that the substance is removed from its original environment (e.g., the natural environment if naturally occurring) and thus alternated from its natural state. Examples of isolated nucleic acids include DNA (such as cDNA), RNA (such as mRNA), and derivatives thereof that are substantially free of other cellular material, or culture medium when produced by recombinant techniques, or substantially free of chemical precursors or other chemicals when chemically synthesized. In a preferred embodiment, nucleic acid molecules encoding peptides of the present invention are isolated or purified.

In the context of the present invention, an "isolated" or "purified" polypeptide, polynucleotide, or antibody is substantially free from one or more contaminating substance that may else be included in the natural source. Thus, an isolated or purified antibody refers to antibodies that are substantially free of cellular material such as carbohydrate, lipid, or other contaminating proteins from the cell or tissue source from which the protein (antibody) is derived, or substantially free of chemical precursors or other chemicals when chemically synthesized. The term "substantially free of cellular material" includes preparations of a polypeptide in which the polypeptide is separated from cellular components of the cells from which it is isolated or recombinantly produced.

Thus, a polypeptide that is substantially free of cellular material includes preparations of polypeptide having less than about 30%, 20%, 10%, or 5% (by dry weight) of heterologous protein (also referred to herein as a "contaminating protein"). When the polypeptide is recombinantly produced, it is also preferably substantially free of culture medium, which includes preparations of polypeptide with culture medium less than about 20%, 10%, or 5% of the volume of the protein preparation. When the polypeptide is produced by chemical synthesis, it is preferably substantially free of chemical precursors or other chemicals, which includes preparations of polypeptide with chemical precursors or other chemicals involved in the synthesis of the protein less than about 30%, 20%, 10%, 5% (by dry weight) of the volume of the protein preparation. That a particular protein preparation contains an isolated or purified polypeptide can be shown, for example, by the appearance of a single band following sodium dodecyl sulfate (SDS)-polyacrylamide gel electrophoresis of the protein preparation and Coomassie Brilliant Blue staining or the like of the gel. In a preferred embodiment, antibodies and polypeptides of the present invention are isolated or purified. An "isolated" or "purified" nucleic acid molecule, such as a cDNA molecule, can be substantially free of other cellular material, or culture medium when produced by recombinant techniques, or substantially free of chemical precursors or other chemicals when chemically synthesized. In a preferred embodiment, nucleic acid molecules encoding antibodies of the present invention are isolated or purified.

As used herein, the term "biological sample" refers to a whole organism or a subset of its tissues, cells or component parts (e.g., body fluids, including but not limited to blood, mucus, lymphatic fluid, synovial fluid, cerebrospinal fluid, saliva, amniotic fluid, amniotic cord blood, urine, vaginal fluid and semen). "Biological sample" further refers to a homogenate, lysate, extract, cell culture or tissue culture prepared from a whole organism or a subset of its cells, tissues or component parts, or a fraction or portion thereof. Lastly, "biological sample" refers to a medium, such as a nutrient broth or gel in which an organism has been propagated, which contains cellular components, such as proteins or polynucleotides.

To the extent that the methods and compositions of the present invention find utility in the context of "prevention" and "prophylaxis", such terms are interchangeably used herein to refer to any activity that reduces the burden of mortality or morbidity from disease. Prevention and prophylaxis can occur "at primary, secondary and tertiary prevention levels". While primary prevention and prophylaxis avoid the development of a disease, secondary and tertiary levels of prevention and prophylaxis encompass activities aimed at the prevention and prophylaxis of the progression of a disease and the emergence of symptoms as well as reducing the negative impact of an already established disease by restoring function and reducing disease-related complications. Alternatively, prevention and prophylaxis can include a wide range of prophylactic therapies aimed at alleviating the severity of the particular disorder, e.g. reducing the proliferation and metastasis of tumors.

To the extent that certain embodiments of the present invention encompass the treatment and/or prophylaxis of cancer and/or the prevention of postoperative recurrence, such methods may include any of the following steps: the surgical removal of cancer cells, the inhibition of the growth of cancerous cells, the involution or regression of a tumor, the induction of remission and suppression of occurrence of cancer, the tumor regression, and the reduction or inhibition of metastasis. Effective treatment and/or the prophylaxis of cancer decreases mortality and improves the prognosis of individuals having cancer, decreases the levels of tumor markers in the blood, and alleviates detectable symptoms accompanying cancer. A treatment may also deemed "efficacious" if it leads to clinical benefit such as, reduction in expression of the SMYD2 gene, or a decrease in size, prevalence, or metastatic potential of the cancer in the subject. When the treatment is applied prophylactically, "efficacious" means that it retards or prevents cancers from forming or prevents or alleviates a clinical symptom of cancer. Efficaciousness is determined in association with any known method for diagnosing or treating the particular tumor type.

### Genes or Proteins:

The present invention relates to the genes of SMYD2 (SET and MYND domain containing 2), HSP90AB1(heat shock protein 90kDa alpha (cytosolic), class B member 1) and RB1 (retinoblastoma 1) as well as proteins encoded by these genes.

The typical nucleic acid and amino acid sequences of genes of interest to the present invention are shown in the following numbers. However, the invention is not limited to these particular sequences:
SMYD2: SEQ ID NO: 62 and 63;
HSP90AB1: SEQ ID NO: 64 and 65;
RB1: SEQ ID NO: 67 and 68.
Above sequence data is also available via following GenBank accession numbers:
SMYD2: NM_020197 and NP_064582;
HSP90AB1: NM_007355 and NP_031381;
RB1: NM_000321 and NP_000312.

Herein, a polypeptide encoded by a gene of interest, for example the SMYD2, HSP90AB1 or RB1 gene, is referred to as the "SMYD2 (or HSP90AB1 or RB1) polypeptide" or "SMYD2 (or HSP90AB1 or RB 1) protein", or simply "SMYD2" (or "HSP90AB1" or "RB 1"). In the context of the present invention, the phrase "SMYD2 (or HSP90AB1 or RB 1) gene" encompasses not only polynucleotides that encode the particular human polypeptide but also polynucleotides that encode functional equivalents of the human gene. The particular gene of interest can be obtained from nature as naturally occurring polynucleotides via conventional cloning methods or through chemical synthesis based on the selected nucleotide sequence. As noted above and discussed in greater detail below, methods for cloning genes using cDNA libraries and such are well known in the art.

According to an aspect of the present invention, functional equivalents are also considered to be above "polypeptides". Herein, a "functional equivalent" of a polypeptide is a polypeptide that has a biological activity equivalent to the polypeptide. Namely, any polypeptide that retains the biological ability of the original reference peptide may be used as such a functional equivalent in the present invention.

Examples of functional equivalents include those in which one or more, e.g., 1-5 amino acids or up to 5% of the original amino acids are substituted, deleted, added, and/or inserted to the natural occurring amino acid sequence of the SMYD2 (or HSP90AB or RB 1) protein. Alternatively, the polypeptide may be composed of an amino acid sequence having at least about 80% homology (also referred to as sequence identity) to the sequence of the respective protein, more preferably at least about 90% to 95% homology, often about 96%, 97%, 98% or 99% homology. The homology of a particular polynucleotide or polypeptide can be determined by following the algorithm in "Wilbur and Lipman, Proc Natl Acad Sci USA 80: 726-30 (1983)". In other embodiments, a functional equivalent may be a polypeptide encoded by a polynucleotide that hybridizes to the polynucleotide having the natural occurring nucleotide sequence of the gene under a stringent condition.
In the context of the present invention, polypeptides may have variations in amino acid sequence, molecular weight, isoelectric point, the presence or absence of sugar chains, or form, depending on the cell or host used to produce it or the purification method utilized. Nevertheless, so long as it has a function equivalent to that of the human protein of interest, it is within the scope of functional equivalents of the SMYD2 (or HSP90AB or RB 1) polypeptide.

With respect to functional equivalents composed of mutated or modified form of the polypeptides of interest, in which one or more, amino acids are substituted, deleted, added, or inserted to the natural occurring sequence, it is generally known that modification of one, two or more amino acid in a protein will not significantly impact or influence the function of the protein. In some cases, it may even enhance the desired function of the original protein. In fact, mutated or modified proteins (i.e., peptides composed of an amino acid sequence in which one, two, or several amino acid residues have been modified through substitution, deletion, insertion and/or addition) have been known to retain the original biological activity (Mark et al., Proc Natl Acad Sci USA 81: 5662-6 (1984); Zoller and Smith, Nucleic Acids Res 10:6487-500 (1982); Dalbadie-McFarland et al., Proc Natl Acad Sci USA 79: 6409-13 (1982)). Accordingly, one of skill in the art will recognize that individual additions, deletions, insertions, or substitutions to an amino acid sequence which alter a single amino acid or a small percentage of amino acids (i.e., less than 5%, more preferably less than 3%, even more preferably less than 1%) or those considered to be a "conservative modifications", wherein the alteration of a protein results in a protein with similar functions, are acceptable in the context of the instant invention. Thus, in one embodiment, the peptides of the present invention may have an amino acid sequence wherein one, two or even more amino acids are added, inserted, deleted, and/or substituted in an originally disclosed reference sequence.

So long as the biological activity the protein is maintained, the site and number of amino acid mutations are not particularly limited. However, it is generally preferred to alter 5% or less of the amino acid sequence, more preferably less than 3%, even more preferably less than 1%. Accordingly, in a preferred embodiment, the number of amino acids to be mutated in such a mutant is generally 30 amino acids or less, preferably 20 amino acids or less, more preferably 10 amino acids or less, more preferably 5 or 6 amino acids or less, and even more preferably 3 or 4 amino acids or less.

An amino acid residue to be mutated is preferably mutated into a different amino acid in which the properties of the amino acid side-chain are conserved (a process known as conservative amino acid substitution). Examples of properties of amino acid side chains are hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (G, A, V, L, I, P); a hydroxyl group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W). Conservative substitution tables providing functionally similar amino acids are well known in the art. For example, the following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M) (see, e.g., Creighton, Proteins 1984).

Such conservatively modified polypeptides are included in functional equivalents of the proteins in the context of the present invention. However, the present invention is not restricted thereto and functional equivalents of the peptides of interest can include non-conservative modifications, so long as the resulting modified peptide retains at least one biological activity of the polypeptide is retained. Furthermore, the modified proteins do not exclude polymorphic variants, interspecies homologues, and those encoded by alleles of these polypeptides.

An example of a polypeptide modified by addition of one, two or more amino acid residues is a fusion protein of the SMYD2 polypeptide, HSP90AB1 polypeptide or RB1 polypeptide. Fusion proteins can be made by techniques well known to a person skilled in the art, for example, by linking the DNA encoding the SMYD2 gene, HSP90AB1 gene or RB1 gene with a DNA encoding another peptide or protein, so that the frames match, inserting the fusion DNA into an expression vector and expressing it in a host. The "other" component of the fusion protein is typically a small epitope composed of several to a dozen amino acids. There is no restriction as to the peptides or proteins fused to the SMYD2 polypeptide, HSP90AB1 polypeptide, or RB1 polypeptide so long as the resulting fusion protein retains any one of the objective biological activities of the SMYD2 polypeptide, HSP90AB1 polypeptide or RB1 polypeptide.

Exemplary fusion proteins contemplated by the present invention include fusions of the SMYD2 polypeptide, HSP90AB1 polypeptide or RB1 polypeptide and other small peptides or proteins such as FLAG (Hopp TP, et al., Biotechnology 6: 1204-10 (1988)), a polyhistidine (His-tag) such as 6xHis containing six His (histidine) residues or 10xHis containing 10 His residues, Influenza aggregate or agglutinin (HA), human c-myc fragment, Vesicular stomatitis virus glycoprotein (VSV-GP), p18HIV fragment, T7 gene 10 protein (T7-tag), human simple herpes virus glycoprotein (HSV-tag), E-tag (an epitope on monoclonal phage), SV40T antigen fragment, lck tag, alpha-tubulin fragment, B-tag, Protein C fragment, and the like. Other examples of proteins that can be fused to a protein of the invention include GST (glutathione-S-transferase), Influenza agglutinin (HA), immunoglobulin constant region, beta-galactosidase, MBP (maltose-binding protein), and such.

Other examples of modified proteins contemplated by the present invention include polymorphic variants, interspecies homologues, and those encoded by alleles of these proteins.

Functional equivalents composed of a particular sequence identity to the natural occurring genes or proteins of interest can be identified and isolated using technology that is conventional in the art, for example, hybridization techniques (Sambrook and Russell, Molecular Cloning: A Laboratory Manual, 3rd ed., Cold Spring Harbor Lab. Press, 2001). Hybridization conditions suitable for isolating a DNA encoding a functional equivalent of a gene of interest can be routinely selected by a person skilled in the art.

As used herein, the phrase "stringent (hybridization) conditions" refers to conditions under which a nucleic acid molecule will hybridize to its target sequence, typically in a complex mixture of nucleic acids, but not detectably to other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology-Hybridization with Nucleic Probes, "Overview of principles of hybridization and the strategy of nucleic acid assays" (1993). Generally, stringent conditions are selected to be about 5-10 degrees C lower than the thermal melting point (Tm) for the specific sequence at a defined ionic strength and pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions may also be achieved with the addition of destabilizing agents such as formamide. For selective or specific hybridization, a positive signal is at least two times of background, preferably 10 times of background hybridization. Exemplary stringent hybridization conditions include the following: 50% formamide, 5x SSC, and 1% SDS, incubating at 42 degrees C, or, 5x SSC, 1% SDS, incubating at 65 degrees C, with wash in 0.2x SSC, and 0.1% SDS at 50 degrees C.

In the context of the present invention, an optimal condition of hybridization for isolating a DNA encoding a functionally equivalent polypeptide can be routinely selected by a person skilled in the art. For example, hybridization may be performed by conducting pre-hybridization at 68 degrees C for 30 min or longer using "Rapid-hyb buffer" (Amersham LIFE SCIENCE), adding a labeled probe, and warming at 68 degrees C for 1 hour or longer. The following washing step can be conducted, for example, in a low stringent condition. An exemplary low stringent condition may include 42 degrees C, 2x SSC, 0.1% SDS, preferably 50 degrees C, 2x SSC, 0.1% SDS. High stringency conditions are often preferably used. An exemplary high stringency condition may include washing 3 times in 2x SSC, 0.01% SDS at room temperature for 20 min, then washing 3 times in 1x SSC, 0.1% SDS at 37 degrees C for 20 min, and washing twice in 1x SSC, 0.1% SDS at 50 degrees C for 20 min. However, several factors, such as temperature and salt concentration, can influence the stringency of hybridization and one skilled in the art can routinely adjust these and other factors to arrive at the desired stringency.

Thus, in the context of the present invention, functional equivalents include polypeptides encoded by DNAs that hybridize under stringent conditions with a whole or part of the DNA sequence encoding the human polypeptides of interest. These functional equivalents include mammal homologues of the human protein, for example, polypeptides encoded by monkey, mouse, rat, rabbit or bovine SMYD2 genes (or HSP90AB1 genes or RB1 genes).

In place of hybridization, a gene amplification method, for example, the polymerase chain reaction (PCR) method, can be utilized to isolate a DNA encoding a functional equivalent of a human polypeptide of interest, using a primer synthesized based on the sequence information of the associated DNA. Examples of illustrative primer sequences are pointed out in Semi-quantitative RT-PCR in the EXAMPLE section.

A functional equivalent of a polypeptide encoded by the DNA isolated through the above hybridization techniques or gene amplification techniques will normally have a high homology (also referred to as sequence identity) to the amino acid sequence of original reference polypeptide. "High homology" (also referred to as "high sequence identity") typically refers to the degree of identity between two optimally aligned sequences (either polypeptide or polynucleotide sequences). Typically, high homology or sequence identity refers to homology of 40% or higher, for example, 60% or higher, for example, 80% or higher, for example, 85%, 90%, 95%, 98%, 99%, or higher. The degree of homology or identity between two polypeptide or polynucleotide sequences can be determined by following the algorithm [Wilbur WJ & Lipman DJ. Proc Natl Acad Sci USA. 1983 Feb; 80 (3):726-30].

Percent sequence identity and sequence similarity can be readily determined using conventional techniques such as the BLAST and BLAST 2.0 algorithms, which are described [Altschul SF, et al., J Mol Biol. 1990 Oct 5; 215 (3):403-10; Nucleic Acids Res. 1997 Sep 1;25(17):3389-402]. Software for performing BLAST analyses is publicly available through the National Center for Biotechnology Information (on the worldwide web at ncbi.nlm.nih.gov/). The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits acts as seeds for initiating searches to find longer HSPs containing them.

The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached.

The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands. For amino acid sequences, the BLASTP program uses as defaults a word size (W) of 3, an expectation (E) of 10, and the BLOSUM62 scoring matrix [Henikoff S & Henikoff JG. Proc Natl Acad Sci USA. 1992 Nov 15;89(22):10915-9].

### Method Of Detecting Or Diagnosing Cancer:

The present invention relates to the discovery that SMYD2 can serve as a diagnostic marker of cancer and thus finds utility in the detection of cancers related thereto. As demonstrated herein, the expression of SMYD2 gene is specifically and significantly elevated in bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer (Fig. 1). Accordingly, the gene identified herein as well as their transcription and translation products find diagnostic utility as a marker for bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer and by measuring the expression of SMYD2 gene in a cell sample, bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer can be diagnosed. Specifically, the present invention provides a method for detecting, diagnosing and/or determining the presence of or a predisposition for developing bladder cancer, lung cancer, breast cancer, cervix cancer, colon cancer, kidney cancer, liver cancer, head and neck cancer, seminoma, cutaneous cancer , pancreatic cancer, lymphoma, ovarian cancer, leukemia or prostate cancer by determining the expression level of SMYD2 gene in a subject-derived biological sample.

In the context of the present invention, the term "diagnosing" can encompass detection as well as predictions and likelihood analyses. Thus, the present invention provides a method for detecting or identifying the presence of cancer cells or the predisposition to develop cancer in a subject, the method including the step of determining the expression level of the SMYD2 gene in a subject-derived biological sample, wherein an increase in the expression level as compared to a normal control level of the gene indicates the presence or suspicion of cancer cells in the tissue.

According to the present invention, an intermediate result for examining the condition of a subject may be provided. Such intermediate result may be combined with additional information to assist a doctor, nurse, or other practitioner to determine that a subject suffers from the disease. That is, the present invention provides a diagnostic marker SMYD2 for examining cancer.

Alternatively, the present invention provides a method for detecting or identifying cancer cells in a subject-derived bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer tissue sample, the method including the step of determining the expression level of the SMYD2 gene in a subject-derived sample, wherein an increase in the expression level as compared to a normal control level of the gene indicates the presence or suspicion of cancer cells in the tissue.

The diagnostic methods of the present invention may be used clinically in making decisions concerning treatment modalities, including therapeutic intervention, diagnostic criteria such as disease stages, and disease monitoring and surveillance for cancer. To improve the accuracy of diagnosis, the expression level of other cancer-associated genes, for example, genes known to be differentially expressed in cancer may also be determined. Furthermore, in the case where the expression levels of multiple cancer-related genes are compared, a similarity in the gene expression pattern between the sample and the reference that is cancerous indicates that the subject is suffering from or at a risk of developing lung cancer.

Accordingly, the expression results for a particular gene of interest may be combined with additional information or another diagnostic indicator, including tissue pathology, levels of known tumor marker(s) in blood, and clinical course of the subject, to assist a doctor, nurse, or other healthcare practitioner in diagnosing a subject as afflicted with the disease. In other words, the present invention may provide a doctor with useful information to diagnose a subject as afflicted with the disease. For example, according to the present invention, when there is doubt regarding the presence of cancer cells in the tissue obtained from a subject, clinical decisions can be reached by considering the expression level of the SMYD2 gene, plus a different aspect of the disease including tissue pathology, levels of known tumor marker(s) in blood, and clinical course of the subject, etc. For example, some well-known diagnostic lung cancer markers in blood include ACT, BFP, CA19-9, CA50, CA72-4, CA130, CA602, CEA, IAP, KMO-1, SCC, SLX, SP1, Span-1, STN, TPA, and cytokeratin 19 fragment. Some well-known bladder cancer markers in blood include NMP22, BFP and TPA. Alternatively, diagnostic breast cancer markers in blood such as CA15-3, BCA225, CSLEX, NCC-ST-439, CEA, TPA and HER2 are also well known. Some well-known diagnostic colon cancer markers in blood include CA72-4,STN,CA19-9,CEA and NCC-ST-439, kidney cancer markers in blood include BFP and IAP, liver cancer markers in blood include AFP and PIVKA-2, head and neck cancer marker in blood include SCC, seminoma markers in blood include AFP, beta-hCG, LDH, cutaneous cancer marker in blood include SCC and pancreatic cancer marker in blood include CA19-9, Span-1,SLX and CEA. Namely, in this particular embodiment of the present invention, the outcome of the gene expression analysis serves as an intermediate result for further diagnosis of a subject's disease state.

Particularly preferred embodiments of the present invention are set forth as items [1] to [11]:
[1] A method of detecting or diagnosing the presence of or a predisposition for developing cancer in a subject, the method comprising the step of:
   (A)determining an expression level of an SMYD2 gene in a subject-derived biological sample, wherein an increase of said level compared to a normal control level of said gene indicates that said subject suffers from or is at risk of developing cancer, wherein the expression level is determined by any one of method selected from the group consisting of:
      (a) detecting an mRNA of the SMYD2 gene;
      (b) detecting a protein encoded by the SMYD2 gene; and
      (c) detecting a biological activity of the protein encoded by the SMYD2 gene, or
   (B)
      (i) isolating or collecting a subject-derived biological sample,
      (ii) contacting the subject-derived biological sample with an oligonucleotide that hybridizes to an mRNA of the SMYD2 gene, or an antibody that binds to a protein encoded by the SMYD2 gene for measuring or determining an expression level of the SMYD2 gene, and
      (iii) measuring or determining an expression level of the SMYD2 gene based on said contacting, wherein an increase of the level as compared to a normal control level of the SMYD2 gene indicates that the subject suffers from or is at risk of developing cancer;
[2] The method of [1], wherein the measured sample expression level is at least 10% greater than the normal control level;
[3] The method of [1], wherein the biological activity is cell-proliferation promoting activity or methyltransferase activity;
[4] The method of any one of [1] to [3], wherein the cancer is selected from the group consisting of bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer;
[5] The method of any one of [1] to [4], wherein the expression level is determined by detecting hybridization of a probe to mRNA of the gene;
[6] The method of any one of [1] to [4], wherein the expression level is determined by detecting the binding of an antibody against the protein encoded by the gene;
[7] The method of any one of [1] to [6], wherein the subject-derived biological sample includes biopsy specimen, sputum, blood, pleural effusion and urine;
[8] The method of any one of [1] to [6], wherein the subject-derived biological sample includes an epithelial cell;
[9] The method of [8], wherein the subject-derived biological sample includes a cancer cell; and
[10] The method of [1], wherein the subject-derived biological sample includes a cancerous epithelial cell.
[11] The method of [4], wherein when the cancer is bladder cancer, the subject-derived biological sample is a bladder tissue derived from the subject; when the cancer is lung cancer, the subject-derived biological sample is a lung tissue; when the cancer is breast cancer, the subject-derived biological sample is a breast tissue; when the cancer is cervix cancer, the subject-derived biological sample is a cervical tissue; when the cancer is colon cancer, the subject-derived biological sample is a colon tissue; when the cancer is kidney cancer, the subject-derived biological sample is a kidney tissue; when the cancer is liver cancer, the subject-derived biological sample is a liver tissue; when the cancer is head and neck cancer, the subject-derived biological sample is a head and neck tissue; when the cancer is seminoma, the subject-derived biological sample is a testicular tissue; when the cancer is cutaneous cancer, the subject-derived biological sample is a dermal tissue; when the cancer is pancreatic cancer, the subject-derived biological sample is a pancreatic tissue; when the cancer is lymphoma, the subject-derived biological sample is a blood sample or a lymph node tissue; when the cancer is ovarian cancer, the subject-derived biological sample is a ovarian tissue; when the cancer is leukemia, the subject-derived biological sample is blood sample or bone marrow tissue; when the cancer is prostate cancer, the subject-derived biological sample is a prostate tissue.

The method of diagnosing cancer including bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer will be described in more detail below.

A subject to be diagnosed by the present method is preferably a mammal. Exemplary mammals include, but are not limited to, e.g., human, non-human primate, mouse, rat, dog, cat, horse, and cow.

The method of the present invention preferably utilizes a biological sample obtained or collected from a subject to be diagnosed to perform the diagnosis. Any biological material can be used as the biological sample for the determination so long as it may include the objective transcription or translation product of SMYD2. Examples of suitable subject-derived biological samples include, but are not limited to, bodily tissues which are desired for diagnosing or are suspicion of suffering from cancer, and fluids, such as a biopsy specimen, blood, sputum, pleural effusion and urine. Preferably, the biological sample contains a cell population including an epithelial cell, more preferably a cancerous epithelial cell or an epithelial cell derived from tissue suspected to be cancerous. Further, if necessary, the cell may be purified from the obtained bodily tissues and fluids, and then used as the biological sample.

For example, in the context of the present invention, suitable cancers for diagnosis or detection include bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. In order to diagnose or detect theses cancers, a subject-derived biological sample may be collected from following organs:
Bladder: for bladder cancer,
Lung: for lung cancer,
Breast: for breast cancer,
Cervix: for cervix cancer,
Colon: for colon cancer,
Kidney: for kidney cancer,
Liver: for liver cancer,
Head and neck: for head and neck cancer,
Spermary: for seminoma,
Dermis: for cutaneous cancer,
Pancreas: for pancreatic cancer,
Blood or lymph node: for lymphoma,
Ovary: for ovarian cancer,
Blood or bone marrow: for leukemia,
Prostate: for prostate cancer.

According to the present invention, the expression level of SMYD2 gene in a subject-derived biological sample is determined and then correlated to a particular healthy or disease state by comparison to a control sample. The expression level can be determined at the transcription (nucleic acid) product level, using methods known in the art. For example, SMYD2 gene may be quantified using probes by hybridization methods (e.g., Northern hybridization). The detection may be carried out on a chip or an array. An array is preferable for detecting the expression level of a plurality of genes (e.g., various cancer specific genes) including SMYD2 gene. Those skilled in the art can prepare such probes utilizing the known sequence information of the SMYD2 gene (SEQ ID NO: 62). For example, the cDNA of SMYD2 gene may be used as a probe. If necessary, the probe may be labeled with a suitable label, such as dyes, fluorescents and isotopes, and the expression level of the gene may be detected as the intensity of the hybridized labels.

Alternatively, the transcription product of SMYD2 gene may be quantified using primers by amplification-based detection methods (e.g., RT-PCR). Such primers can also be prepared based on the available sequence information of the gene. For example, the primer pairs (SEQ ID NOs: 5 and 6) used in the Example may be employed for the detection by RT-PCR or Northern blot, but the present invention is not restricted thereto.

A probe or primer suitable for use in the context of the present method will hybridize under stringent, moderately stringent, or low stringent conditions to the mRNA of SMYD2 gene. Details of "stringent conditions" are described in the in the section entitled "Genes and Proteins".

Alternatively, diagnosis may involve the quantitative detection of a translation product (i.e., a polypeptide or protein), using methods known in the art. For example, the quantity of SMYD2 protein may be determined using an antibody against the protein of interest and correlated to a disease or normal state. The quantity of the translation products/protein may be determined by any conventional technology, including, for example, immunoassay methods that use an antibody specifically recognizing the protein. Antibodies suitable for use in the context of the methods of the present invention may be monoclonal or polyclonal. Furthermore, any immunogenic fragments or modifications (e.g., chimeric antibody, scFv, Fab, F(ab')2, Fv, etc.) of the antibody may be used for the detection, so long as the fragment retains the binding ability to SMYD2 protein. Methods to prepare these kinds of antibodies for the detection of proteins are well known in the art, and any method may be employed in the present invention to prepare such antibodies and equivalents thereof.

Alternatively, one may determine the expression level of an SMYD2 gene based on its translation product, for example through the study of the intensity of staining may be observed via immunohistochemical analysis using an antibody against SMYD2 protein. More particularly, the observation of strong staining indicates increased presence of the protein and at the same time high expression level of a SMYD2 gene.

Furthermore, the translation product may be detected based on its biological activity. As discovered herein, the SMYD2 protein was demonstrated herein to be involved in the growth of cancer cells. Thus, the cancer cell growth promoting ability and methyltransferase activity of the SMYD2 protein may be used as an index of the SMYD2 protein existing in the biological sample. Herein, cell growth promoting ability is also referred to as "cell proliferative activity", "cell-proliferation promoting activity" or "cell-proliferation enhancing activity". Herein, methyltransferase activity to substrate is useful for quantification of SMYD2 protein based on its biological activity. The methylation level of the substrate (especially, histone H4 protein or fragment thereof, histone H3 protein or fragment thereof, HSP90AB1 protein or fragment thereof, RB 1 protein or fragment thereof) can be determined by the methods well known in the art.

Moreover, in addition to the expression level of SMYD2 gene, the expression level of other cancer-associated genes, for example, genes known to be differentially expressed in bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer may also be determined to improve the accuracy of the diagnosis.

In the context of the present invention, methods for detecting or identifying cancer in a subject or cancer cells in a subject-derived sample begin with a determination of SMYD2 gene expression level. Once determined, using any of the aforementioned techniques, this value is compared to a control level. In the context of the present invention, gene expression levels are deemed to be "altered" or "increased" when the gene expression changes or increases by, for example, 10%, 25%, or 50% from, or at least 0.1 fold, at least 0.2 fold, at least 0.5 fold, at least 2 fold, at least 5 fold, or at least 10 fold or more compared to a control level. Accordingly, the expression levels of cancer marker genes including SMYD2 gene in a biological sample can be considered to be increased if it increase from a control level of the corresponding cancer marker gene by, for example, 10%, 25%, or 50%; or increases to more than 1.1 fold, more than 1.5 fold, more than 2.0 fold, more than 5.0 fold, more than 10.0 fold, or more.

In the context of the present invention, the phrase "control level" refers to the expression level of the SMYD2 gene detected in a control sample and encompasses both a normal control level and a cancer control level. The phrase "normal control level" refers to a level of the SMYD2 gene expression detected in a normal healthy individual or in a population of individuals known not to be suffering from cancer. A normal individual is one with no clinical symptom of cancer. A normal control level can be determined using a normal cell obtained from a non-cancerous tissue. A "normal control level" may also be the expression level of the SMYD2 gene detected in a normal healthy tissue or cell of an individual or population known not to be suffering from cancer. On the other hand, the phrase "cancer control level" or "cancerous control level" refers to an expression level of the SMYD2 gene detected in the cancerous tissue or cell of an individual or population suffering from cancer.

An increase in the expression level of SMYD2 detected in a subject-derived sample as compared to a normal control level indicates that the subject (from which the sample has been obtained) suffers from or is at risk of developing cancer. In the context of the present invention, subject-derived samples may be any tissues obtained from test subjects, e.g., patients suspected of having cancer. For example, tissues may include epithelial cells. More particularly, tissues may be suspicious cancerous epithelial cells. A similarity between the expression level of a sample and the cancer control level indicates that the subject (from which the sample has been obtained) suffers from or is at risk of developing cancer. When the expression levels of other cancer-related genes are also measured and compared, a similarity in the gene expression pattern between the sample and the reference that is cancerous indicates that the subject is suffering from or at a risk of developing cancer.

The control level may be determined at the same time with the test biological sample by using a sample(s) previously collected and stored from a subject/subjects whose disease state (cancerous or non-cancerous) is/are known. Alternatively, the control level may be determined by a statistical method based on the results obtained by analyzing previously determined expression level(s) of SMYD2 gene in samples from subjects whose disease state are known. Furthermore, the control level can be a database of expression patterns from previously tested cells. Moreover, according to an aspect of the present invention, the expression level of SMYD2 gene in a biological sample may be compared to multiple control levels, which control levels are determined from multiple reference samples. It is preferred to use a control level determined from a reference sample derived from a tissue type similar to that of the patient-derived biological sample. Moreover, it is preferred, to use the standard value of the expression levels of SMYD2 gene in a population with a known disease state. The standard value may be obtained by any method known in the art. For example, a range of mean +/- 2 S.D. or mean +/- 3 S.D. may be used as standard value.

When the expression level of SMYD2 gene in a subject-derived biological sample is increased as compared to the normal control level or is similar to the cancerous control level, the subject may be diagnosed to be suffering from or at a risk of developing cancer. Furthermore, in the case where the expression levels of multiple cancer-related genes are compared, a similarity in the gene expression pattern between the sample and the reference that is cancerous indicates that the subject is suffering from or at a risk of developing cancer.

Difference between the expression levels of a test biological sample and the control level can be normalized by the expression level of control nucleic acids, e.g., housekeeping genes, whose expression levels are known not to differ depending on the cancerous or non-cancerous state of the cell. Exemplary control genes include, but are not limited to, beta-actin, glyceraldehyde 3 phosphate dehydrogenase, and ribosomal protein P1.

### A Kit For Diagnosing Cancer And/Or Monitoring The Efficacy Of A Cancer Therapy:

The present invention provides a kit for detecting or diagnosing cancer and/or monitoring the efficacy of a cancer therapy. Preferably, the cancer is bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer. Specifically, the kit includes at least one reagent for detecting the expression of an SMYD2 in a subject-derived biological sample, which reagent may be selected from the group of:
(a) a reagent for detecting an mRNA of the SMYD2 gene;
(b) a reagent for detecting a SMYD2 protein; and
(c) a reagent for detecting a biological activity of the SMYD2 protein.

Suitable reagents for detecting an mRNA of the SMYD2 gene include nucleic acids that specifically bind to or identify the SMYD2 mRNA, such as oligonucleotides that have a sequence complementary to a part of the SMYD2 mRNA. These kinds of oligonucleotides are exemplified by primers and probes that are specific to the SMYD2 mRNA. These kinds of oligonucleotides may be prepared based on methods well known in the art. If needed, the reagent for detecting the SMYD2 mRNA may be immobilized on a solid matrix. Moreover, more than one reagent for detecting the SMYD2 mRNA may be included in the kit.

A probe or primer of the present invention typically is a substantially purified oligonucleotide. The oligonucleotide typically comprises a region of nucleotide sequence that hybridizes under stringent conditions to at least about 2000, 1000, 500, 400, 350, 300, 250, 200, 150, 100, 50, or 25 bases of consecutive sense strand nucleotide sequence of a nucleic acid comprising an SMYD2 sequence, or an anti sense strand nucleotide sequence of a nucleic acid comprising an SMYD2 sequence, or of a naturally occurring mutant of these sequences. In particular, for example, in a preferred embodiment, an oligonucleotide having 5-50 in length can be used as a primer for amplifying the genes, to be detected. Alternatively, in hybridization based detection procedures, a polynucleotide having a few hundreds (e.g., about 100-200) bases to a few kilo (e.g., about 1000-2000) bases in length can also be used for a probe (e.g., northern blotting assay or DNA microarray analysis).

On the other hand, suitable reagents for detecting the SMYD2 protein include antibodies to the SMYD2 protein. The antibody may be monoclonal or polyclonal. Furthermore, any fragment or modification (e.g., chimeric antibody, scFv, Fab, F(ab')2, Fv, etc.) of the antibody may be used as the reagent, so long as the fragment retains the binding ability to the SMYD2 protein. Methods to prepare these kinds of antibodies for the detection of proteins are well known in the art, and any method may be employed in the present invention to prepare such antibodies and equivalents thereof. Furthermore, the antibody may be labeled with signal generating molecules via direct linkage or an indirect labeling technique. Labels and methods for labeling antibodies and detecting the binding of antibodies to their targets are well known in the art and any labels and methods may be employed for the present invention. Moreover, more than one reagent for detecting the SMYD2 protein may be included in the kit.

An antibody may be labeled with a signal generating molecule via direct linkage or an indirect labeling technique. Labels and methods for labeling antibodies and detecting the binding of antibodies to their targets are well known in the art and any labels and methods may be employed for the present invention. Moreover, more than one reagent for detecting the SMYD2 protein may be included in the kit.

Alternatively, expression of the SMYD2 protein in a biological sample may be detected and measured using its biological activity as an index. The biological activity can be determined by, for example, measuring the cell proliferating activity or methyltransferase activity due to the expressed SMYD2 protein in a biological sample.

For example, the cell is cultured in the presence of a subject-derived biological sample, and then by detecting the speed of proliferation, or by measuring the cell cycle or the colony forming ability the cell proliferating activity of the biological sample can be determined. If needed, the reagent for detecting the SMYD2 mRNA may be immobilized on a solid matrix. Moreover, more than one reagent for detecting the biological activity of the SMYD2 protein may be included in the kit.

On the other hand, the methyltransferase activity in a biological sample can be determined by incubating the biological sample with a substrate such as a histone protein (e.g. histone H4 protein or histone H3 protein) or fragment thereof, an HSP90AB 1 protein or fragment thereof, or an RB1 protein or fragment thereof, detecting methylation level of the substrate using antibody against methylated substrate. Thus, the present kit may include substrate (especially histone H4 protein or fragment thereof, histone H3 protein or fragment thereof, HSP90AB 1 protein or fragment thereof, or RB1 protein or fragment thereof) and anti-methylated substrate antibody. Examples of such antibodies include antibodies that bind to the methylated lysine 36 of histone H3 protein, the methylated lysine 531 and/or lysine 574 of HSP90AB1 protein, or the methylated lysine 810 of RB1 protein. Otherwise, the present kit may include an appropriate labeled methyl donor for detecting formaldehyde released by histone methylation. The labeled methyl donor can be an S- adenosyl [methyl-³H] methionine (SAM) or an L-[methyl-³H] methionine.

The kit may contain more than one of the aforementioned reagents. Furthermore, the kit may include a solid matrix and reagent for binding a probe against the SMYD2 gene or antibody against the SMYD2 protein, a medium and container for culturing cells, positive and negative control reagents, and a secondary antibody for detecting an antibody against the SMYD2 protein. A kit of the present invention may further include other materials desirable from a commercial and user standpoint, including buffers, diluents, filters, needles, syringes, and package inserts (e.g., written, tape, CD-ROM, etc.) with instructions for use. These reagents and such may be retained in a container with a label. Suitable containers include bottles, vials, and test tubes. The containers may be formed from a variety of materials, such as glass or plastic.

According to an aspect of the present invention, the kit of the present invention for diagnosing cancer may further include either of positive or negative controls sample, or both. In the context of the present invention, positive control samples may be established bladder cancer cell lines, lung cancer cell lines, breast cancer cell lines, cervix cancer cell lines, colon cancer cell lines, kidney cancer cell lines, liver cancer cell lines, head and neck cancer cell lines, seminoma cell lines, cutaneous cancer cell lines, pancreatic cancer cell lines, lymphoma cell lines, ovarian cancer cell lines, leukemia cell lines or prostate cancer cell lines. Alternatively, the SMYD2 positive samples may also be a clinical bladder cancer tissue(s), lung cancer tissue(s), breast cancer tissue(s), cervix cancer tissue(s), colon cancer tissue(s), kidney cancer tissue(s), liver cancer tissue(s), head and neck cancer tissue(s), seminoma tissue(s), cutaneous cancer tissue(s), pancreatic cancer tissue(s), lymphoma cells, ovarian cancer tissues, leukemia cells or prostate cancer tissues obtained from cancer patient(s). Alternatively, positive control samples may be prepared by determined a cut-off value and preparing a sample containing an amount of an SMYD2 mRNA or protein more than the cut-off value. Herein, the phrase "cut-off value" refers to the value dividing between a normal range and a cancerous range. For example, one skilled in the art may be determine a cut-off value using a receiver operating characteristic (ROC) curve. The present kit may include an SMYD2 standard sample providing a cut-off value amount of an SMYD2 mRNA or polypeptide. On the contrary, negative control samples may be prepared from non-cancerous cell lines or non-cancerous tissues such as a normal bladder tissue(s), lung tissue(s), breast tissue(s), cervical tissue(s), colon tissue(s), kidney tissue(s), liver tissue(s), head tissues and neck tissue(s), testicular tissue(s), dermal tissue(s), pancreatic tissue(s), lymph node tissue(s), ovarian tissue(s), bone marrow tissue(s) or prostate tissue(s) may be prepared by preparing a sample containing an SMYD2 mRNA or protein less than cut-off value.

As an embodiment of the present invention, when the reagent is a probe against the SMYD2 protein, the reagent may be immobilized on a solid matrix, such as a porous strip, to form at least one detection site. The measurement or detection region of the porous strip may include a plurality of sites, each containing a nucleic acid (probe). A test strip may also contain sites for negative and/or positive controls. Alternatively, control sites may be located on a strip separated from the test strip. Optionally, the different detection sites may contain different amounts of immobilized nucleic acids, i.e., a higher amount in the first detection site and lesser amounts in subsequent sites. Upon the addition of test sample, the number of sites displaying a detectable signal provides a quantitative indication of the amount of SMYD2 mRNA, present in the sample. The detection sites may be configured in any suitably detectable shape and are typically in the shape of a bar or dot spanning the width of a test strip.

### Screening For An Anti-Cancer Substance:

Using an SMYD2 gene, an SMYD2 polypeptide or functional equivalent thereof, or transcriptional regulatory region of the gene, it is possible to screen substances that alter the expression of the SMYD2 gene or the biological activities of the SMYD2 polypeptide. Such substances may be used as candidate pharmaceuticals for treating or preventing cancer. Thus, the present invention provides methods of screening for candidate substances for either or both of the treatment and prevention of cancer using the SMYD2 gene, the SMYD2 polypeptide or functional equivalent thereof, or a transcriptional regulatory region of the SMYD2 gene.

Substances isolated and identified by the screening method of the present invention as suitable candidates are expected to reduce, suppress, and/or inhibit the expression of the SMYD2 gene, or the activity of the translation product of the SMYD2 gene, and thus, is a candidate for either or both of treating and preventing cancer (in particular, bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer).

Namely, the substances screened through the present methods are deemed to have a clinical benefit and can be further tested for its ability to limit or prevent cancer cell growth in animal models or test subjects.

In the context of the present invention, substances to be identified through the present screening methods include any substance or composition including several substances. Furthermore, the test substance exposed to a cell or protein according to the screening methods of the present invention may be a single substance or a combination of substances. When a combination of substances is used in the methods, the substances may be contacted sequentially or simultaneously.

Alternatively, the present invention provides a method of evaluating therapeutic effect of a test substance on treating or preventing cancer or inhibiting cancer cell growth.

Any test substance, for example, cell extracts, cell culture supernatant, products of fermenting microorganism, extracts from marine organism, plant extracts, purified or crude proteins, peptides, non-peptide substances, synthetic micromolecular substances (including nucleic acid constructs, such as antisense RNA, siRNA, Ribozymes, and aptamer etc.) and natural substances can be used in the screening methods of the present invention. The test substance of the present invention can be also obtained using any of the numerous approaches in combinatorial library methods known in the art, including (1) biological libraries, (2) spatially addressable parallel solid phase or solution phase libraries, (3) synthetic library methods requiring deconvolution, (4) the "one-bead one-substance" library method and (5) synthetic library methods using affinity chromatography selection. The biological library methods using affinity chromatography selection is limited to peptide libraries, while the other four approaches are applicable to peptide, non-peptide oligomer or small molecule libraries of substances (Lam, Anticancer Drug Des 1997, 12: 145-67). Examples of methods for the synthesis of molecular libraries can be found in the art (DeWitt et al., Proc Natl Acad Sci USA 1993, 90: 6909-13; Erb et al., Proc Natl Acad Sci USA 1994, 91: 11422-6; Zuckermann et al., J Med Chem 37: 2678-85, 1994; Cho et al., Science 1993, 261: 1303-5; Carell et al., Angew Chem Int Ed Engl 1994, 33: 2059; Carell et al., Angew Chem Int Ed Engl 1994, 33: 2061; Gallop et al., J Med Chem 1994, 37: 1233-51). Libraries of substances may be presented in solution (see Houghten, Bio/Techniques 1992, 13: 412-21) or on beads (Lam, Nature 1991, 354: 82-4), chips (Fodor, Nature 1993, 364: 555-6), bacteria (US Pat. No. 5,223,409), spores (US Pat. No. 5,571,698; 5,403,484, and 5,223,409), plasmids (Cull et al., Proc Natl Acad Sci USA 1992, 89: 1865-9) or phage (Scott and Smith, Science 1990, 249: 386-90; Devlin, Science 1990, 249: 404-6; Cwirla et al., Proc Natl Acad Sci USA 1990, 87: 6378-82; Felici, J Mol Biol 1991, 222: 301-10; US Pat. Application 2002103360). A compound in which a part of the structure of the substance screened by any one of the present screening methods is converted by addition, deletion and/or replacement, is included in the substances obtained by the screening methods of the present invention.

Furthermore, when the screened test substance is a protein, for obtaining a DNA encoding the protein, either the whole amino acid sequence of the protein may be determined to deduce the nucleic acid sequence coding for the protein, or partial amino acid sequence of the obtained protein may be analyzed to prepare an oligo DNA as a probe based on the sequence, and screen cDNA libraries with the probe to obtain a DNA encoding the protein. The obtained DNA is confirmed it's usefulness in preparing the test substance which is a candidate for treating or preventing cancer.

Test substances useful in the screenings described herein can also include antibodies that specifically bind to SMYD2 protein or partial peptides thereof that lack the biological activity of the original proteins in vivo.

Although the construction of test substance libraries is well known in the art, herein below, additional guidance in identifying test substances and construction libraries of such substances for the present screening methods are provided.

In one aspect of the present invention, suppression of the expression level and/or biological activity of SMYD2 protein lead to suppression of the growth of cancer cells. Therefore, when a substance suppresses the expression and/or activity of SMYD2 protein, such suppression is indicative of a potential therapeutic effect in a subject. In the context of the present invention, a potential therapeutic effect refers to a clinical benefit with a reasonable expectation. Examples of such clinical benefit include but are not limited to;
(a) reduction in expression of the SMYD2 gene,
(b) a decrease in size, prevalence, growth, or metastatic potential of the cancer in the subject,
(c) preventing cancers from forming, or
(d) preventing or alleviating a clinical symptom of cancer.

### (i) Molecular modeling:

Construction of test substance libraries is facilitated by knowledge of the molecular structure of substances known to have the properties sought, and/or the molecular structure of SMYD2 protein. One approach to preliminary screening of test substances suitable for further evaluation utilizes computer modeling of the interaction between the test substance and SMYD2 protein.

Computer modeling technology allows for the visualization of the three-dimensional atomic structure of a selected molecule and the rational design of new substances that will interact with the molecule. The three-dimensional construct typically depends on data from x-ray crystallographic analysis or NMR imaging of the selected molecule. The molecular dynamics require force field data. The computer graphics systems enable prediction of how a new substance will link to the target molecule and allow experimental manipulation of the structures of the substance and target molecule to perfect binding specificity. Prediction of what the molecule-substance interaction will be when small changes are made in one or both requires molecular mechanics software and computationally intensive computers, usually coupled with user-friendly, menu-driven interfaces between the molecular design program and the user.

An example of the molecular modeling system described generally above includes the CHARMM and QUANTA programs, Polygen Corporation, Waltham, Mass. CHARMM performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles have been published on the subject of computer modeling of drugs interactive with specific proteins, examples of which include Rotivinen et al. Acta Pharmaceutica Fennica 1988, 97: 159-66; Ripka, New Scientist 1988, 54-8; McKinlay & Rossmann, Annu Rev Pharmacol Toxiciol 1989, 29: 111-22; Perry & Davies, Prog Clin Biol Res 1989, 291: 189-93; Lewis & Dean, Proc R Soc Lond 1989, 236: 125-40, 141-62; and, with respect to a model receptor for nucleic acid components, Askew et al., J Am Chem Soc 1989, 111: 1082-90.

Other computer programs that screen and graphically depict chemicals are available from companies such as BioDesign, Inc., Pasadena, Calif., Allelix, Inc, Mississauga, Ontario, Canada, and Hypercube, Inc., Cambridge, Ontario. See, e.g., DesJarlais et al., J Med Chem 1988, 31: 722-9; Meng et al., J Computer Chem 1992, 13: 505-24; Meng et al., Proteins 1993, 17: 266-78; Shoichet et al., Science 1993, 259: 1445-50.

Once a putative inhibitor has been identified, combinatorial chemistry techniques can be employed to construct any number of variants based on the chemical structure of the identified putative inhibitor, as detailed below. The resulting library of putative inhibitors, or "test substances" may be screened using the methods of the present invention to identify test substances suited to the treatment and/or prophylaxis of cancer and/or the prevention of post-operative recurrence of cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

### (ii) Combinatorial chemical synthesis:

Combinatorial libraries of test substances may be produced as part of a rational drug design program involving knowledge of core structures existing in known inhibitors. This approach allows the library to be maintained at a reasonable size, facilitating high throughput screening. Alternatively, simple, particularly short, polymeric molecular libraries may be constructed by simply synthesizing all permutations of the molecular family making up the library. An example of this latter approach would be a library of all peptides six amino acids in length. Such a peptide library could include every 6 amino acid sequence permutation. This type of library is termed a linear combinatorial chemical library.

Preparation of combinatorial chemical libraries is well known to those of skill in the art, and may be generated by either chemical or biological synthesis. Combinatorial chemical libraries include, but are not limited to, peptide libraries (see, e.g., US Patent 5,010,175; Furka, Int J Pept Prot Res 1991, 37: 487-93; Houghten et al., Nature 1991, 354: 84-6). Other chemistries for generating chemical diversity libraries can also be used. Such chemistries include, but are not limited to: peptides (e.g., PCT Publication No. WO 91/19735), encoded peptides (e.g., WO 93/20242), random bio-oligomers (e.g., WO 92/00091), benzodiazepines (e.g., US Patent 5,288,514), diversomers such as hydantoins, benzodiazepines and dipeptides (DeWitt et al., Proc Natl Acad Sci USA 1993, 90:6909-13), vinylogous polypeptides (Hagihara et al., J Amer Chem Soc 1992, 114: 6568), nonpeptidal peptidomimetics with glucose scaffolding (Hirschmann et al., J Amer Chem Soc 1992, 114: 9217-8), analogous organic syntheses of small substance libraries (Chen et al., J. Amer Chem Soc 1994, 116: 2661), oligocarbamates (Cho et al., Science 1993, 261: 1303), and/or peptidylphosphonates (Campbell et al., J Org Chem 1994, 59: 658), nucleic acid libraries (see Ausubel, Current Protocols in Molecular Biology 1995 supplement; Sambrook et al., Molecular Cloning: A Laboratory Manual, 1989, Cold Spring Harbor Laboratory, New York, USA), peptide nucleic acid libraries (see, e.g., US Patent 5,539,083), antibody libraries (see, e.g., Vaughan et al., Nature Biotechnology 1996, 14(3):309-14 and PCT/US96/10287), carbohydrate libraries (see, e.g., Liang et al., Science 1996, 274: 1520-22; US Patent 5,593,853), and small organic molecule libraries (see, e.g., benzodiazepines, Gordon EM. Curr Opin Biotechnol. 1995 Dec 1;6(6):624-31.; isoprenoids, US Patent 5,569,588; thiazolidinones and metathiazanones, US Patent 5,549,974; pyrrolidines, US Patents 5,525,735 and 5,519,134; morpholino substances, US Patent 5,506,337; benzodiazepines, 5,288,514, and the like).

Materials and methods for the preparation of combinatorial libraries are commercially available (see, e.g., 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA). In addition, numerous combinatorial libraries are themselves commercially available (see, e.g., ComGenex, Princeton, N.J., Tripos, Inc., St. Louis, MO, 3D Pharmaceuticals, Exton, PA, Martek Biosciences, Columbia, MD, etc.).

### (iii) Other candidates:

Another approach uses recombinant bacteriophage to produce libraries. Using the "phage method" (Scott & Smith, Science 1990, 249: 386-90; Cwirla et al., Proc Natl Acad Sci USA 1990, 87: 6378-82; Devlin et al., Science 1990, 249: 404-6), very large libraries can be constructed (e.g., 10⁶-10⁸ chemical entities). A second approach uses primarily chemical methods, of which the Geysen method (Geysen et al., Molecular Immunology 1986, 23: 709-15; Geysen et al., J Immunologic Method 1987, 102: 259-74); and the method of Fodor et al. (Science 1991, 251: 767-73) are examples. Furka et al. (14th International Congress of Biochemistry 1988, Volume #5, Abstract FR:013; Furka, Int J Peptide Protein Res 1991, 37: 487-93), Houghten (US Patent 4,631,211) and Rutter et al. (US Patent 5,010,175) describe methods to produce a mixture of peptides that can be tested as agonists or antagonists.

Aptamers are macromolecules composed of nucleic acid that bind tightly to a specific molecular target. Tuerk and Gold (Science. 249:505-510 (1990)) discloses SELEX (Systematic Evolution of Ligands by Exponential Enrichment) method for selection of aptamers. In the SELEX method, a large library of nucleic acid molecules (e.g., 10¹⁵ different molecules) can be used for screening.

In addition to the full length of SMYD2 polypeptide, fragments of the polypeptides may be used for the present screening, so long as it the fragment utilized retains at least one biological activity of the natural occurring SMYD2 polypeptide. Such examples of biological activities contemplated by the present invention include cell proliferation enhancing activity and/or methyltransferase activity of the native SMYD2 polypeptide.

SMYD2 polypeptides or functional equivalent thereof may be further linked to other substances, so long as the polypeptides and fragments retain at least one of their biological activities. Useful substances include: peptides, lipids, sugar and sugar chains, acetyl groups, natural and synthetic polymers, etc. These kinds of modifications may be performed to confer additional functions or to stabilize the polypeptide and fragments.

### Screening For An SMYD2 Polypeptide Binding Substance:

In context of the present invention, over-expression of an SMYD2 gene was detected in bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer in spite of no expression in normal organs (Fig. 1A-3F). Furthermore, knockdown of SMYD2 by siRNAs and inactivation of SMYD2 led to inhibition cancer cell growth (Fig. 2). Due to the increased expression level of SMYD2 gene in bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer and knockdown effect of SMYD2 by siRNAs, a substance that binds to SMYD2 polypeptide is expected to suppress the proliferation of cancer cells, and thus be useful for treating or preventing cancer, including bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. Therefore, the present invention also provides a method of screening for a candidate substance that suppresses the proliferation of cancer cells, and a method of screening for a candidate substance for treating or preventing cancer, particularly bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer, using a binding activity to the SMYD2 polypeptide as an index. One particular embodiment of this screening method includes the steps of:
(a) contacting a test substance with an SMYD2 polypeptide or functional equivalent thereof;
(b) detecting the binding activity between the SMYD2 polypeptide or functional equivalent thereof and the test substance; and
(c) selecting the test substance that binds to the SMYD2 polypeptide or functional equivalent thereof as a candidate substance for treating or preventing cancer.

Alternatively, according to the present invention, the potential therapeutic effect of a test substance on treating or preventing cancer can also be evaluated or estimated. In some embodiments, the present invention provides a method for evaluating or estimating a therapeutic effect of a test substance on treating or preventing cancer or inhibiting cancer associated with over-expression of SMYD2, the method including steps of:
(a) contacting a test substance with an SMYD2 polypeptide or functional equivalent thereof;
(b) detecting the binding activity between the SMYD2 polypeptide or functional equivalent thereof and the test substance; and
(c) correlating the potential therapeutic effect of the test substance with binding activity detected in the step (b), wherein the potential therapeutic effect is shown when the test substance binds to the polypeptide or functional equivalent thereof as a candidate substance for treating or preventing cancer.

In the context of the present invention, the therapeutic effect may be correlated with the binding level of the test substance and the SMYD2 polypeptide. For example, when the test substance binds to an SMYD2 polypeptide, the test substance may identified or selected as a candidate substance having the requisite therapeutic effect. Alternatively, when the test substance does not bind to an SMYD2 polypeptide, the test substance may be identified as the substance having no significant therapeutic effect.

The screening methods of the present invention are described in more detail below. The SMYD2 polypeptide to be used for screening may be a recombinant polypeptide or a protein derived from the nature or a partial peptide thereof. The polypeptide to be contacted with a test substance can be, for example, a purified polypeptide, a soluble protein, a form bound to a carrier or a fusion protein fused with other polypeptides. In preferred embodiments, the polypeptide is isolated from cells expressing SMYD2, or chemically synthesized to be contacted with a test substance in vitro.

As a method of screening for proteins that bind to the SMYD2 polypeptide, many methods well known by a person skilled in the art can be used. Such a screening can be conducted by, for example, the immunoprecipitation method, specifically, in the following manner. The gene encoding the SMYD2 polypeptide is expressed in host (e.g., animal) cells and so on by inserting the gene to an expression vector for foreign genes, such as pSV2neo, pcDNA I, pcDNA3.1, pCAGGS and pCD8.

The promoter to be used for the expression may be any promoter that can be used commonly and include, for example, the SV40 early promoter (Rigby in Williamson (ed.), Genetic Engineering, vol. 3. Academic Press, London, 83-141 (1982)), the EF-alpha promoter (Kim et al., Gene 91: 217-23 (1990)), the CAG promoter (Niwa et al., Gene 108: 193 (1991)), the RSV LTR promoter (Cullen, Methods in Enzymology 152: 684-704 (1987)) the SR alpha promoter (Takebe et al., Mol Cell Biol 8: 466 (1988)), the CMV immediate early promoter (Seed and Aruffo, Proc Natl Acad Sci USA 84: 3365-9 (1987)), the SV40 late promoter (Gheysen and Fiers, J Mol Appl Genet 1: 385-94 (1982)), the Adenovirus late promoter (Kaufman et al., Mol Cell Biol 9: 946 (1989)), the HSV TK promoter and so on.

The introduction of the gene into host cells to express a foreign gene can be performed according to any methods, for example, the electroporation method (Chu et al., Nucleic Acids Res 15: 1311-26 (1987)), the calcium phosphate method (Chen and Okayama, Mol Cell Biol 7: 2745-52 (1987)), the DEAE dextran method (Lopata et al., Nucleic Acids Res 12: 5707-17 (1984); Sussman and Milman, Mol Cell Biol 4: 1641-3 (1984)), the Lipofectin method (Derijard B., Cell 76: 1025-37 (1994); Lamb et al., Nature Genetics 5: 22-30 (1993): Rabindran et al., Science 259: 230-4 (1993)) and so on.

The polypeptide encoded by the SMYD2 gene can be expressed as a fusion protein including a recognition site (epitope) of a monoclonal antibody by introducing the epitope of the monoclonal antibody, whose specificity has been revealed, to the N- or C- terminus of the polypeptide. Any commercially available epitope-antibody system can be used (Experimental Medicine 13: 85-90 (1995)). Vectors that can express a fusion protein with, for example, beta-galactosidase, maltose binding protein, glutathione S-transferase, green fluorescence protein (GFP) and so on by the use of its multiple cloning sites are commercially available. A fusion protein prepared by introducing only small epitopes composed of several to a dozen amino acids so as not to change the property of the SMYD2 polypeptide by the fusion is also provided herein. Epitopes, such as polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage) and such, and monoclonal antibodies recognizing them can be used as the epitope-antibody system for screening proteins binding to the SMYD2 polypeptide (Experimental Medicine 13: 85-90 (1995)).

In the context of immunoprecipitation, an immune complex is formed by adding these antibodies to cell lysate prepared using an appropriate detergent. The immune complex is composed of the SMYD2 polypeptide, a polypeptide including the binding ability with the polypeptide, and an antibody. Immunoprecipitation can be also conducted using antibodies against the SMYD2 polypeptide, besides using antibodies against the above epitopes, which antibodies can be prepared as described above. An immune complex can be precipitated, for example by Protein A sepharose or Protein G sepharose when the antibody is a mouse IgG antibody. If the polypeptide encoded by SMYD2 gene is prepared as a fusion protein with an epitope, such as GST, an immune complex can be formed in the same manner as in the use of the antibody against the SMYD2 polypeptide, using a substance specifically binding to these epitopes, such as glutathione-Sepharose 4B.

Immunoprecipitation can be performed by following or according to, for example, the methods in the literature (Harlow and Lane, Antibodies, 511-52, Cold Spring Harbor Laboratory publications, New York (1988)).

SDS-PAGE is commonly used for analysis of immunoprecipitated proteins and the bound protein can be analyzed by the molecular weight of the protein using gels with an appropriate concentration. Since the protein bound to the SMYD2 polypeptide is difficult to detect by a common staining method, such as Coomassie staining or silver staining, the detection sensitivity for the protein can be improved by culturing cells in culture medium containing radioactive isotope, ³⁵S-methionine or ³⁵S-cystein, labeling proteins in the cells, and detecting the proteins. The target protein can be purified directly from the SDS-polyacrylamide gel and its sequence can be determined, when the molecular weight of a protein has been revealed.

Alternatively, West-Western blotting analysis (Skolnik et al., Cell 65: 83-90 (1991)) can be used to screen for proteins binding to the SMYD2 polypeptide. In particular, a protein binding to the SMYD2 polypeptide can be obtained by preparing a cDNA library from cultured cells expected to express a protein binding to the SMYD2 polypeptide using a phage vector (e.g., ZAP), expressing the protein on LB-agarose, fixing the protein expressed on a filter, reacting the purified and labeled SMYD2 polypeptide with the above filter, and detecting the plaques expressing proteins bound to the SMYD2 polypeptide according to the label. The SMYD2 polypeptide may be labeled by utilizing the binding between biotin and avidin, or by utilizing an antibody that specifically binds to the SMYD2 polypeptide, or a peptide or polypeptide (for example, GST) that is fused to the SMYD2 polypeptide. Methods using radioisotope or fluorescence and such may be also used.

The terms "label" and "detectable label" are used herein to refer to any component detectable by spectroscopic, photochemical, biochemical, immunochemical, electrical, optical or chemical means. Such labels include biotin for staining with labeled streptavidin conjugate, magnetic beads (e.g., DYNABEADS™), fluorescent dyes (e.g., fluorescein, Texas red, rhodamine, green fluorescent protein, and the like), radiolabels (e.g., ³H, ¹²⁵I, ³⁵S, 14C, or ³²P), enzymes (e.g., horse radish peroxidase, alkaline phosphatase and others commonly used in an ELISA), and calorimetric labels for example colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,275,149; and 4,366,241. Means of detecting such labels are well known to those of skill in the art. Thus, for example, radiolabels can be detected using photographic film or scintillation counters, fluorescent markers can be detected using a photodetector to detect emitted light. Enzymatic labels are typically detected by providing the enzyme with a substrate and detecting, the reaction product produced by the action of the enzyme on the substrate, and calorimetric labels are detected by simply visualizing the colored label.

Alternatively, in another embodiment, the screening method of the present invention may utilize a two-hybrid cell system ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman, Cell 68: 597-612 (1992)", "Fields and Sternglanz, Trends Genet 10: 286-92 (1994)").

In the two-hybrid system, the SMYD2 polypeptide is fused to an SRF-binding region or GAL4-binding region and expressed in yeast cells. A cDNA library is prepared from cells expected to express a protein binding to the SMYD2 polypeptide, such that the library, when expressed, is fused to the VP16 or GAL4 transcriptional activation region. The cDNA library is then introduced into the above yeast cells and the cDNA derived from the library is isolated from the positive clones detected (when a protein binding to the SMYD2 polypeptide is expressed in yeast cells, the binding of the two activates a reporter gene, making positive clones detectable). A protein encoded by the cDNA can be prepared by introducing the cDNA isolated above to E. coli and expressing the protein. Examples of suitable reporter genes include, but are not limited to, Ade2 gene, lacZ gene, CAT gene, luciferase gene and such can be used in addition to the HIS3 gene.

A substance binding to the SMYD2 polypeptide can also be screened using affinity chromatography. For example, the SMYD2 polypeptide may be immobilized on a carrier of an affinity column, and a test substance, containing a protein capable of binding to the polypeptide of the invention, is applied to the column. A test substance herein may be, for example, cell extracts, cell lysates, etc. After loading a test substance, the column is washed, and substances bound to the SMYD2 polypeptide can be prepared. When the test substance is a protein, the amino acid sequence of the obtained protein is analyzed, an oligo DNA is synthesized based on the sequence, and cDNA libraries are screened using the oligo DNA as a probe to obtain a DNA encoding the protein.

A biosensor using the surface plasmon resonance phenomenon may be used as a mean for detecting or quantifying the bound substance in the present invention. When such a biosensor is used, the interaction between the SMYD2 polypeptide and a test substance can be observed real-time as a surface plasmon resonance signal, using only a minute amount of the SMYD2 polypeptide and without labeling (for example, BIAcore, Pharmacia). Therefore, it is possible to evaluate the binding between the SMYD2 polypeptide and a test substance using a biosensor such as BIAcore.

The methods of screening for molecules that bind when the immobilized SMYD2 polypeptide is exposed to synthetic chemical substances, or natural substance banks or a random phage peptide display library, and the methods of screening using high-throughput based on combinatorial chemistry techniques (Wrighton et al., Science 273: 458-64 (1996); Verdine, Nature 384: 11-13 (1996); Hogan, Nature 384: 17-9 (1996)) to isolate not only proteins but chemical substances that bind to the SMYD2 protein (including agonist and antagonist) are well known to those skilled in the art.

In addition to the full length of an SMYD2 polypeptide, fragment of the SMYD2 polypeptide may be used for the present screening method, so long as the fragment retains at least one biological activity of the naturally occurring SMYD2 polypeptide. Such biological activities include cell proliferation promoting activity and methyltransferase activity, and so on.

SMYD2 polypeptides or fragment thereof may be further linked to other substances, so long as the polypeptide or functional equivalent retains at least one biological activity. Usable substances include: peptides, lipids, sugar and sugar chains, acetyl groups, natural and synthetic polymers, etc. These kinds of modifications may be performed to confer additional functions or to stabilize the polypeptide or functional equivalent.

SMYD2 polypeptides or functional equivalents used for the present method may be obtained from nature as naturally occurring proteins via conventional purification methods or through chemical synthesis based on the selected amino acid sequence. For example, conventional peptide synthesis methods that can be adopted for the synthesis include:
1) Peptide Synthesis, Interscience, New York, 1966;
2) The Proteins, Vol. 2, Academic Press, New York, 1976;
3) Peptide Synthesis (in Japanese), Maruzen Co., 1975;
4) Basics and Experiment of Peptide Synthesis (in Japanese), Maruzen Co., 1985;
5) Development of Pharmaceuticals (second volume) (in Japanese), Vol. 14 (peptide synthesis), Hirokawa, 1991;
6) WO99/67288; and
7) Barany G. & Merrifield R.B., Peptides Vol. 2, "Solid Phase Peptide Synthesis", Academic Press, New York, 1980, 100-118.

Alternatively, SMYD2 polypeptides may be obtained through any known genetic engineering methods for producing polypeptides (e.g., Morrison J., J Bacteriology 1977, 132: 349-51; Clark-Curtiss & Curtiss, Methods in Enzymology (eds. Wu et al.) 1983, 101: 347-62). For example, first, a suitable vector including a polynucleotide encoding the objective protein in an expressible form (e.g., downstream of a regulatory sequence including a promoter) is prepared, transformed into a suitable host cell, and then the host cell is cultured to produce the protein. More specifically, a gene encoding the SMYD2 polypeptide is expressed in host (e.g., animal) cells and such by inserting the gene into a vector for expressing foreign genes, such as pSV2neo, pcDNA I, pcDNA3.1, pCAGGS, or pCD8. A promoter may be used for the expression. Any commonly used promoters may be employed including, for example, the SV40 early promoter (Rigby in Williamson (ed.), Genetic Engineering, vol. 3. Academic Press, London, 1982, 83-141), the EF-alpha promoter (Kim et al., Gene 1990, 91:217-23), the CAG promoter (Niwa et al., Gene 1991, 108:193), the RSV LTR promoter (Cullen, Methods in Enzymology 1987, 152:684-704), the SR alpha promoter (Takebe et al., Mol Cell Biol 1988, 8:466), the CMV immediate early promoter (Seed et al., Proc Natl Acad Sci USA 1987, 84:3365-9), the SV40 late promoter (Gheysen et al., J Mol Appl Genet 1982, 1:385-94), the Adenovirus late promoter (Kaufman et al., Mol Cell Biol 1989, 9:946), the HSV TK promoter, and such. The introduction of the vector into host cells to express the SMYD2 gene can be performed according to any methods, for example, the electroporation method (Chu et al., Nucleic Acids Res 1987, 15:1311-26), the calcium phosphate method (Chen et al., Mol Cell Biol 1987, 7:2745-52), the DEAE dextran method (Lopata et al., Nucleic Acids Res 1984, 12:5707-17; Sussman et al., Mol Cell Biol 1985, 4:1641-3), the Lipofectin method (Derijard B, Cell 1994, 7:1025-37; Lamb et al., Nature Genetics 1993, 5:22-30; Rabindran et al., Science 1993, 259:230-4), and such.

The SMYD2 polypeptide may also be produced in vitro adopting an in vitro translation system.

The SMYD2 polypeptide to be contacted with a test substance can be, for example, a purified polypeptide, a soluble protein, or a fusion protein fused with other polypeptides.

Test substances screened by the present method as substances that bind to SMYD2 polypeptide can be candidate substances that have the potential to treat or prevent cancers. Potential of these candidate substances to treat or prevent cancers may be evaluated by second and/or further screening to further identify or confirm the therapeutic efficacy of the substance for cancers. For example, these candidate substances may be further examined for suppression of cancer cell proliferation by contacting the substance with a cancer cell over-expressing the SMYD2 gene.

### Screening For A Substance That Suppresses The Biological Activity Of SMYD2:

In the course of the present invention, the SMYD2 gene was revealed to be specifically and significantly over-expressed in bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer (Fig.1D, F). Furthermore, the suppression of the SMYD2 gene by small interfering RNA (siRNA) resulted in growth inhibition and/or cell death of cancer cells (Fig. 2A, B, D, E, F). Moreover, an inactivated SMYD2 protein reduced colony formation activity (Fig. 2C).

Furthermore, the substitutions of methylation sites for SMYD2 to alanines in HSP90AB1 and RB1, identified as novel substrates for SMYD2 in the course of the present invention, diminished the growth promoting effect of HSP90AB1 (Fig. 5H) and RB1 (Fig. 12).

These results clearly demonstrates that the SMYD2 polypeptide is involved in cancer cell survival, and thus, substances that inhibit a biological activity of the SMYD2 polypeptide may serve as suitable candidate substances for cancer therapy.

Thus, the present invention also provides a method for screening for a candidate substance for either or both of treating and preventing cancer using a biological activity of the SMYD2 polypeptide as an index.

Exemplary cancers include bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

Specifically, the present invention provides the following methods:
A method of screening for a candidate substance for either or both of treating and preventing cancer, including steps of:
   (a) contacting a test substance with an SMYD2 polynucleotide or a functional equivalent thereof;
   (b) detecting a biological activity of the SMYD2 polypeptide or a functional equivalent thereof of step (a);
   (c) comparing the biological activity detected in step (b) with that detected in the absence of the test substance;
   (d) selecting the test substance that reduces or inhibits the biological activity of the SMYD2 polypeptide or functional equivalent thereof.

In the context of the present invention, the therapeutic effect of the test substance on suppressing the biological activity (e.g., the cell-proliferation promoting activity or the methyltransferase activity) of SMYD2 polypeptide or a candidate substance for either or both of treating and preventing cancer may be evaluated. Therefore, the present invention also provides a method of screening for a candidate substance for suppressing the biological activity of the SMYD2 polypeptide, or a candidate substance for either or both of treating and preventing cancer, using the SMYD2 polypeptide or functional equivalent thereof, including the following steps:
(a) contacting a test substance with the SMYD2 polypeptide or a functional equivalent thereof; and
(b) detecting the biological activity of the polypeptide or a functional equivalent thereof of step (a), and
(c) correlating the biological activity of (b) with the therapeutic effect of the test substance.

In the context of the present invention, the therapeutic effect may be correlated with the biological activity of the SMYD2 polypeptide or a functional equivalent thereof. For example, when the test substance suppresses or inhibits the biological activity of the SMYD2 polypeptide or a functional equivalent thereof as compared to a level detected in the absence of the test substance, the test substance may identified or selected as the candidate substance having the therapeutic effect. Alternatively, when the test substance does not suppress or inhibit the biological activity of the SMYD2 polypeptide or a functional equivalent thereof as compared to a level detected in the absence of the test substance, the test substance may be identified as the substance having no significant therapeutic effect.

Alternatively, in some embodiments, the present invention provides a method for evaluating or estimating a therapeutic effect of a test substance on either or both of treating and preventing cancer or inhibiting cancer associated with over-expression of the SMYD2 gene, the method including steps of:
(a) contacting a test substance with an SMYD2 polypeptide or a functional equivalent thereof;
(b) detecting the biological activity of the polypeptide or functional equivalent thereof of step (a); and
(c) correlating the potential therapeutic effect and the test substance, wherein the potential therapeutic effect is shown, when a substance suppresses the biological activity of the SMYD2 polypeptide or functional equivalent thereof as compared to the biological activity of the polypeptide detected in the absence of the test substance.

In the context of expression, binding and biological activity, the term "suppress" is used interchangeably with the terms "reduce" and "inhibit" to encompass effects ranging from partial to full. Accordingly, the phrase "suppress the biological activity" as defined herein refers to at least 10% suppression of the biological activity of SMYD2 in comparison with in absence of the substance, more preferably at least 25%, 50% or 75% suppression and most preferably at 90% suppression.

As described above, examples of cancers contemplated by the present invention include, but are not limited to, bladder cancer, lung cancer, breast cancer, cervix cancer, colon cancer, kidney cancer, liver cancer, head and neck cancer, seminoma, cutaneous cancer, pancreatic cancer , lymphoma, ovarian cancer, leukemia and prostate cancer.

In the context of the present invention, the therapeutic effect may be correlated with the biological activity of the SMYD2 polypeptide or functional equivalent thereof. For example, when the test substance suppresses or inhibits the biological activity of the SMYD2 polypeptide or functional equivalent thereof as compared to a level detected in the absence of the test substance, the test substance may identified or selected as the candidate substance having the therapeutic effect. Alternatively, when the test substance does not suppress or inhibit the biological activity of the SMYD2 polypeptide or functional equivalent thereof as compared to a level detected in the absence of the test substance, the test substance may identified as the substance having no significant therapeutic effect.

The screening methods of the present invention are described in more detail below. Any polypeptides can be used for the screening methods of the present invention, so long as they retain at least one biological activity of the SMYD2 polypeptide. Examples of such biological activities include cell proliferation enhancing activity and methyltransferase activity of the SMYD2 polypeptide. For example, SMYD2 polypeptide can be used and polypeptides functionally equivalent to the SMYD2 polypeptide can also be used. Such polypeptides may be expressed endogenously or exogenously by cells. For details of the functional equivalent of the SMYD2 polypeptide, see the item "Genes and proteins". For example, fragments of an SMYD2 polypeptide retaining the SET domain, such as fragments containing the amino acid sequence from the 17th to 247th amino acid of SEQ ID NO: 63, can be functional equivalents of the SMYD2 polypeptide.

Substances isolated by the screening methods of the present invention are deemed to be a candidate antagonists (inhibitors) of the SMYD2 polypeptide. The term "antagonist" refers to molecules that inhibit the function of the polypeptide by binding thereto. The term also refers to molecules that reduce or inhibit expression of the gene encoding the SMYD2 polypeptide. Moreover, a substance isolated by this screening is a candidate for substances which inhibit the in vivo interaction of the SMYD2 polypeptide with molecules (including DNAs and proteins).

When the biological activity to be detected in the present method is cell proliferation promoting activity, it can be detected, for example, by preparing cells which express the SMYD2 polypeptide, culturing the cells in the presence of a test substance, and determining the speed of cell proliferation, measuring the cell cycle and such, as well as by measuring survival cells or the colony forming activity, e.g. by MTT assay, colony formation assay or FACS.

The substances that reduce the speed of proliferation of the cells expressed SMYD2 are selected as candidate substances for treating or preventing cancer, such as bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. In some embodiments, cells expressing SMYD2 gene are isolated and cultured cells exogenously or endogenously expressing SMYD2 gene in vitro.

More specifically, the method includes the step of:
(a) contacting a test substance with cells over-expressing SMYD2 gene;
(b) measuring cell-proliferation promoting activity; and
(c) selecting the test substance that reduces the cell proliferation promoting activity in the comparison with the cell proliferation promoting activity in the absence of the test substance.

In preferable embodiments, the method of the present invention may further include the step of:
(d) selecting the test substance that has no effect to the cells no or little expressing SMYD2 gene.

When the biological activity to be detected in the present method is methyltransferase activity, the methyltransferase activity can be determined by contacting a SMYD2 polypeptide with a substrate (e.g., histone H4 protein or fragment thereof (e.g., SEQ ID NO: 66), histone H3 protein or fragment thereof, HSP90AB1 protein or fragment thereof containing the lysine 531 and/or lysine 574 of SEQ ID NO: 65, RB1 protein or fragment thereof containing the lysine 810 of SEQ ID NO: 68 (e.g., SEQ ID NO: 69)) and a co-factor (e.g., S-adenosyl-L-methionine, S-adenosyl-L-[methyl-³ H]methionine, or L-[methyl-³H] methionine) under a condition suitable for methylation of the substrate and detecting the methylation level of the substrate.

More specifically, the present invention provides following methods [1] to [10]:
[1] The method of screening for a candidate substance for either or both of treating and preventing cancer, wherein the method includes the steps of:
   (a) contacting a test substance with an SMYD2 polypeptide or functional equivalent thereof, a substrate and a co-factor under a condition suitable for methylation of the substrate;
   (b) detecting the methylation level of the substrate; and
   (c) selecting the test substance that reduces the methylation level of the substrate in the comparison with the methylation level in the absence of the test substance;
[2] The method of [1], wherein the substrate is a histone protein or fragment thereof including at least one methylation site;
[3] The method of [2], wherein .the histone is a histone H4 or a histone H3;
[4] The method of [1], wherein the substrate is an HSP90AB1 polypeptide or a fragment thereof including at least one methylation site;
[5] The method of [4], wherein the methylation site is the lysine 531 and/or lysine 574 of HSP90AB1 polypeptide (SEQ ID NO: 65).
[6] The method of [1], wherein the substrate is an RB1 polypeptide or a fragment thereof including at least one methylation site;
[7] The method of [6], wherein the methylation site is the lysine 810 of RB1 polypeptide (SEQ ID NO: 68).
[8] The method of any one of [1] to [7], wherein the cofactor is an S-adenosyl methionine;
[9] The method of any one of [1] to [8], wherein the polypeptide is contacted with the substrate and cofactor in the presence of an enhancing agent for the methylation; and
[10] The method of [9], wherein the enhancing agent for the methylation is S-adenosyl homocysteine hydrolase (SAHH).

In the context of the present invention, methyltransferase activity of an SMYD2 polypeptide can be determined by methods known in the art (See, for example, Brown et al, Mol Cancer 2006;5:26, Huang et al, Nature 2006;444:629-632, Saddic et al, J Biol Chem 2010;285:37733-37740).

For example, the SMYD2 polypeptide and a substrate can be incubated with a labeled methyl donor, under a suitable assay condition. Histone H4 peptides (i.e., histone H4 protein or fragment thereof (e.g., SEQ ID NO: 66)), histone H3 peptides (i.e., histone H3 protein or fragment thereof), HSP90AB1 peptides (i.e., HSP90AB1 polypeptide or fragment thereof), or RB1 peptides (i.e., RB1 polypeptide or fragment thereof (e.g., SEQ ID NO: 69)) as the substrates, and a labeled S-adenosyl-L-methionine (such as S-adenosyl-[methyl-¹⁴C]-L-methionine, S-adenosyl-[methyl-³H]-L-methionine and L-[methyl-³H] methionine) as the methyl donor preferably can be used, respectively. Transfer of the radiolabel to the substrate (e.g., the histone H4 peptides, the histone H3 peptides, the HSP90AB1 peptides, or the RB1 peptides) can be detected, for example, by SDS-PAGE electrophoresis and fluorography. Alternatively, following the reaction, the substrate can be separated from the methyl donor by filtration, and the amount of radiolabel retained on the filter quantitated by scintillation counting. Other suitable labels that can be attached to methyl donors, such as chromogenic and fluorescent labels, and methods of detecting transfer of these labels to substrates are known in the art. An example of the methyltransferase assay will be described in "Example 6: Screening for inhibitors of methyltransferase activity of SMYD2".

Alternatively, the methyltransferase activity of the SMYD2 polypeptide can be determined using an unlabeled methyl donor (e.g., S-adenosyl-L-methionine) and reagents that selectively recognize a methylated substrate (e.g., histone H4 peptide, histone H3 peptide, HSP90AB1 peptide, RB1 peptide, etc.). For example, after incubation of the SMYD2 polypeptide, a substrate to be methylated and a methyl donor, under a condition capable of methylation of the substrate, the methylated substrate can be detected by an immunological method. Any immunological techniques using an antibody recognizing a methylated substrate can be used for the detection. For example, an antibody against a methylated histone is commercially available (abcam Ltd.). ELISA or Immunoblotting with antibodies recognizing methylated substrates can be used for the present invention.

In the context of the present invention, the histone H4 or fragment thereof (e.g., SEQ ID NO: 66), the histone H3 protein or fragment thereof, or the HSP90AB1 polypeptide or fragment thereof, or the RB1 polypeptide or fragment thereof (e.g., SEQ ID NO: 69) can be preferably used as a substrate to be methylated by the SMYD2 polypeptide. The histone H3 fragment to be used as a substrate preferably retains the lysine 36. The histone H4 fragment to be used as a substrate preferably retains the lysine 20. The HSP90AB1 fragment to be used as a substrate preferably retains the lysine 531 and/or lysine 574. The RB1 fragment to be used as a substrate preferably retains the lysine 810. Such histone H4 fragment, histone H3 fragment, HSP90AB1 fragment or RB1 fragment is composed of preferably at least 10 amino acid residues, more preferably at least 15 amino acid residues, and further more preferably at least 20 amino acid residues. An example of such histone H4 fragment includes a peptide having the amino acid sequence of SEQ ID NO: 66. An example of such HSP90AB1 fragment includes a peptide containing the amino acid sequence from the 500th to 724th amino acid of SEQ ID NO: 65. An example of such RB1 fragment includes a peptide containing the amino acid sequence of SEQ ID NO: 69, preferably a peptide containing the amino acid sequence from the 773th to 813th amino acid of SEQ ID NO: 68. Alternatively, a modified peptide of the histone H3 or fragment thereof a modified peptide of the histone H4 or fragment thereof, a modified peptide of the HSP90AB1 or fragment thereof, or a modified peptide of the RB1 or fragment thereof may be used for which the methyltransferase has increased affinity/activity. Such peptides can be designed by exchanging and/or adding and/or deleting amino acids and testing the substrate in serial experiments for methyltransferase assay using the SMYD2 polypeptide.

In the present invention, any functional equivalent of the SMYD2 polypeptide can be used so long as such it retains the methyltransferase activity of the original (native, wild-type) SMYD2 polypeptide. To that end, the functional equivalent of the SMYD2 polypeptide preferably retains a SET-domain of the SMYD2 polypeptide (e.g., 17-247 of SEQ ID NO: 63). An example of such functional equivalent includes a polypeptide having an amino acid sequence from the 1st to 250th amino acid of SEQ ID NO: 63 for histone H3, H4 and HSP90AB1, and a polypeptide retaining an amino acid sequence from the 1st to 250th amino acid of SEQ ID NO: 63 and an amino acid sequence from the 330th to 433th amino acid of SEQ ID NO: 63.

The SMYD2 polypeptide or functional equivalent thereof may be expressed as a fusion protein including a recognition site (epitope) of a monoclonal antibody by introducing the epitope of the monoclonal antibody, whose specificity has been revealed, to the N- or C- terminus of the polypeptide. A commercially available epitope-antibody system can be used (Experimental Medicine 13: 85-90 (1995)). Vectors which can express a fusion protein with, for example, beta-galactosidase, maltose binding protein, glutathione S-transferase (GST), green fluorescence protein (GFP) and so on by the use of its multiple cloning sites are commercially available. Also, a fusion protein prepared by introducing only small epitopes consisting of several to a dozen amino acids so as not to change the property of the SMYD2 polypeptide by the fusion is also reported. Epitopes, such as polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage) and such.

The present invention contemplates the use of an agent that enhances the methylation of the substance. A preferred enhancing agent for methylation is SAHH or a functional equivalent thereof. Such agents enhance the methylation of the substance and thereby enable determination of the methyltransferase activity with higher sensitivity thereby. Accordingly, SMYD2 may be contacted with substrate and cofactor under the existence of the enhancing agent. In one embodiment, the SMYD2 polypeptide and the substrate are isolated from cells expressing SMYD2 and the substrate, or chemically synthesized to be contacted with a test substance in vitro.

Methyltransferase activity can also be detected by preparing cells that express the SMYD2 polypeptide, culturing the cells in the presence of a test substance, and determining the methylation level of a histone, HSP90AB1 or RB1, for example, by using the antibody specific binding to methylation site thereof.

More specifically, the method includes the steps of:
[1] contacting a test substance with cells expressing the SMYD2 gene;
[2] detecting a methylation level of the lysine 20 histone H4 protein, the lysine 36 of histone H3 protein (H3K9), the lysine 531 and/or lysine 574 of HSP90AB1 protein or the lysine 810 of RB1 protein; and
[3] selecting the test substance that reduces the methylation level in the comparison with the methylation level in the absence of the test substance.

As noted above, the phrase "suppress the biological activity" is defined herein as preferably at least 10% suppression of the biological activity of the SMYD2 polypeptide in comparison with in absence of the substance, more preferably at least 25%, 50% or 75% suppression and most preferably at 90% suppression. Accordingly, a test substance may be characterized as "reducing the methylation level" if it provides a reduction on the order of 10%, more preferably at least 25%, 50% or 75% reduction and most preferably at 90% reduction.

In the preferred embodiments, control cells which do not express the SMYD2 gene are used. Accordingly, the present invention also provides a method of screening for a candidate substance for inhibiting the cell growth or a candidate substance for either or both of treating and preventing SMYD2 gene associating disease, using the SMYD2 polypeptide or functional equivalents thereof including the steps as follows:
(a) culturing cells which express an SMYD2 polypeptide or a functional equivalent thereof in the presence or absence of a test substance, and control cells that do not express an SMYD2 polypeptide or a functional equivalent thereof in the presence of the test substance;
(b) detecting a biological activity (e.g., cell growth) of the cells which express the SMYD2 polypeptide or functional equivalent thereof and control cells; and
(c) selecting the test substance that inhibits the biological activity of the cells which express the SMYD2 polypeptide or functional equivalent thereof as compared to the biological activity detected in the absence of said test substance and that does not inhibit the biological activity of the control cells.

As revealed herein, suppressing a biological activity of the SMYD2 polypeptide reduces cell growth. Thus, by screening for a substance that inhibits a biological activity of the SMYD2 polypeptide, a candidate substance that have the potential to treat or prevent cancers can be identified. The potential of these candidate substances to treat or prevent cancers may be evaluated by second and/or further screening to identify therapeutic substance, compounds or agent for cancers. For example, when a substance that inhibits the biological activity of an SMYD2 polypeptide also inhibits the activity of a cancer, it may be concluded that such a substance has an SMYD2 specific therapeutic effect.

### Screening For A Substance That Alters The Expression Of SMYD2:

As demonstrated herein, a decrease in the expression of SMYD2 gene by siRNA results in the inhibition of cancer cell proliferation (Fig. 2). Thus, it is herein revealed that suppressing (reducing, inhibiting) the expression of the SMYD2 gene suppresses (reduces, inhibits) cell growth. Thus, by screening for a candidate substance that reduces the expression or activity of the reporter gene, a candidate substance that has the potential to treat or prevent cancers can be identified. Potential of these candidate substances to treat or prevent cancers may be evaluated by second and/or further screening to identify therapeutic substance for cancers.

Accordingly, the present invention provides a method of screening for a substance that inhibits the expression of SMYD2 gene. A substance that inhibits the expression of SMYD2 gene is expected to suppress the proliferation of cancer cells, and thus is useful for treating or preventing cancer relating to SMYD2 gene, particularly wherein the cancer is bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

Therefore, the present invention also provides a method for screening a candidate substance that suppresses the proliferation of cancer cells, and a method for screening a candidate substance for treating or preventing cancer relating to SMYD2 gene, wherein the cancer is bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

In the context of the present invention, such screening method may include, for example, the following steps:
(a) contacting a test substance with a cell expressing an SMYD2 gene;
(b) detecting the expression level of the SMYD2 gene in the cell; and
(c) selecting the test substance that reduces the expression level of the SMYD2 gene as compared to the expression level detected in the absence of the test substance.

Alternatively, in some embodiments, the present invention also provides a method for evaluating or estimating a therapeutic effect of a test substance on treating or preventing cancer or inhibiting cancer associated with over-expression of an SMYD2 gene, the method including steps of:
(a) contacting a test substance with a cell expressing an SMYD2 gene;
(b) detecting the expression level of the SMYD2 gene in the cell; and;
(c) correlating the potential therapeutic effect and the test substance, wherein the potential therapeutic effect is shown, when a test substance reduces the expression level of an SMYD2 gene as compared to the expression level detected in the absence of the test substance.

The screening methods of the present invention are described in more detail below. Cells expressing the SMYD2 gene include, for example, cell lines established from bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer, or cell lines transfected with SMYD2 gene expression vectors; any of such cells can be used for the above screening method of the present invention. The expression level of the SMYD2 gene can be estimated by methods well known to one skilled in the art, for example, RT-PCR, Northern blot assay, Western blot assay, immunostaining and flow cytometry analysis. In the context of the present invention, the phrase "reduce the expression level" is defined as preferably at least 10% reduction of expression level of SMYD2 gene in comparison to the expression level in absence of the substance, more preferably at least 25%, 50% or 75% reduced level and most preferably at 95% reduced level. The substance herein includes chemical substance, double-strand nucleotide, and so on. The preparation of the double-strand nucleotide is in aforementioned description. In the course of the method of screening, a substance that reduces the expression level of SMYD2 gene can be selected as candidate substances to be used for the treatment or prevention of cancer, such as bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer. In some embodiments, cells expressing SMYD2 gene are isolated and cultured cells exogenously or endogenously expressing SMYD2 gene in vitro.

Alternatively, the screening method of the present invention may include the following steps:
(a) contacting a test substance with a cell into which a vector, including the transcriptional regulatory region of SMYD2 gene and a reporter gene that is expressed under the control of the transcriptional regulatory region, has been introduced;
(b) measuring the expression or activity level of the reporter gene; and
(c) selecting the candidate substance that reduces the expression or activity level of the reporter gene.

In the context of the present invention, the therapeutic effect of the test substance on inhibiting the cell growth or a candidate substance for treating or preventing SMYD2 gene associating disease may be evaluated. Therefore, the present invention also provides a method of screening for a candidate substance that suppresses the proliferation of cancer cells, and a method of screening for a candidate substance for treating or preventing an SMYD2 gene associated disease.

Alternatively, in some embodiments, the present invention also provides a method for evaluating or estimating a therapeutic effect of a test substance on treating or preventing cancer or inhibiting cancer associated with over-expression of SMYD2, the method including steps of:
(a) contacting a test substance with a cell into which a vector, including the transcriptional regulatory region of SMYD2 gene and a reporter gene that is expressed under the control of the transcriptional regulatory region, has been introduced;
(b) measuring the expression or activity level of the reporter gene; and
(c) correlating the potential therapeutic effect and the test substance, wherein the potential therapeutic effect is shown, when a test substance reduces the expression or activity level of the reporter gene.

In the context of the present invention, such screening method may include, for example, the following steps:
(a) contacting a test substance with a cell into which a vector, composed of the transcriptional regulatory region of the SMYD2 gene and a reporter gene that is expressed under the control of the transcriptional regulatory region, has been introduced;
(b) detecting the expression or activity level of the reporter gene; and
(c) correlating the expression or activity level of (b) with the therapeutic effect of the test substance.

In the context of the present invention, the therapeutic effect may be correlated with the expression or activity level of the reporter gene. For example, when the test substance reduces the expression or activity level of the reporter gene as compared to a level detected in the absence of the test substance, the test substance may identified or selected as the candidate substance having the therapeutic effect. Alternatively, when the test substance does not reduce the expression or activity level of the reporter gene as compared to a level detected in the absence of the test substance, the test substance may identified as the substance having no significant therapeutic effect.

Suitable reporter genes and host cells are well known in the art. Illustrative reporter genes include, but are not limited to, luciferase, green fluorescence protein (GFP), Discosoma sp. Red Fluorescent Protein (DsRed), Chrolamphenicol Acetyltransferase (CAT), lacZ and beta-glucuronidase (GUS), and host cell is COS7, HEK293, HeLa and so on. The reporter construct required for the screening can be prepared by connecting reporter gene sequence to the transcriptional regulatory region of SMYD2. The transcriptional regulatory region of SMYD2 herein includes the region from transcriptional start site to at least 500bp upstream, preferably 1000bp, more preferably 5000 or 10000bp upstream. A nucleotide segment containing the transcriptional regulatory region can be isolated from a genome library or can be propagated by PCR. The reporter construct required for the screening can be prepared by connecting reporter gene sequence to the transcriptional regulatory region of any one of these genes. Methods for identifying a transcriptional regulatory region, and also assay protocol are well known (Molecular Cloning third edition chapter 17, 2001, Cold Springs Harbor Laboratory Press).

The vector containing the reporter construct is infected to host cells and the expression or activity of the reporter gene is detected by method well known in the art (e.g., using luminometer, absorption spectrometer, flow cytometer and so on). "reduces the expression or activity" as defined herein are preferably at least 10% reduction of the expression or activity of the reporter gene in comparison with in absence of the substance, more preferably at least 25%, 50% or 75% reduction and most preferably at 95% reduction. In some embodiments, the cells are isolated and cultured cells into which a vector, composed of the transcriptional regulatory region of the SMYD2 gene and a reporter gene that is expressed under the control of the transcriptional regulatory region, has been introduced in vitro.

### Screening Using The Binding Of SMYD2 And Either HSP90AB1 Or RB1 As An Index:

As demonstrated herein, the direct interaction of SMYD2 with HSP90AB1 protein was shown by pull-down assay (Fig.3B, C). Pull-down of SMYD2 protein was carried out using anti-Flag antibody and incubated mixture of HA-tagged HSP90AB1 and Flag-tagged SMYD2 proteins. SMYD2 -binding HSP90AB1 protein was detected by subsequent western blotting using antibody to HSP90AB1 protein. Accordingly, the present invention provides a method of screening for a substance that inhibits the binding between SMYD2 protein and HSP90AB1 protein.

Furthermore, in the course of the present invention, the direct interaction of SMYD2 with RB1 protein was shown by pull-down assay (Fig.6B, C). Pull-down of SMYD2 protein was carried out using anti-Flag antibody and incubated mixture of HA-tagged RB1 and Flag-tagged SMYD2 proteins. SMYD2 -binding RB1 protein was detected by subsequent western blotting using anti- HA antibody. Accordingly, the present invention provides a method of screening for a substance that inhibits the binding between SMYD2 protein and RB1 protein.

Substances that inhibit the binding between SMYD2 protein and HSP90AB1 protein or RB1 protein can be screened by detecting a binding level between SMYD2 protein and HSP90AB1 protein or RB1 protein as an index. Accordingly, the present invention provides a method of screening for a substance for inhibiting the binding between SMYD2 protein and HSP90AB1 protein or RB1 protein using a binding level between SMYD2 protein and HSP90AB1 protein or RB1 protein as an index.

Furthermore, in the course of the present invention, it is revealed that methylations of HSP90AB1 protein and RB 1 protein by SMYD2 protein are involved in cancer cell growth (Fig. 5H, Fig. 12). Accordingly, substances that inhibit the interaction between SMYD2 protein and HSP90AB1 protein or RB1 protein are expected to be suppressing cancer cell proliferation through suppressing methylation of HSP90AB1 protein or RB1 protein by SMYD2 protein. Accordingly, the present invention also provides a method of screening for a candidate substance for inhibiting or reducing a growth of cancer cells expressing SMYD2 gene, e.g., bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer, and therefore, a candidate substance for treating or preventing cancers, e.g. bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer.

Further, substances obtained by the present screening method may be also useful for inhibiting cell proliferation.

Of particular interest to the present invention are the following methods of [1] to [7]:
[1] A method of screening for a substance that interrupts a binding between an SMYD2 polypeptide and an HSP90AB1 polypeptide or an RB1 polypeptide, the method including the steps of:
   (a) contacting an SMYD2 polypeptide or functional equivalent thereof with an HSP90AB1 polypeptide or functional equivalent thereof or an RB1 polypeptide or functional equivalent thereof in the presence of a test substance;
   (b) detecting a binding level between the polypeptides;
   (c) comparing the binding level detected in the step (b) with those detected in the absence of the test substance; and
   (d) selecting the test substance that reduce the binding level.
[2] A method of screening for a candidate substance useful in the treatment and/or prevention of cancer or the inhibition of cancer cell growth, the method including the steps of:
   (a) contacting an SMYD2 polypeptide or functional equivalent thereof with an HSP90AB1 polypeptide or functional equivalent thereof or an RB1 polypeptide or functional equivalent thereof in the presence of a test substance;
   (b) detecting a binding level between the polypeptides;
   (c) comparing the binding level detected in the step (b) with those detected in the absence of the test substance; and
   (d) selecting the test substance that reduce the binding level.
[3] The method of [1] or [2], wherein the functional equivalent of SMYD2 polypeptide including the HSP90AB1 binding domain of the SMYD2 polypeptide.
[4] The method of [1] or [2], wherein the functional equivalent of HSP90AB1 polypeptide including the SMYD2-binding domain of the HSP90AB1 polypepide.
[5] The method of [1] or [2], wherein the functional equivalent of SMYD2 polypeptide including the RB1 binding domain of the SMYD2 polypeptide.
[6] The method of [1] or [2], wherein the functional equivalent of RB1 polypeptide including the SMYD2-binding domain of the RB 1 polypeptide.
[7] The method of any one of [1] to [6], wherein the cancer is selected from the group consisting of bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

Alternatively, in some embodiments, the present invention also provides a method for evaluating or estimating a therapeutic effect of a test substance on treating or preventing cancer or inhibiting cancer, the method including steps of:
(a) contacting an SMYD2 polypeptide or functional equivalent thereof with an HSP90AB1 polypeptide or functional equivalent thereof or an RB1 polypeptide or functional equivalent thereof in the presence of a test substance;
(b) detecting a binding level between the polypeptides;
(c) comparing the binding level detected in the step (b) with those detected in the absence of the test substance; and
(d) correlating the potential therapeutic effect and the test substance, wherein the potential therapeutic effect is shown, when a test substance reduce the binding level.

Further, in another embodiment, the present invention also provides a method for evaluating or estimating a therapeutic effect of a test substance on treating or preventing cancer or inhibiting cancer, the method including steps of:
(a) contacting a polypeptide comprising an HSP90AB1-binding domain of an SMYD2 polypeptide with a polypeptide comprising an SMYD2-binding domain of an HSP90AB1 polypeptide in the presence of a test substance;
(b) detecting binding between the polypeptides; and
(c) correlating the potential therapeutic effect and the test substance, wherein the potential therapeutic effect is shown, when a test substance inhibits binding between the polypeptides.

In the context of the present invention, functional equivalents of an SMYD2 polypeptide and HSP90AB1 polypeptide are polypeptides that have a biological activity equivalent to an SMYD2 polypeptide (SEQ ID NO: 63), HSP90AB1 polypeptide (SEQ ID NO: 65), respectively. Particularly, the functional equivalent of SMYD2 polypeptide is a fragment of an SMYD2 polypeptide containing the binding domain to an HSP90AB1 polypeptide. In preferred embodiments, the functional equivalent of the SMYD2 polypeptide is a fragment of an SMYD2 polypeptide containing the amino acid sequence of the amino acid position 100-247 of SEQ ID NO: 63. Similarly, the functional equivalent of HSP90AB1 polypeptide is a fragment of HSP90AB1 polypeptide containing the SMYD2-binding domain. In preferred embodiments, the functional equivalent of the HSP90AB1 polypeptide is a fragment of HSP90AB1 polypeptide containing the amino acid sequence of the amino acid position 500-724 of SEQ ID NO; 65.

Further, in another embodiment, the present invention also provides a method for evaluating or estimating a therapeutic effect of a test substance on treating or preventing cancer or inhibiting cancer, the method including steps of:
(a) contacting a polypeptide comprising an RB1-binding domain of an SMYD2 polypeptide with a polypeptide comprising an SMYD2-binding domain of an RB1 polypeptide in the presence of a test substance;
(b) detecting binding between the polypeptides; and
(c) correlating the potential therapeutic effect and the test substance, wherein the potential therapeutic effect is shown, when a test substance inhibits binding between the polypeptides.

In the context of the present invention, functional equivalents of an SMYD2 polypeptide and RB1 polypeptide are polypeptides that have a biological activity equivalent to an SMYD2 polypeptide (SEQ ID NO: 63) or RB1 polypeptide (SEQ ID NO: 68), respectively. Particularly, the functional equivalent of SMYD2 is a fragment of SMYD2 polypeptide containing the binding domain to an RB1 polypeptide. In preferred embodiments, the functional equivalent of the SMYD2 polypeptide is a fragment of SMYD2 containing the amino acid sequence of the amino acid position 330-443 of SEQ ID NO: 63. Similarly, the functional equivalent of RB1 polypeptide is a fragment of RB1 polypeptide containing the SMYD2 -binding domain. In preferred embodiments, the functional equivalent of the RB1 polypeptide is a fragment of RB1 polypeptide containing the amino acid sequence of the amino acid position 773-813 of SEQ ID NO; 68.

As a method of screening for substances that inhibits the binding of SMYD2 polypeptide to HSP90AB1 polypeptide or RB1 polypeptide, many methods well known by one skilled in the art can be used.

A polypeptide to be used for screening can be a recombinant polypeptide or a protein derived from natural sources, or a partial peptide thereof. In preferred embodiments, the polypeptides are isolated from cells expressing SMYD2, HSP90AB1, or RB1, or chemically synthesized to be contacted with a test substance in vitro. Any test substance aforementioned can be used for screening.

As a method of detecting the binding between an SMYD2 protein and HSP90AB1 protein or RB1 protein, any methods well known by a person skilled in the art can be used. Such a detection can be conducted using, for example, an immunoprecipitation, West-Western blotting analysis (Skolnik et al., Cell 65: 83-90 (1991)), a two-hybrid system utilizing cells ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman, Cell 68: 597-612 (1992)", "Fields and Sternglanz, Trends Genet 10: 286-92 (1994)"), affinity chromatography and a biosensor using the surface plasmon resonance phenomenon.

In some embodiments, the present screening method may be carried out in a cell-based assay using cells expressing both of an SMYD2 protein and an HSP90AB1 protein or an RB1 protein. Cells expressing SMYD2 protein and HSP90AB1 protein or RB1 protein include, for example, cell lines established from cancer, e.g. bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer. Alternatively the cells may be prepared by transforming cells with polynucleotide encoding SMYD2 gene and HSP90AB1 gene or RB1 gene. Such transformation may be carried out using an expression vector encoding both SMYD2 gene and HSP90AB1 gene or RB1 gene, or expression vectors encoding either SMYD2 gene or, HSP90AB1 gene or RB1 gene. The present screening method can be conducted by incubating such cells in the presence of a test substance. The binding of SMYD2 protein to HSP90AB1 protein or RB1 protein can be detected by immunoprecipitation assay using an anti- SMYD2 antibody, anti-HSP90AB1 antibody or anti-RB1 antibody.

In the context of the present invention, the therapeutic effect of a candidate substance on inhibiting the cell growth or a candidate substance for treating or preventing cancer relating to SMYD2 gene (e.g., bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer) may be evaluated. Therefore, the present invention also provides a method of screening for a candidate substance capable of suppressing the cell proliferation, or a candidate substance for the treatment and/prevention of cancer (e.g., bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer), using an SMYD2 polypeptide or functional equivalent thereof including the steps of:
(a) contacting an SMYD2 polypeptide or functional equivalent thereof with an HSP90AB1 polypeptide or functional equivalent thereof or an RB1 polypeptide or functional equivalent thereof in the presence of a test substance;
(b) detecting a binding level between the polypeptides;
(c) comparing the binding level detected in the step (b) with those detected in the absence of the test substance; and
(d) correlating the binding level of (c) with the therapeutic effect of the test substance;

In the present invention, the therapeutic effect may be correlated with the binding level between an SMYD2 polypeptide and an HSP90AB1 polypeptide or an RB1 polypeptide. For example, when the test substance suppresses the binding level between the polypeptides as compared to a level detected in the absence of the test substance, the test substance may identified or selected as the candidate substance having the therapeutic effect. Alternatively, when the test substance does not suppress or inhibit the binding level between the polypeptides as compared to a level detected in the absence of the test substance, the test substance may identified as the substance having no significant therapeutic effect.

### Screening Using The Phosphorylation Of RB1 Through RB1 Methylation By SMYD2:

As demonstrated herein, SMYD2 protein methylated RB1 protein, and LC-MS/MS analysis revealed the lysine 810 of RB1 to be methylated by SMYD2 (Fig. 7B). Moreover, the methylation of the lysine 810 of RB1 by SMYD2 enhanced RB1 phosphorylation at the serine 807 and/or serine 811 (Fig. 9, 10). Furthermore, RB1 methylated by SMYD2 accelerated E2F transcriptional activity and promotes cell cycle progression (Fig. 10C, Fig. 12).

These results demonstrates that phosphorylation of RB1 through RB1 methylation by SMYD2 is involved in cancer cell growth. Accordingly, substances that reduce phosphorylation level of RB1 polypeptide though RB1 methylation by SMYD2 may become candidate substances for treating or preventing cancer, or inhibiting cancer cell growth.

Thus, the present invention also provides a method of screening for a candidate substance for either or both of the treatment and prevention cancer using a phosphorylation of RB1 though a methylation of RB1 by SMYD2 as an index.

Exemplary cancers include bladder cancer, lung cancer, breast cancer, cervix cancer, colon cancer, kidney cancer, liver cancer, head and neck cancer, seminoma, cutaneous cancer , pancreatic cancer, lymphoma, ovarian cancer, leukemia and prostate cancer. In the context of the present invention, such screening method may include, for example, the following steps:
(a) contacting a test substance with a cell expressing an SMYD2 gene and an RB 1 gene;
(b) detecting the phosphorylation level of the RB1 polypeptide in the cell of (a); and
(c) selecting the test substance that decreases the phosphorylation level of the RB1 polypeptide in comparison with the phosphorylation level detected in the absence of the test substance.

Alternatively, in some embodiments, the present invention also provides a method for evaluating or estimating a therapeutic effect of a test substance on treating or preventing cancer or inhibiting cancer associated with over-expression of an SMYD2 gene, the method including steps of:
(a) contacting a test substance with a cell expressing an SMYD2 gene and an RB1 gene;
(b) detecting the phosphorylation level of the RB1 polypeptide in the cell of (a); and
(c) correlating the potential therapeutic effect and the test substance, wherein the potential therapeutic effect is shown, when a substance reduces the phosphorylation level of the RB1 polypeptide as compared to the phosphorylation level detected in the absence of the test substance.

The screening methods of the present invention are described in more detail below. Cells expressing the SMYD2 gene and the RB 1 gene include, for example, cell lines established from bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia or prostate cancer, or cell lines transfected with both of SMYD2 gene expression vectors and RB1 expression vectors; any of such cells can be used for the above screening method of the present invention. In some embodiments, cells expressing SMYD2 gene and RB1 gene are isolated and cultured cells exogenously or endogenously expressing SMYD2 and RB1 gene in vitro.

The phosphorylation level can be estimated by methods well known to one skilled in the art, for example, Western blot assay and immunostaining analysis.

The phosphorylation of RB1 polypeptide can be detected by western blotting analysis using an antibody against the phosphorylated RB1 at serine 807 and/or serine 811 amino acid residues of SEQ ID NO: 68.

As noted above, the phrase "reduce the phosphorylation level" refers to at least 10% reduction of phosphorylation level of RB1 polypeptide in comparison to the phosphorylation level in absence of the substance, more preferably at least 25%, 50% or 75% reduced level and most preferably at 95% reduced level. The substance herein includes chemical substance and so on. In the method of screening, a substance that decreases the phosphorylation level of RB1 polypeptide can be selected as candidate substances to be used for the treatment or prevention of cancer, such as bladder cancer, lung cancer, breast cancer, cervix cancer, colon cancer, kidney cancer, liver cancer, head and neck cancer, seminoma, cutaneous cancer, pancreatic cancer, lymphoma, ovarian cancer, leukemia and prostate cancer.

### A Kit For Screening A Candidate Substance For Treating Or Preventing Cancer, Or Inhibiting Cancer Cell Growth:

The present invention further provides a kit for measuring a methyltransferase activity of an SMYD2 polypeptide. The kit can be used for screening for a candidate substrate for treating or preventing cancer, or inhibiting cancer cell growth. In the course of the present invention, in addition to a histone protein (preferably H3 or H4) as known substrates, an HSP90AB1 protein and an RB1 protein were identified as novel substrates of SMYD2 polypeptide. Thus, the present invention provides a kit for measuring a methyltransferase activity of an SMYD2 polypeptide, containing a histone polypeptide or a functional equivalent thereof, an HSP90AB1 polypeptide or a functional equivalent thereof, or an RB1 polypeptide or functional equivalent thereof, as a substrate of SMYD2 polypeptide. Such kit can be used for measuring SMYD2-mediated methyltransferase activity in a sample containing an SMYD2 polypeptide or an SMYD2 polypeptide purified or isolated from a sample.

Furthermore, the present invention provides a kit for detecting for the ability of a test substance to inhibit methylation of histone, HSP90AB1 or RB1 polypeptide by an SMYD2 polypeptide, containing an SMYD2 polypeptide and a histone, HSP90AB1 polypeptide or RB1 polypeptide as a substrate for SMYD2 polypeptide.

The above kits of the present invention find a use for identifying a substance that modulate a methylation level of a histone, HSP90AB1 or RB1 polypeptide by an SMYD2 polypeptide. Furthermore, the kits of the present invention are useful for screening for a candidate substance for treating or preventing cancer, or inhibiting cancer cell growth.

Specifically, the present invention provides the following kits of [1] to [6]:
[1] A kit for screening for a candidate substance for treating or preventing cancer, or inhibiting cancer cell growth, wherein the kit comprises the following components (a) to (d):
   (a) an SMYD2 polypeptide or a functional equivalent thereof;
   (b) a component selected from the group consisting of (i) to (iii);
      (i) a histone protein or a fragment thereof that comprises at least one methylation site,
      (ii) an HSP90AB1 polypeptide or a functional equivalent thereof that comprises at least one methylation site,
      (iii) an RB1 polypeptide or a functional equivalent thereof that comprises at least one methylation site
   (c) a reagent selected from the group consisting of (i) to (iii);
      (i) a reagent for detecting the methylation level of the histone protein or the fragment thereof,
      (ii) a reagent for detecting the methylation level of the HSP90AB1 polypeptide or the functional equivalent thereof,
      (iii) a reagent for detecting the methylation level of the RB1 polypeptide or the functional equivalent thereof; and
   (d) a methyl donor.
[2] The kit of [1], wherein the histone protein is a histone H4 or a histone H3.
[3] The kit of [1], wherein the reagent in the step (c) (i) is an antibody against the methylated histone H4 protein or the methylated histone H3 protein.
[4] The kit of [1], wherein the reagent in the step (c) (ii) is an antibody against the HSP90AB1 polypeptide methylated at lysine 531 and/or lysine 574.
[5] The kit of [1], wherein the reagent in the step (c) (iii) is an antibody against RB1 polypeptide methylated at lysine 810.
[6] The kit of any one of [1] to [5], wherein the methyl donor is S-adenosyl methionine.

Details of the kits of the present invention are described below.

Histone H3 protein or H4 protein contained in the kits of the present invention may either the full length of H3 protein or H4 protein or a functional equivalent thereof such as a fragment of the full length of histone H3 protein or H4 protein. Herein, the functional equivalent of histone H3 protein or H4 protein refers to a modified polypeptide, a fragment or a modified fragment of the full length of histone H3 protein or H4 protein, capable of being methylated by an SMYD2 polypeptide. Preferably, the functional equivalents of histone H3 protein or H4 protein retains at least one methylation site capable to be methylated by SMYD2 polypeptide. Such methylation site includes the lysine 20 of histone H4 protein and the lysine 36 of histone H3 protein.

Thus, preferred examples of the functional equivalent of histone H3 protein or H4 protein include a fragment of the histone H3 protein or H4 protein, such fragments may contain the lysine 36 of histone H3 or the lysine 20 of histone H4, having more than 10 amino acid residues. More preferably, such fragment may be a fragment consisting of the amino acid sequence of SEQ ID NO: 66.

HSP90AB1 polypeptide contained in the kits of the present invention may either the full length of HSP90AB1 (e.g., SEQ ID NO: 65), or a functional equivalent thereof such as a fragment of the full length of HSP90AB1 polypeptide. Herein, the functional equivalent of HSP90AB1 polypeptide refers to a modified polypeptide, a fragment or a modified fragment of the full length of HSP90AB1, capable of being methylated by an SMYD2 polypeptide. Preferably, the functional equivalents of HSP90AB1 polypeptide retains at least one methylation site capable to be methylated by SMYD2 polypeptide. Such methylation sites include the lysine 531 and lysine 574 of HSP90AB1 polypeptide (SEQ ID NO: 65).

Thus, preferred examples of the functional equivalent of HSP90AB1 polypeptide include a fragment of the HSP90AB1 polypeptide retaining a lysine residue corresponding to the lysine 531 and/or lysine 574 of the amino acid sequence of SEQ ID NO: 65. Preferably, such fragments may contain a contiguous sequence from the amino acid sequence of SEQ ID NO: 65 including the lysine 531 and/or 574, having more than 10 amino acid residues. More preferably, the fragments may have more than 15, 20, 25, 30, 50, 75, 100, 150, 200, 250, 300, 350 or 400 amino acid residues. Further more preferably, the fragments may contain amino acid residues 500-724 of SEQ ID NO: 65.

The RB1 polypeptide contained in the kits of the present invention may be either of the full length of RB1 polypeptide (e.g., SEQ ID NO: 68), or a functional equivalent thereof such as a fragment of the full length of RB1 polypeptide. Herein, the functional equivalent of RB1 polypeptide refers to a modified polypeptide, a fragment or a modified fragment of the full length of RB1 polypeptide, capable of being methylated by an SMYD2 polypeptide. Preferably, the functional equivalents of RB1 polypeptide retains at least one methylation site capable to be methylated by SMYD2 polypeptide. Such methylation site includes the lysine 810 of RB1 polypeptide (SEQ ID NO: 68).

Thus, preferred examples of the functional equivalent of RB1 polypeptide include a fragment of the RB1 polypeptide retaining a lysine residue corresponding to the lysine 810 of the amino acid sequence of SEQ ID NO: 68. Preferably, such fragments may contain a contiguous sequence from the amino acid sequence of SEQ ID NO: 68 including the lysine 810, having more than 10 amino acid residues. More preferably, the fragments may have more than 15, 20, 25, 30, 50, 75, 100, 150, 200, 250, 300, 350 or 400 amino acid residues. Further more preferably, the fragments may contain amino acid residues 773-813 of SEQ ID NO: 68.

The histone protein, HSP90AB1 polypeptide or RB1 polypeptide, or functional equivalent thereof may have one or more labeled methyl group(s) such as radiolabeled methyl group(s). Examples of other suitable labels that can be attached to the methyl group(s) includes chromogenic labels, fluorescent labels and such. Histone protein, HSP90AB1 polypeptide or RB1 polypeptide with labeled methyl group(s) can be prepared by methods well-known in the art.

The SMYD2 polypeptide contained in the kits of the present invention may be either the full length of SMYD2 polypeptide (e.g., SEQ ID NO: 63), or a functional equivalent thereof such as a fragment of the full length of SMYD2 polypeptide. Herein, the functional equivalent of SMYD2 polypeptide refers to a modified polypeptide, a fragment or a modified fragment of the full length of SMYD2 polypeptide, having methyltransferase activity for histone protein, HSP90AB1 polypeptide, or RB1 polypeptide.

Herein, the HSP90AB1-binding region of the SMYD2 polypeptide was discovered to be located in a region having amino acid residues 100-247 of SEQ ID NO: 63. Therefore, in the combination of the HSPAB1 polypeptide or functional equivalent thereof, the suitable functional equivalents of SMYD2 polypeptide may be a polypeptide containing amino acid residues 100-247 of SEQ ID NO: 63.

Alternatively, in the course of the present invention, the RB1-binding region of the SMYD2 polypeptide was found to be located in a region having amino acid residues 330-443 of SEQ ID NO: 63. Therefore, in the combination of the RB1 polypeptide or functional equivalent thereof, the suitable functional equivalents of SMYD2 polypeptide may be a polypeptide containing amino acid residues 330-443 of SEQ ID NO: 63.

Reagents for detecting the methylation level of the histone protein, HSP90AB1 or RB1 polypeptide may be any reagents that is able to be used for detection of methylation level of the histone protein, HSP90AB1 or RB1 polypeptide. For example, antibodies against a methylated histone protein, HSP90AB1 or RB1 polypeptide, in particular antibodies against a methylated lysine 36 of histone H3, a methylated lysine 20 of histone H4, a methylated lysine 531 or 574 of the amino acid sequence of SEQ ID NO: 65, or a methylated lysine 810 of the amino acid sequence of SEQ ID NO: 68 may be preferably used as a such reagent. The anti-methylated histone, HSP90AB1 or RB1 antibody may be monoclonal or polyclonal. Furthermore, any fragment or modification (e.g., chimeric antibody, scFv, Fab, F(ab')2, Fv, etc.) of the antibody may be used as the reagent, so long as the fragment retains the binding ability to the methylated histone, HSP90AB1 or RB1 polypeptide. Methods to prepare these kinds of antibodies are well known in the art, and any method may be employed in the present invention to prepare such antibodies and equivalents thereof. Furthermore, the antibody may be labeled with signal generating molecules via direct linkage or an indirect labeling technique. Labels and methods for labeling antibodies and detecting the binding of antibodies to their targets are well known in the art and any labels and methods may be employed for the present invention. For example, radiolabels, chromogenic labels, fluorescent labels and such may be preferably used for labeling the antibody. When the kit contains an anti-methylated histone, HSP90AB1 or RB1 antibody with label, the kit may further contain reagent(s) for detecting a signal generated by the label. Alternatively, the antibodies may be conjugated with such enzyme that catalyses a chromogenic reaction, for example, peroxidase, alkaline phosphatase and such. When the kit contains an anti-methylated histone, HSP90AB1 or RB1 antibody conjugated with the enzyme, the kit may further contain a chromogenic substrate for the enzyme. Alternatively, a secondary antibody labeled or conjugated with an enzyme that catalyses a chromogenic reaction may be contained in the kit of the present invention.

Alternatively, the reagents for detecting the methylation level of the histone protein, HSP90AB1 polypeptide, or RB1 polypeptide may be reagents for detecting hydrogen peroxide or formaldehyde released by histone protein, HSP90AB 1 polypeptide or RB1 polypeptide methylation. Such reagents are well-known in the art.

The kit may contain more than one of the aforementioned reagents. Furthermore, the kit may include a solid matrix for binding an anti-methylated histone H3 or H4 antibody, an anti-methylated HSP90AB1 antibody or an anti-methylated RB1 antibody, a medium or buffer and container for incubating the polypeptides under suitable condition for methylation, a cofactor for methylation such as SAM (S-adenosyl methionine), positive and negative control samples.

The kit of the present invention may further include other materials desirable from a commercial and user standpoint, including buffers, diluents, filters, needles, syringes, and package inserts (e.g., written, tape, CD-ROM, etc.) with instructions for use. These substances and such may be included in a container with a label. Suitable containers include bottles, vials, and test tubes. The containers may be formed from a variety of materials, such as glass or plastic.

Hereinafter, the present invention is described in more detail with reference to the Examples. However, the following materials, methods and examples only illustrate aspects of the present invention and in no way are intended to limit the scope of the present invention. As such, methods and materials similar or equivalent to those described herein can be used in the practice or testing of the present invention.

### Examples

### Example 1: Materials and Methods

### Bladder tissue samples and RNA preparation.

Bladder tissue samples and RNA preparation were described previously (Wallard, M.J. et al. Br J Cancer 94, 569-577 (2006)). Briefly, 125 surgical specimens of primary urothelial carcinoma were collected, either at cystectomy or transurethral resection of bladder tumor (TURBT), and snap frozen in liquid nitrogen. 28 specimens of normal bladder urothelial tissue were collected from areas of macroscopically normal bladder urothelium in patients with no evidence of malignancy. Vimentin is primarily expressed in mesenchymally derived cells, and was used as a stromal marker. Uroplakin is a marker of urothelial differentiation and is preserved in up to 90% of epithelially derived tumors (Olsburgh, J. et al. The Journal of pathology 199, 41-49 (2003)). Use of tissues for this study was approved by Cambridgeshire Local Research Ethics Committee (Ref 03/018). RNA samples of normal tissues (brain, breast, colon, esophagus, eye, heart, liver, lung, pancreas, placenta, kidney, rectum, spleen, stomach and testis) were purchased from BioChain.

### Cell culture.

CCD-18Co, HFL1, 5637, SW780, SCaBER, UMUC3, RT4, T24, HT-1197, HT-1376, A549, H2170, SW480, HCT116, LoVo and 293T cells were from American Type Culture Collection (ATCC) in 2001 and 2003, and tested and authenticated by DNA profiling for polymorphic short tandem repeat (STR) markers except for SW780. The SW780 line was established in 1974 by A. Leibovitz from a grade I transitional cell carcinoma. RERF-LC-AI and SBC5 cells were from Japanese Collection of Research Bioresources (JCRB) in 2001 and tested and authenticated by DNA profiling for polymorphic short tandem repeat (STR) markers. 253J, 253J-BV and SNU-475 cells were from Korean Cell Line Bank (KCLB) in 2001, and tested and authenticated by DNA profiling for polymorphic short tandem repeat (STR) markers. EJ28 cells were from Cell Line Service (CLS) in 2003, and tested and authenticated by DNA profiling for polymorphic short tandem repeat (STR) markers. ACC-LC-319 cells were from Aichi Cancer Center in 2003, and tested and authenticated by DNA profiling for SNP, mutation and deletion analysis. All cell lines were grown in monolayers in appropriate media: Dulbecco's modified Eagle's medium (D-MEM) for EJ28, RERF-LC-AI, HeLa, COS-7 and 293T cells; Eagle's minimal essential medium (E-MEM) for CCD-18Co, WI-38, 253J, 253J-BV, HT-1376, SCaBER, UMUC3 and SBC5 cells; Leibovitz's L-15 for SW480 and SW780 cells; McCoy's 5A medium for RT4 , T24 and HCT116^{p53+/+} cells; RPMI1640 medium for 5637, A549, H2170, ACC-LC-319 and SNU-475 cells. LoVo cells were cultured in Ham's F-12 medium supplemented with 20% fetal bovine A549 cells supplemented with 10% fetal bovine serum and 1% antibiotic/antimycotic solution (Sigma-Aldrich, St. Louis, MO, USA). All cells were maintained at 37degrees C in humid air with 5% CO₂ condition (SAEC, 5637, 253J, 253J-BV, EJ28, HT-1197, HT-1376, J82, RT4, SCaBER, T24, UMUC3, A549, H2170, ACC-LC-319, RERF-LC-AI, SBC5 and 293T RT4, A549, SBC5, 293T, HCT116^{p53+/+}, HeLa and COS-7) or without CO₂ (SW480 and SW780). Cells were transfected with FuGENE6™ (Roche Applied Science, Penzberg, Germany) according to manufacturer's protocols.

### Quantitative real-time PCR (qRT-PCR).

As described above, 125 bladder cancer and 28 normal bladder tissues were prepared in Addenbrooke's Hospital, Cambridge. For quantitative RT-PCR reactions, specific primers for all human GAPDH (housekeeping gene), SDH (housekeeping gene), SMYD2 were designed (Primer sequences in Table 1). PCR reactions were performed using the LightCycler^{(registered trademark)} 480 System (Roche Applied Science) following the manufacture's protocol.

**[Table 1]**

| **Primer sequences for quantitative RT-PCR.** | | |
|---|---|---|
| **Gene** | **Name** | **Primer sequence** |
| *GAPDH* (housekeeping gene) | GAPDH-f | GCAAATTCCATGGCACCGTC (SEQ ID NO: 1) |
| | GAPDH-r | TCGCCCCACTTGATTTTGG (SEQ ID NO: 2) |
| *SDH* (housekeeping gene) | SDH-f | TGGGAACAAGAGGGCATCTG (SEQ ID NO: 3) |
| | SDH-r | CCACCACTGCATCAAATTCATG (SEQ ID NO: 4) |
| *SMYD2* | SMYD2-f | ATCTCCTGTACCCAACGGAAG (SEQ ID NO: 5) |
| | SMYD2-r | CACCTTGGCCTTATCCTTGTCC (SEQ ID NO: 6) |

### Immunohistochemical staining.

Paraffin-embedded tissue slides were purchased from BioChain (Hayaward, CA, USA). Immunohistochemistry was performed using VECTASTAIN^{(registered trademark)} ABC REAGENT (PK-7100, Vector Laboratories, CA, USA) and DAB SUBSTRATE KIT FOR PEROXIDASE^{(registered trademark)} (SK-4100, Vector Laboratories, CA, USA). Slides of paraffin-embedded bladder tumor specimens and normal human tissues were de-paraffinized in xylene and followed by rehydration in 99% ethanol. After wash by 1 x PBS (-), the slides were processed under high pressure (125 degrees C, 30 sec) in antigen-retrieval solution, high pH 9 (S2367; Dako Cytomation, Carpinteria, CA, USA) and quenching was performed by 0.3% hydrogen peroxide (H₂O₂) in methanol for 15 min. After blocking by 3% BSA, tissue sections were incubated overnight with a goat anti-SMYD2 polyclonal antibody (sc-79084, Santa Cruz Biotechnology, Santa Cruz, CA, USA) at a 1:250 dilution ratio, followed by reaction with an anti-goat biotinylated IgG for 1 hour. After incubation with VECTASTAIN^{(registered trademark)} ABC REAGENT, color developing was performed using DAB SUBTRATE KIT FOR PEROXIDASE^{(registered trademark)}. Finally, tissue specimens were stained with Mayer's hematoxylin (Muto pure chemicals, Tokyo, Japan Hematoxylin QS, Vector Laboratories) for 20 s to discriminate the nucleus from the cytoplasm.

### siRNA transfection.

siRNA oligonucleotide duplexes were purchased from Sigma-Aldrich for targeting the human SMYD2 transcripts. siNegative control (siNC), which is a mixture of three different oligonucleotide duplexes, was used as control siRNAs. The siRNA sequences are described in Table 2. siRNA duplexes (100 nM final concentration) were transfected into bladder and lung cancer cell lines with Lipofectamine 2000 (Life Technologies, Carlsbad, CA, USA).

**[Table 2]**

| **siRNA sequence.** | | | |
|---|---|---|---|
| siRNA name | | Sequence | |
| siEGFP | | Sense | GCAGCACGACUUCUUCAAG (SEQ ID NO: 7) |
| | | Antisense | CUUGAAGAAGUCGUGCUGC (SEQ ID NO: 8) |
| siNegative control (cocktail) | Target #1 | Sense | AUCCGCGCGAUAGUACGUA (SEQ ID NO: 9) |
| | | Antisense | UACGUACUAUCGCGCGGAU (SEQ ID NO: 10) |
| | Target #2 | Sense | UUACGCGUAGCGUAAUACG (SEQ ID NO: 11) |
| | | Antisense | CGUAUUACGCUACGCGUAA (SEQ ID NO: 12) |
| | Target #3 | Sense | UAUUCGCGCGUAUAGCGGU (SEQ ID NO: 13) |
| | | Antisense | ACCGCUAUACGCGCGAAUA (SEQ ID NO: 14) |
| siSMYD2 #1 | | Sense | GAUUUGAUUCAGAGUGACA (SEQ ID NO: 15) |
| | | Antisense | UGUCACUCUGAAUCAAAUC (SEQ ID NO: 16) |
| siSMYD2 #2 | | Sense | GAAAUGACCGGUUAAGAGA (SEQ ID NO: 17) |
| | | Antisense | UCUCUUAACCGGUCAUUUC (SEQ ID NO: 18) |

### Clonogenicity assays.

COS-7 cells, cultured in DMEM 10% FBS, were transfected with a p3xFLAG-Mock, p3xFLAG-SMYD2 wild-type (WT) or a p3xFLAG-SMYD2 enzyme-dead mutant vector (delta-NHSC/delta-GEEV). The transfected COS-7 cells were cultured for 2 days and seeded in 10 cm-dish at the density of 10000 cells per 10 cm-dish in triplicate. Subsequently, the cells were cultured in DMEM 10% FBS containing 0.4 (mg/ml) Geneticin/G-418 for 2 weeks until colonies were visible. Colonies were stained with Giemsa (MERCK, Whitehouse station, NJ, USA) and counted by Colony Counter software.

### Mass Spectrometry.

A protein band of SDS-polyacrylamide gel electrophoresis was excised and reduced with dithiothreitol and carboxymethylated by iodeacetic acid. After washing the gel, the band was digested with Achrmobacter Protease I (API, Lys-C a gift from Dr. Masaki, Ibaraki University) at 37 degrees C overnight (Masaki T, et al (1981). Biochim Biophys Acta 660, 44-50.). An aliquot of digest was analyzed by nano LC-MS/MS using LCQ Deca XP plus (Thermo Fisher Scientific, San Jose, CA). The peptides were separated using nano ESI spray column (100 micrometer i.d. x 50 mm L) packed with a reversed-phase material (Inertsil ODS-3, 3 micrometer, GL Science, Tokyo, Japan) at a flow rate 200 nl/min. The mass spectrometer was operated in the positive-ion mode and the spectra were acquired in a data-dependent MS/MS mode. The MS/MS spectra were searched against the in-house database using local MASCOT server (version: 2.2.1, Matrix Sciences, UK). The reduced and carboxylmethyated gel band was also digested with endoproteinase Asp-N (Roche Applied Science) at 37 degrees C overnight. An aliquot of digest was desalted and applied to MALDI-TOF-MS using a Ultraflex (Bruker Daltonik GmbH, Bremen, Germany). And a selected peak was analyzed MALDI-TOF/TOF tandem mass spectrometry in a LIFT mode.

### Amino Acid Analysis.

The excised protein bands blotted on the PVDF membrane were individually inserted in clean 6 mm X 32 mm glass tubes containing 50 pmol of norvaline as internal standard and hydrolyzed in 6 N HCl vapor at 110 degrees C for 20 hours. The hydrolyzed samples were derivatized in situ by 6-aminoquinolyl-N-hydroxysuccinimidyl carbamate (AQC) for fluorophore detection. The AQC-amino acids were separated by ion-pair chromatography on a C18 reversed-phase column (Inertsil ODS-3, 4.6 mm i.d. X 150 mm, 3 micrometer, GL Sciences, Tokyo, Japan). Both a laser induced fluorescence detector (LIF726, GL Sciences) and a fluorescence detector with Xe flush lamp (G1312A, Agilent Technologies, Santa Clara, CA) were used to reveal the existence of mono-methylated Lys (Masuda, A. et al. Anal Chem 82, 8939-8945(2010)).

### Immunocytochemistry.

Cells were fixed with PBS (-) containing 4% paraformaldehyde for 30 min and rendered permeable with PBS (-) containing 0.1% Triton X-100 at room temperature for 2 min. Subsequently, the cells were covered with PBS (-) containing 3% bovine serum albumin for 1 hour at room temperature to block non-specific hybridization, and then were incubated with rabbit anti-Rb (sc-102, Santa Cruz Biotechnology), anti-p-Rb (Ser 807/811)-R (sc-16670-R, Santa Cruz Biotechnology), goat anti-SMYD2 (sc-79084, Santa Cruz Biotechnology), mouse anti-FLAG (Sigma-Aldrich) at a 1:500 dilution ratio, mouse anti-HSP90 antibody (sc-13119, Santa Cruz Biotechnology, Santa Cruz, CA, USA) at a 1:1000 dilution ratio and goat anti-SMYD2 (sc-79084, Santa Cruz Biotechnology, Santa Cruz, CA, USA) at a 1:500 dilution ratio. After washing with PBS (-), cells were stained by an Alexa Fluor ^{(registered trademark)} 488-conjugated anti-rabbit secondary antibody (Life Technologies) or an Alexa Fluor^{(registered trademark)} 594-conjugated anti-mouse secondary antibody (Life Technologies) at a 1:500 dilution ratio. Nuclei were counter-stained with 4', 6'-diamidine-2'-phenylindole dihydrochloride (DAPI). Fluorescent images were obtained under a TCS SP2 AOBS microscope (Leica Mycrosystems, Wetzlar, Germany).

### Immunoprecipitation.

293T or COS-7 cells were seeded at a density of 5 x 10⁵ cells on a 100-mm dish. The next day, the cells were transfected with expression vector constructs using FuGENE 6 (Roche Applied Science) according to the manufacturer's recommendation. After 48 hour, transfected 293T cells were washed with PBS and lysed in CelLytic™ M Cell Lysis Reagent (Sigma-Aldrich) containing complete protease inhibitor cocktail (Roche Applied Science). Five hundred micrograms of whole-cell extract was incubated with anti-FLAG M2 agarose (Sigma-Aldrich) for 1 hour at 4 degrees C. After the beads were washed 3 times with 1 ml of TBS buffer (pH 7.6), the FLAG-tagged proteins bound to the beads were eluted by boiling in Lane Marker Sample Buffer (Thermo Scientific Thermo Fisher Scientific, Hudson, NH). Samples were then subjected to SDS-PAGE, and detected by silver staining or Western blot.

### Western blot.

Whole cell lysates were prepared from the cells with RIPA-like buffer or CelLytic™ M Cell Lysis Reagent (Sigma-Aldrich) containing complete protease inhibitor cocktail (Roche Applied Science) and total protein or immunoprecipitated samples were transferred to nitrocellulose membrane. The membrane was probed with anti-SMYD2 (sc-79084, Santa Cruz Biotechnology), anti-Rb (sc-102, Santa Cruz Biotechnology), anti-phospho-Rb (Ser 807/811)-R (sc-16670-R, Santa Cruz Biotechnology), anti-phospho-Rb (Ser 780) (C84F6, Cell Signaling Technology, Denvers, MA),anti-HSP90 (sc-13119, Santa Cruz Biotechnology), anti-ACTB (I-19, Santa Cruz Biotechnology), anti-FLAG (Sigma-Aldrich), anti-HA (Santa Cruz Biotechnology), anti-His (631212, Clontech Laboratories, Mountain View, CA),anti-HOP (Stressgen Bioreagents), anti-Cdc37 (Santa Cruz Biotechnology) and anti-p23 (abcam) antibodies. An anti-mono-methylated HSP90AB1K574 antibody was made by Sigma-Aldrich. Protein bands were detected by incubating with horseradish peroxidase-conjugated antibodies (GE Healthcare, Little Chalfont, UK) and visualizing with Enhanced Chemiluminescence (GE Healthcare). An anti-mono-methylated RB1 K810 antibody was made by Sigma-Aldrich. Protein bands were detected by MemCode™Reversible Protein Stain Kit (24580, Thermo Fisher Scientific).

### In vitro methyltransferase assay.

For in vitro methylation assay, His-WT-RB1, His-K810A-RB1, His-HSP90AB1 and His-SMYD2 were used as described above. 1 microgram of HSP90AB1 RB1 was incubated with 1 microgram of SMYD2 in 1.0 M Tris-HCl (pH 8.8), 1.0 micro-Ci/ml _{L} -[methyl-³H] methionine (Perkin Elmer) and MilliQ water for 1 hour. After boiled in sample buffer, the samples were subjected to SDS-PAGE, followed by visualization by fluorography.

### In vitro Kinase Assay

CDK4/ Cyclin D1(ab55695, Abcam, Cambridge, UK) was used for kinase assay in reaction buffer containing 40 mM MOPS (pH 7.0), 1 mM EDTA, 20 mM ATP for 10 min at 30 degrees C. After boiling in sample buffer, the samples were subjected to SDS-PAGE.

### In vivo labelling.

In vivo labelling was performed as described previously (Cho, H.S. et al. Cancer Res (2010)). Cells were starved for 1 hour in methionine-free medium, including cycloheximide (100 microgram/ml) and chloramphenicol (40 microgram/ml). They were then labeled with _{L}-[methyl-³H] methionine (10 micro-Ci/ml, Perkin Elmer) for 5 hours. FLAG-mock, SMYD2 (WT) or SMYD2 (delta-NHSC/ delta-GEEV) was immunoprecipitated with an anti-HSP90 antibody (Santa Cruz Biotechnology) and methylated HSP90 was visualized by fluorography.

### In vitro cross-linking assay.

In vitro cross-linking was performed as described previously (Allan, R.K., Mok, D., Ward, B.K. & Ratajczak, T. J Biol Chem 281, 7161-7171 (2006)). After in vitro methyltransferase assay in the presence or absence of SMYD2, HSP90AB1 was incubated with 94.7 mM PBS (pH 7.4) and 10 mM BS³ (Thermo Scientific) for 30 minutes at room temperature. Cross-linking reaction was quenched by adding 1 M Tris-HCl. After boiled in sample buffer, each reaction mixture was subjected to SDS-PAGE and WB using an anti-HSP90 antibody (Santa Cruz Biotechnology). After WB, the membrane was stained by Ponceau S.

### In vivo cross-linking assay.

HeLa cells were seeded at a density of 5 x 10⁵ cells on a 100-mm dish. The next day, the cells were treated with siSMYD2#2. 24 hours after siRNA treatment, the cells were transfected with pCAGGS-n3FC-HSP90AB1 (WT) or pCAGGS-n3FC-HSP90AB1 (K531A/K574A). 24 hours after transfection, EMEM was replaced by Dulbecco's Modified Eagle's Limiting Medium (DMEM-LM) (ThermoScientific) containing L-Photo-Leucine and L-Photo-Methionine (Thermo Scientific), subjected to UV irradiation (Stratalinker^{(registered trademark)} UV Crosslinker, American Laboratory Trading, 10800 J). Then, the cells were harvested and total protein (5 microgram) was transferred to nitrocellulose membrane, followed by SDS-PAGE and WB using anti-FLAG (Sigma-Aldrich), anti-SMYD2 (Santa Cruz Biotechnologies) and anti-ACTB (Santa Cruz Biotechnologies) antibodies. Protein bands were detected as described above.

### Primer sequences.

Oligonucleotides to construct mammalian expression vectors and expression vectors for recombinant proteins in E. coli are described in Table 3-1, Table 3-2 and Table 4, respectively.

**[Table 3-1]**

| **Oligonucleotides to construct mammalian expression vectors.** | | |
|---|---|---|
| **Gene** | **Name** | **Primer sequence** |
| *SMYD2* (1-433) | SMYD2 (1-433)-f | |
| | SMYD2 (1-433)-r | CCGCTCGAGGTGGCTTTCAATTTCCTGTTTGATC (SEQ ID NO: 20) |
| *SMYD2* ( 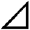 NHSC/ 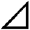 GEEV) 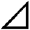 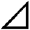 | SMYD2 (*Δ*NHSC)-f | |
| | SMYD2 (*Δ*GEEV)-f | |
| | pCAGGS-r | TATTTGTGAGCCAGGGCATT (SEQ ID NO: 23) |
| *SMYD2* (1-100) | SMYD2 (1-100)-f | |
| | SMYD2 (1-100)-r | CCGCTCGAGCCAGTTTTCCCCAAAAACAACC (SEQ ID NO: 25) |
| *SMYD2* (1-250) | SMYD2 (1-250)-f | |
| | SMYD2 (1-250)-r | CCGCTCGAGTCTATCTTCCGTTGGGTACAGGAG (SEQ ID NO: 27) |
| *SMYD2* (100-433) | SMYD2 (100-433)-f | |
| | SMYD2 (100-433)-r | CCGCTCGAGGTGGCTTTCAATTTCCTGTTTGATC (SEQ ID NO: 29) |
| *SMYD2* (250-433) | SMYD2 (250-433)-f | |
| | SMYD2 (250-433)-r | CCGCTCGAGGTGGCTTTCAATTTCCTGTTTGATC (SEQ ID NO: 31) |
| *SMYD2* (330-433) | SMYD2 (330-433)-f | |
| | SMYD2 (330-433)-r | CCGCTCGAGGTGGCTTTCAATTTCCTGTTTGATC (SEQ ID NO: 33) |

**[Table 3-2]**

| **Oligonucleotides to construct mammalian expression vectors.** | | |
|---|---|---|
| **Gene** | **Name** | **Primer sequence** |
| *HSP90AB1* (1-724) | HSP90AB1 (1-724)-f | |
| | HSP90AB1 (1-724)-r | TGCGTCGACATCGACTTCTTCCATGCGAGAC (SEQ ID NO: 35) |
| *HSP90AB1* (1-500) | HSP90AB1 (1-500)-f | |
| | HSP90AB1 (1-500)-r | TGCGTCGACCACAAAAGCTGAGTTGGCCAC(SEQ ID NO: 37) |
| *HSP90AB1* (250-724) | HSP90AB1 (250-724)-f | |
| | HSP90AB1 (250-724)-r | TGCGTCGACATCGACTTCTTCCATGCGAGAC (SEQ ID NO: 39) |
| *HSP90AB1* (500-724) | HSP90AB1 (500-724)-f | |
| | HSP90AB1 (500-724)-r | TGCGTCGACATCGACTTCTTCCATGCGAGAC (SEQ ID NO: 41) |
| *HSP90AB1* (K531A) | HSP90AB1 (K531A)-f | |
| | HSP90AB1 (K531A)-r | |
| *HSP90AB1* (K574A) | HSP90AB1 (K574A)-f | GCGGTTGAGAAGGTGACAATCTCC (SEQ ID NO: 44) |
| | HSP90AB1 (K574A)-r | CTTATCTAAGATTTCTTTCATGAGCTTG (SEQ ID NO: 45) |

**[Table 4]**

| **Oligonucleotides to construct expression vectors for recombinant proteins in *E.coli.*** | | | |
|---|---|---|---|
| **Gene** | **Tag** | **Name** | **Primer sequence** |
| *SMYD2* (1-433) | His | SMYD2-Bam-f | |
| | | SMYD2-GEX-r | |
| *HSP90AB1* (1-724) | GST, His His | HSP90AB1 (1-724)-GEX-f | TGCCAATTGATGCCTGAGGAAGTGCACC (SEQ ID NO: 48) |
| | | HSP90AB1 (1-724)-GEX-r | |
| *HSP90AB1* (1-250) | GST, His | HSP90AB1 (1-250)-f | TGCCAATTGATGCCTGAGGAAGTGCACC (SEQ ID NO: 50) |
| | | HSP90AB1 (1-250)-r | |
| *HSP90AB1* (1-500) | GST, His | HSP90AB1 (1-500)-f | TGCCAATTGATGCCTGAGGAAGTGCACC (SEQ ID NO: 52) |
| | | HSP90AB1 (1-500)-r | |
| *HSP90AB1* (250-724) | GST, His | HSP90AB1 (250-724)-f | |
| | | HSP90AB1 (250-724)-r | |
| *HSP90AB1* (500-724) | GST, His | HSP90AB1 (500-724)-f | |
| | | HSP90AB1 (500-724)-r | |
| *HSP90A81* (K531 A) | His | HSP90AB1 (K531 A)-f | |
| | | HSP90AB1 (K531 A)-r | |
| *HSP90AB1* (K574A) | His | HSP90AB1 (K574A)-f | GCGGTTGAGAAGGTGACAATCTCC (SEQ ID NO: 60) |
| | | HSP90AB1 (K574A)-r | CTTATCTAAGATTTCTTTCATGAGCTTG (SEQ ID NO: 61) |

### Example 2: SMYD2 is over-expressed in human cancer and regulates the growth of cancer cells.

The present inventors first examined expression levels of a number of histone methyltransferases in a small subset of clinical bladder samples and found significant differences in expression levels between normal and cancer cells for the SMYD2 gene. Consequently, 125 bladder cancer samples and 28 normal control samples (British) were analyzed, and significant elevation of SMYD2 expression was found in tumor cells compared with in normal cells (P < 0.0001, Mann-Whitney U-test) (Fig. 1A and Table 5: patient characteristics). Expression levels of SMYD2 between bladder tumor and various types of normal tissues were also compared, and it was found that expression levels of SMYD2 in bladder tumor tissues are significantly higher than those in normal organ tissues including heart, lung, liver and kidney (Fig. 1B). Additionally, immunohistochemistry showed over-expression of SMYD2 in bladder cancer sections at the protein level (Fig. 1C). Furthermore, the Oncomine database demonstrated that SMYD2 is over-expressed in various types of human cancer like colon cancer and prostate cancer besides bladder cancer (Fig. 1F).

**[Table 5]**

| **Statistical analysis of *SMYD2* expression levels in clinical bladder tissues.** | | | | |
|---|---|---|---|---|
| | | **SMYD2** | | |
| **Characteristic** | **n** | **Mean** | **SD** | **95%Cl** |
| **Normal (Control)** | 28 | 1.055 | 0.512 | 0.866 - 1.245 |
| **Tumor (Total)** | 125 | 7.092 | 10.474 | 5.256 - 8.929 |
| Tumor grade | | | | |
| G1 | 12 | 6.879 | 4.391 | 4.395 - 9.363 |
| G2 | 63 | 8.633 | 13.833 | 5.217 - 12.049 |
| G3 | 49 | 5.195 | 5.001 | 3.794 - 6.595 |
| Metastasis | | | | |
| Negative | 98 | 7.442 | 11.551 | 5.155 - 9.729 |
| Positive | 27 | 5.823 | 4.831 | 4.001 - 7.646 |
| Gender | | | | |
| Male | 91 | 6.623 | 8.600 | 4.856 - 8.390 |
| Female | 32 | 5.210 | 5.120 | 3.436 - 6.984 |
| Recurrence | | | | |
| No | 28 | 9.221 | 13.198 | 4.332 - 14.110 |
| Yes | 51 | 5.541 | 5.465 | 4.041 - 7.041 |
| Died | 8 | 5.539 | 6.733 | 0.873 - 10.205 |
| Smoke | | | | |
| No | 27 | 5.882 | 4.601 | 4.147 - 7.618 |
| Yes | 49 | 7.476 | 11.231 | 4.331 - 10.620 |

To know whether SMYD2 is indispensable for cancer cell viability, the present inventors first examined expression levels of SMYD2 in various cell lines, and found over-expression of SMYD2 in bladder, lung, colon and liver cancer cell lines as compared with the normal fibroblast-derived normal cell line WI-38 (Fig. 1D). Over-expression of SMYD2 in cancer cells were also confirmed at the protein level (Fig. 1E). Then the expressions of SMYD2 in bladder cancer cells (SW780 and RT4) were inhibited by two independent siRNAs (Table 2; siRNA sequences). After confirming knockdown effect of those siRNAs (Fig. 2A), cell growth assay was performed and significant growth suppression was found in the cells treated with SMYD2 siRNAs, relative to control siRNA (siNC) (Fig. 2B). Significant growth suppression was also observed in lung cancer cell lines (A549, LC319 and SBC5) (Fig. 2F). To examine whether SMYD2 possesses oncogenic activity, the present inventors conducted a clonogenicity assay. A wild-type SMYD2 (SMYD2 WT) vector and an enzyme-dead SMYD2 (SMYD2 delta-NHSC/ delta-GEEV) vector were transfected into COS-7 cells together with a mock vector as a control. A clonogenicity assay was performed on each culture (Fig. 2C). Cells transfected a wild-type SMYD2 vector formed more colonies than those transfected with an enzyme-dead SMYD2 vector or a mock control vector, therefore it is the methylation activity of SMYD2 that promotes oncogenesis in cells. Because SMYD2 is over-expressed at an early stage in cancer progression, SMYD2 appears to play a crucial role in human carcinogenesis.

To elucidate the effects of SMYD2 over-expression on the growth of cancer cells in more detail, the effect of SMYD2 over-expression was examined using human embryonic kidney fibroblast (HEK293) cells containing the Flp-In T-REx system (T-REx-293, Invitrogen). The V5 tagged SMYD2 expression vector, empty vector (mock) or V5 tagged CAT expression vector (control) were transfected into the T-REx-293 cells to establish stable cell lines expressing SMYD2. The present inventors analyzed the cell cycle status by FACS analysis (Fig. 2D) and found that the proportions at the S phase were significantly increased in the T-REx-SMYD2 cells compared with those in the control cells (P < 0.01 [Mock, SMYD2] and P < 0.05 [CAT, SMYD2], respectively). Conversely, the proportion at the G₀/G₁ phase in the T-REx-SMYD2 cells was slightly lower than that in the control cells (P < 0.01 [Mock, SMYD2] and P < 0.01 [CAT, SMYD2], respectively). BrdU and 7-AAD staining were also performed to analyze the detailed cell cycle status of cancer cells, and it was confirmed that the proportion of cancer cells at the S phase was significantly decreased after the knockdown of SMYD2 (Fig. 2E and 2G).

### Example 3: SMYD2 forms a complex with HSP90AB1.

In order to clarify how SMYD2 promotes cancer cell growth, the present inventors attempted to identify interacting partners of SMYD2. 293T cells were transfected with a FLAG-mock or a FLAG-SMYD2 vector, and immunoprecipitation (IP) and mass spectrometry (MS) analysis was conducted. Consequently, HSP90AB1 was identified as an interacting partner of SMYD2 (Fig. 3A). Since HSP90 protein has been considered to play critical roles in human cancer through chaperoning many oncoproteins and facilitating their functions (Trepel, J., Mollapour, M., Giaccone, G. & Neckers, L. Nat Rev Cancer 10, 537-549 (2010)), the functional relationship between SMYD2 and HSP90AB1 was examined. The interaction between SMYD2 and HSP90AB1 was confirmed by co-immunoprecipitation analysis (Fig. 3B and 3C). To determine the binding region of SMYD2 to HSP90AB1, plasmid clones designed to express different portions of SMYD2 were constructed and co-immunoprecipitation analysis were performed (Fig. 3D). Then, it was found that SMYD2 binds to HSP90AB1 through a central region, including a part of SET domain (Fig. 3E). With regard to the binding region of HSP90AB1 to SMYD2, it was found that the C-terminal region may be important for the interaction with SMYD2 (Fig. 3F and 3G). In addition, immunocytochemical analysis revealed their co-localization in the cytoplasm (Fig. 3H). These results indicate that SMYD2 forms a complex with HSP90AB1.

### Example 4: SMYD2 methylates HSP90AB1.

HSP90 protein is known to be subject to multiple post-translational modifications (PTMs), but there are no reports regarding methylation (Scroggins, B.T. et al. Mol Cell 25, 151-159 (2007), Martinez-Ruiz, A. et al. Proc Natl Acad Sci U S A 102, 8525-8530 (2005), Mollapour, M. et al. Mol Cell 37, 333-343 (2010), Mollapour, M. et al. Mol Cell 41, 672-681 (2011)). Therefore, whether SMYD2 could methylate HSP90AB1 and affect its functions was examined. First, in vitro methyltransferase assay was performed and it was found that HSP90AB1 is methylated by SMYD2 in a dose-dependent manner (Fig. 4A). The present inventors then validated whether HSP90AB1 is also methylated by SMYD2 in cells. 293T cells were transfected with a FLAG-mock vector, a FLAG-SMYD2 (WT) vector or a FLAG-SMYD2 (delta-NHSC/ delta-GEEV), followed by in vivo labelling experiments as described in Example1. In consequence, the specific signal corresponding to methylated HSP90AB1 was observed and the methylation was dependent on the enzyme activity of SMYD2 (Fig. 4B). Next, to determine which portion of HSP90AB1 is methylated by SMYD2, the present inventors prepared several deletion mutants of recombinant HSP90AB1 protein and performed an in vitro methyltransferase assay (Fig. 4C and Fig. 4H). The data revealed that the C-terminal portion of GST-HSP90AB1 (500-724 aa) is methylated by SMYD2. Subsequently, the present inventors tried to identify methylation sites of HSP90AB1 by LC-MS/MS analysis and identified lysines 531 and 574 are methylated by SMYD2 (Fig. 4D and 4E). Substitution of K574 to alanine decreased the signal of SMYD2-dependent HSP90AB1 methylation, and the methylation signal was more reduced when using both K531 and K574 substituted HSP90AB1 protein (Fig. 4F). This result was also confirmed using partial HSP90AB1 (500-724 aa) (Fig. 4G). Because these methylation sites are highly conserved from Danio rerio to Homo sapiens, it is possible that methylation of these sites may be important for the regulation of HSP90AB1 functions (Fig. 4I).

### Example 5: SMYD2-dependent methylation alters the chaperonin complex formation of HSP90AB1.

The present inventors next analyze effects of SMYD2-dependent methylation on functions of HSP90AB1 in more detail. After methyltransferase reaction of SMYD2, a dimerization assay was performed using bis [sulfosuccinimidyl] suberate (BS³), a crosslinking reagent, was followed by SDS-PAGE and Western blot. In consequence, the present inventors found methylation-dependent dimerization of HSP90AB1 (Fig. 5F), which implies that SMYD2-dependent HSP90AB1 may promote the formation of its dimerization. Subsequently, the present inventors validated whether methylation-dependent dimerization by SMYD2 is observed in culture cells. After siSMYD2 treatment to exclude effects of endogenous SMYD2, a FLAG-HSP90AB1 (WT) vector and a HA-mock vector or a HA-SMYD2 vector was co-transfected into 293T cells. Fourty-eight hours after transfection, an in vivo cross-linking assay was performed and it was found that SMYD2 promotes crosslinking of HSP90AB1 (Fig. 5A). Co-immunoprecipitation analysis showed that double substitution (K531A/K574A) negatively affected the dimerization process of HSP90AB1 (Fig. 5B, compare lanes between 5 and 6). To determine which residue is more important for this process, a mutant expression vector containing single substitution (K531A, K574A, respectively) was prepared and it was found that K574A, not K531A, of HA-HSP90AB1 resulted in reduced affinity to FLAG-HSP90AB1 (WT) (Fig. 5C, compare lanes between 7 and 8). This result indicates that methylation of K574 may be more important for dimerization of HSP90AB1.

HSP90 exerts its chaperone functions in collaboration with co-chaperones (Young, J.C., Agashe, V.R., Siegers, K. & Hartl, F.U. Nat Rev Mol Cell Biol 5, 781-791 (2004), and some PTMs are reported to alter affinity of HSP90 to co-chaperones (Scroggins, B.T. et al. Mol Cell 25, 151-159 (2007), Mollapour, M. et al. Mol Cell 37, 333-343 (2010), Mayer, M.P. Mol Cell 37, 295-296 (2010)). Therefore, the present inventors investigated a possibility that HSP90AB1 methylation by SMYD2 affects the binding of HSP90AB1 to co-chaperones. A FLAG-HSP90AB1 (WT) vector or a FLAG-HSP90AB1 (K531A/K574A) vector 293T cells were transfected into 293T cells, followed by immunoprecipitation and Western blot analysis. The present inventors found that K531A/K574A substitution of HSP90AB1 disrupted its interaction with HOP and Cdc37, not p23 (Fig. 5D, compare lanes between 3 and 4). In this case, methylation status of HSP90AB1 were also monitored using a specific antibody recognizing mono-methylated HSP90AB1K574 (Fig. 5D and 5G). In addition, according to an experiment using single mutated constructs at K531A and K574A of HSP90AB1, the present inventors found that substitution of lysine to alanine at residue 574 resulted in decreased binding affinity to HOP and Cdc37, not p23 (Fig. 5E, compare lanes between 7 and 8). Finally, to elucidate significance of methylated HSP90AB1 in tumor growth, the present inventors generated stable transfectants of HeLa cells over-expressing FLAG-HSP90AB1 (WT) and FLAG-HSP90AB1 (K531A/K574A). After confirming the stable expression of wild-type and substituted HSP90 proteins (Fig. 5Ha, lanes a1 and b2), a growth assay was performed using the stable cell lines and it was found that growth promoting effect of HSP90AB1 was diminished by substitution of methylation sites to alanines (Fig. 5Hb, P < 0.05). Taken together, these data suggested that SMYD2-dependent methylation appears to facilitate the dimerization process and the interaction with co-chaperones of HSP90AB1 and contribute to human carcinogenesis.

### Example6: Screening for inhibitors of methyltransferase activity of SMYD2

His-tagged SMYD2 (His-tagged polypeptide consisting of amino acid sequence of SEQ ID NO: 63) was incubated in methyltransferase buffer (50 mM Tris-HCl, 100 mM NaCl, 4 mM MgCl2, 10 mM DTT, pH 8.8) along with 1.8 microM biotinylated-histone H4 peptide, 0.18 micro-M S-adenosyl-L-[methyl-³H] methionine and 50 microgram of streptavidin-coated PVT beads in a total volume of 15 microliter. After incubation for 30 min at room temperature, reactions were stopped by adding potassium phosphate buffer (pH 6.0), then light emitted from the beads are measured using a scintillation counter.

As a result of evaluating a number of chemically synthesized compounds by aforementioned assay, some compounds that inhibit methyltransferase activity of SMYD2 were identified.

### Example 7: SMYD2 methylates Lys 810 of RB1 both in vitro and in vivo.

In order to identify a critical substrate of SMYD2 involved in human carcinogenesis, the present inventors performed an in vitro methyltransferase assay using various tumor-related proteins as substrates, and found a strong methylation signal when RB1 protein was used as a substrate (Fig.6A). To further verify the interaction between RB1 and SMYD2 proteins, the present inventors carried out a co-immunoprecipitation assay after co-transfection of FLAG-SMYD2 and HA-RB1 or FLAG-RB1 and HA-SMYD2 expression vectors into 293T cells, and confirmed their bindings (Figs. 6B and C). In addition, an immunoprecipitation assay using deletion mutants of SMYD2 showed that the C-terminal portion of SMYD2 is essential to interact with RB1 (Fig. 6D). Furthermore, the present inventors also confirmed the co-localization of endogenous RB1 and SMYD2 proteins in the small cell lung cancer cell line SBC5 by immunocytochemical analysis (Fig. 6E).

The present inventors next constructed plasmid vectors that were designed to express parts of RB1 protein to identify a methylation site of RB1 by SMYD2 and prepared recombinant proteins expressed in E.coli. Using those proteins, the present inventors conducted an in vitro methyltransferase assay (Fig.7A) and found that the C-terminal region (773 aa to 928 aa) of RB1 protein includes the methylation site(s). Subsequent LC-MS/MS analysis indicated lysine 810 on RB1 to be mono-methylated by SMYD2 (Fig.7B). The SMYD2-dependent lysine mono-methylation was also confirmed by amino-acid analysis (Fig. 11). In order to validate the identified methylation site of RB1, the present inventors prepared a partial RB1 protein, which was replaced lysine 810 to alanine (K810A-RB1 (773-813)) and performed an in vitro methyltransferase assay (Fig.7C). The specific methylation signal of the wild-type RB1 protein by SMYD2 was by the replacement of K810. On the basis of these results, the present inventors generated a polyclonal antibody targeting K810-mono methylated RB1 peptide. To validate the specificity of the antibody, the present inventors performed an in vitro methyltrasnferase assay with or without SMYD2 and observed a SMYD2-dependent methylation signal (Fig.7D). The present inventors also found that this antibody could recognize neither the K810-substituted RB1 protein treated with wild-type SMYD2 in vitro (Fig.7E) nor the wild-type RB1 protein treated with enzyme-dead SMYD2 in vivo (Fig.7F). These results imply that SMYD2 methylates lysine 810 of RB1 protein both in vitro and in vivo, and the antibody the present inventors generated can specifically recognize K810-methylated RB1.

### Example 8: SMYD2 enhances phosphorylation of RB1 at Ser 807/811 through methylation of Lys 810.

As it is well-known that phosphorylation plays a crucial role in the regulation of RB1 functions (Weinberg RA .Cell 81, 323-330(1995), Sherr CJ, et al.Cancer Cell 2, 103-112.(2002)), the present inventors examined the effect of Lys 810 methylation on phosphorylation status of RB1. The present inventors first performed western blot analysis of two non-cancerous cell lines and seven cancer cell lines to examine phosphorylation status of RB1 at Ser 807/811 and found some correlation between the higher phosphorylation status of RB1 and high SMYD2 expression (Fig.8A). In order to clarify whether SMYD2 affects RB 1 phosphorylation status through methylation of Lys 810, the present inventors conducted gain-of-function and loss-of-function experiments. After introduction of FLAG-SMYD2 into 293T cells, the present inventors detected a significant elevation of phosphorylation status of RB 1 at Ser 807/811 compared with the cells transfected with a mock vector (Fig.8B). Subsequent immunocytochemical analysis detected that over-expression of WT-SMYD2 enhanced phosphorylation of RB1 at Ser 807/811 in HeLa cells (Figure 8C). Concordantly, phosphorylation of RB1 at Ser 807/811 was significantly reduced after knockdown of SMYD2 (Fig.8D). To examine the effect of methyltransferase activity of SMYD2 on phosphorylation status of RB1, the present inventors transfected a vector designed to express a partial RB1 (FLAG-RB1(773-813)) together with a wild-type SMYD2 expression vector (HA-SMYD2) or with an enzyme-dead SMYD2 expression vector (HA-SMYD2 (delta-NHSC/GEEV)) into 293T cells, and conducted immunoprecipitation using anti-FLAG M2 agarose. As shown in Fig. 7E, the phosphorylation level of RB 1 at Ser 807/811 in the cells transfected with WT-SMYD2 was significantly higher than that in the cells with enzyme-dead SMYD2. Hence, SMYD2-dependent RB1 methylation appears to enhance phosphorylation status of RB1 at Ser 807/811.

In order to evaluate the effect of SMYD2-dependent methylation on the phosphorylation status of RB1, the present inventors performed an in vitro kinase assay using RB1 as a substrate reacted with or without SMYD2 (Fig.9A). After confirmation of Lys 810 methylation of RB1 (Fig.9B, top), the present inventors reacted the samples with the CDK4/Cyclin D1 complex, which is an important regulator of RB 1 phosphorylation, and monitored phosphorylation status of RB1 at Ser 807/811 by western blot (Fig.9B, bottom). Importantly, methylated RB1 showed higher phosphorylation levels than non-methylated protein. In addition, when the present inventors examined the dose-dependent effect of SMYD2 on the RB1 phosphorylation at Ser 807/811, it was increased in a dose-dependent manner, correlating with methylation levels of RB1 at Lys 810 (Fig.9C). Likewise, mutant RB1 containing a substitution of Lys 810 to alanine showed much weaker phosphorylation levels than wild-type RB1 (Fig.9D), implying that methylation of RB1 at Lys 810 appears to enhance phosphorylation levels of RB1. The present inventors then prepared a K810 mono-methylated RB1 peptide (K810me-RB1 peptide (SEQ ID NO: 70)) and a K810 unmethylated RB1 peptide (Control-RB1 peptide (SEQ ID NO: 69)), and investigated the effect of K810 mono-methylation on the phosphorylation of RB1 at Ser 807/811 by the CDK4/Cyclin D1 complex in more detail (Fig.9E). After confirmation of K810 mono-methylation by dot blot analysis using an anti-RB1 K810me antibody, the present inventors conducted a kinase assay and found significantly higher phosphorylation levels of RB1 at Ser 807/811 in the K810me-RB1 peptide than the the unmethylated peptides (Fig.9F). The CDK4 dose-dependent elevation of RB1 phosphorylation at Ser 807/811 in the K810me-RB1 was also confirmed (Fig.9G). These findings indicate that K810 mono-methylation of RB 1 by SMYD2 can enhance the phosphorylation level of RB 1 at Ser 807/811.

### Example 9: Lys 810 methylation of RB1 promotes cell cycle progression.

To further evaluate the effect of methylation on phosphorylation status of RB1 in vivo, the present inventors transfected a FLAG-WT-RB1 vector or a FLAG-K810A-RB1 vector with a HA-WT-SMYD2 vector into 293T cells, and carried out immunoprecipitation with anti-FLAG M2 agarose (Fig.10A). Consistent with previous data, WT-RB1 showed higher phosphorylation levels of RB1 at Ser 807/811 than Lys 810-subtituted RB1 (K810A-RB1), and this result was also confirmed using a partial RB1 (773 - 813) (Fig.10B). Taken together, methylation of RB1 at Lys 810 also seems to enhance the phosphorylation status of RB1 in vivo.

It is known that CDK-mediated phosphorylation of RB1 prevents the interaction of RB1 with E2F1, a multifunctional transcription factor that activates the genes required for the cell cycle progression at the G₁/S transition, and enables E2F1-dependent gene expression (Sherr CJ. et al. Cancer Cell 2, 103-112.(2002)). As Lys 810 methylation enhanced the phosphorylation of RB1, the present inventors performed an E2F reporter assay to examine the effect of RB1 methylation on the cell cycle. E2F-luciferase activity was significantly low in the cells over-expressing Lys 810-substituted RB1 compared to the cells over-expressing wild-type RB1 (Fig.10C). This result indicates that Lys 810 methylation of RB1 may promote E2F transcriptional activity in vivo. Furthermore, the present inventors established stable cell lines, which can express wild-type RB1 (WT) and K810-substituted RB1 (K810A) by induction of doxycycline, using Flp-In™ T-REx™ 293 cell line system. Consistent with the aforementioned data, cells expressing wild-type RB1 showed higher cell growth rate than cells expressing RB1 (K810A) (Fig. 12). Taken together, Lys 810 methylation of RB1 by SMYD2 appears to promote cell cycle progression through increase of RB1 phosphorylation.

### Discussion

The present inventors previously demonstrated that certain histone methyltransferases (HMTs) play a vital role in human cancer pathogenesis, in addition to normal cellular biology (Hamamoto, R. et al. Nat Cell Biol 6, 731-740 (2004), Takawa, M. et al. Cancer Sci (2011), Yoshimatsu, M. et al. Int J Cancer 128, 562-573 (2011)). In addition, other groups have proposed involvement of HMTs in malignant alterations of human cells (Portela, A. & Esteller, M. Nat Biotechnol 28, 1057-1068 (2010), Schneider, R., Bannister, A.J. & Kouzarides, T. Trends Biochem Sci 27, 396-402 (2002), Sparmann, A. & van Lohuizen, M. Nat Rev Cancer 6, 846-856 (2006)). Together, this evidence clearly suggests that deregulation of HMTs makes a significant contribution to human carcinogenesis, though a more in-depth comprehension about the relationship between abnormalities of HMTs and human cancer still remains to be clarified.

In the course of the present invention, it was demonstrated that SMYD2 is over-expressed in bladder as well as various other cancer tissues and that SMYD2 methylates HSP90AB1 and RB1 as novel substrates. Recently, certain HMTs have been shown to methylate non-histone proteins and thereby alter their functions such as transcriptional activity, protein stability and binding affinity to interacting partners (Esteve, P.O. et al. Proc Natl Acad Sci U S A 106, 5076-5081 (2009), Guo, Z. et al. Nat Chem Biol 6, 766-773 (2010)).

The assays of present invention have clarified that methylation of HSP90AB1 by SMYD2 affects its functions like dimerization process and binding affinity to co-chaperones and that this methylation process promotes cancer cell proliferation (Fig.5). HSP90 is ubiquitously expressed in eukaryotic cells and comprises up to 1-2 % of total proteins (Borkovich, K.A., Farrelly, F.W., Finkelstein, D.B., Taulien, J. & Lindquist, S. Mol Cell Biol 9, 3919-3930 (1989)). Structurally, HSP90 consists of three domains: the N-domain (ATP binding pocket), the M-domain (binding regions for co-chaperones and client proteins) and the C-terminal dimerization domain (dimerization motif) (Wandinger, S.K., Richter, K. & Buchner, J. J Biol Chem 283, 18473-18477 (2008))). Importantly, HSP90 serves as an evolutionally conserved molecular chaperone that helps a number of newly synthesized polypeptides and unstable folded proteins fold correctly so as to prevent them from misaggregating (Wandinger, S.K., Richter, K. & Buchner, J. J Biol Chem 283, 18473-18477 (2008), Young, J.C., Agashe, V.R., Siegers, K. & Hartl, F.U. Nat Rev Mol Cell Biol 5, 781-791 (2004)). Because client proteins include transcriptional factors and proteins kinases that are crucial for signal transduction and adaptive responses to stress, HSP90 appears to play an essential role in regulating multiple cellular functions (Zhao, R. et al. Cell 120, 715-727 (2005), Chiosis, G., Vilenchik, M., Kim, J. & Solit, D. Drug Discov Today 9, 881-888 (2004)). To exert chaperone functions, homo-dimerization and coordination with co-chaperone proteins such as p21, HOP and Cdc37 (Taipale, M., Jarosz, D.F. & Lindquist, S. Nat Rev Mol Cell Biol 11, 515-528 (2010)., Wayne, N. & Bolon, D.N. J Biol Chem 282, 35386-35395 (2007)) are essential as well as ATPase activity. Client proteins are clamped by ATP-bound HSP90 protein, and the folding process is conducted by HSP90 in cooperation with other chaperones and co-chaperones, followed by release of the matured clients that depend on conformational change of HSP90 by ATP hydrolysis (Ali, M.M. et al. Nature 440, 1013-1017 (2006), Vaughan, C.K. et al. Mol Cell 23, 697-707 (2006), Hessling, M., Richter, K. & Buchner, J. Nat Struct Mol Biol 16, 287-293 (2009)). Additionally, it has been reported that functions of HSP90 are regulated by multiple PTMs such as phosphorylation and acetylation (Scroggins, B.T. et al. Mol Cell 25, 151-159 (2007), Martinez-Ruiz, A. et al. Proc Natl Acad Sci U S A 102, 8525-8530 (2005), Mollapour, M. et al. Mol Cell 37, 333-343 (2010), Mollapour, M. et al. Mol Cell 41, 672-681 (2011)).

In cancer cells, more co-chaperones are present in chaperone complexes as compared with normal cells (Kamal, A. et al. Nature 425, 407-410 (2003)) and this cancer-specific chaperone machinery enables cancer cells to protect oncoproteins from misfolding and proteasomal degradation (Trepel, J., Mollapour, M., Giaccone, G. & Neckers, L. Nat Rev Cancer 10, 537-549 (2010)). In clinical settings, chaperone proteins are over-expressed in human cancers (Whitesell, L. & Lindquist, S.L. Nat Rev Cancer 5, 761-772 (2005)), and increased expression of chaperones are associated with poor prognosis (Jameel, A. et al. Int J Cancer 50, 409-415 (1992), Pick, E. et al. Cancer Res 67, 2932-2937 (2007)) and drug resistance (Trieb, K. et al. Br J Cancer 82, 85-87 (2000)). Taken together, this evidence suggests that chaperone complexes, including HSP90, are deeply involved in human oncogenesis. In fact, inhibitors targeting HSP90, that bind to the ATP binding pocket, have been developed and are undergoing clinical evaluation (Trepel, J., Mollapour, M., Giaccone, G. & Neckers, L. Nat Rev Cancer 10, 537-549 (2010)). A representative inhibitor, 17-allylamino derivative of geldanamycin (17-AAG or tanespimycin), is one of the geldanamycin derivatives that has been under evaluation in clinical trials (Solit, D.B. & Chiosis, G. Drug Discov Today 13, 38-43 (2008)). For instance, a phase II trial was conducted to validate side effects and therapeutic efficiency of 17-AAG combined with Trastuzumab for HER-2 positive metastatic breast cancer patients, and this combinational therapy was proved to improve the prognosis of patients with tolerable toxicity. The data make sure that further study may explore its therapeutic relevance (Modi, S. et al. Clin Cancer Res (2011)).

The RB1 gene, a member of the pocket family with p107 and p130, was the first known tumor suppressor (Friend SH. et al. Nature 323, 643-646(1986), Ianari A. et al. Cancer Cell 15, 184-194(2009)). The RB protein mainly functions as a transcriptional cofactor that can regulate numerous transcriptional factors and affect the expression of a large number of target genes. In addition, it is well-known that the RB1 protein is targeted by the transforming proteins of the DNA tumor viruses such as adenoviral E1A and is functionally inactivated in the majority of human tumor cells due to mutations of either the RB1 gene itself or its upstream regulators (Trimarchi JM. et al. Nat Rev Mol Cell Biol 3, 11-20(2002)). Its tumor suppressive activity is largely dependent on its ability to directly bind to members of the E2F transcriptional family and prevent them from promoting transcription of genes required for cell proliferation (Trimarchi JM. et al. Nat Rev Mol Cell Biol 3, 11-20(2002)). The RB/E2F pathway has been highlighted by researchers because it is often altered in cancer cells and deregulates the cell proliferation control system (Knudsen ES. et al. Clin Cancer Res 16, 1094-1099(2010)). With regard to the cell proliferation regulation, mitogens reverse transcriptional inhibition of E2F-dependent promoters through sequential activation of CDK-cyclin complexes, which then phosphorylate RB and attenuate its transcriptional co-repressor capability (Knudsen ES .et al. Nat Rev Cancer 8, 714-724(2008), Harbour JW. et al. Cell 98, 859-869(1999), Hinds PW. et al. Cell 70, 993-1006(1992)). This phosphorylation is sufficient to induce RB protein to release E2F, and subsequently induce the E2F-responsive genes at the late G₁ phase. Importantly, the majority of human tumors carry mutations that disable RB protein-mediated repression of E2F (Sherr CJ. et al. Cancer Cell 2, 103-112(2002)). These mutations either inactivate the RB1 gene itself or promote phosphorylation of the RB protein in the absence of normal mitogenic signals through the activation of the cyclin D-CDK4/6 kinases or inactivation of the CDK inhibitor p16. These alterations result in the inappropriate release of E2F, thereby inducing transcriptional activation of E2F target genes and consequently enhancing cell proliferation in cancer cells (Ianari A. et al. Cancer Cell 15, 184-194 (2009)).

In the course of the present invention, it was discovered that Lys 810 of RB1 is mono-methylated by SMYD2, which then promotes cell cycle progression through elevation of RB1 phosphorylation and E2F1 transcriptional activity (Fig.10D). This finding adds the new insight into the deregulation mechanism of the RB/E2F pathway in human cancer cells. Intriguingly, other groups have recently identified lysine methylation on RB protein (Carr SM. et al. EMBO J 30, 317-327 (2011), Saddic LA. et al. J Biol Chem 285, 37733-37740(2010)). Taken together, lysine methylation is likely to play a critical role in regulation of RB functions. Thus, further functional analyses may unveil the importance of lysine methylation in the RB/E2F pathway.

Although some novel targets have been identified and newly emerging drugs are undergoing clinical trials for bladder cancer, current chemotherapy fails to ensure satisfying outcomes, and adverse events are not negligible (Black, P.C., Agarwal, P.K. & Dinney, C.P. Urol Oncol 25, 433-438 (2007), Sonpavde, G. et al. Lancet Oncol 11, 861-870 (2010)). Therefore, the discovery of ideal therapeutic targets that extend the capability of cancer chemotherapy for bladder cancer remains a crucial goal. In the course of the present invention, it was discovered that expression levels of SMYD2 in bladder and other cancer tissues are significantly higher than those in corresponding non-neoplastic tissues. Furthermore, knockdown of SMYD2 significantly suppresses the growth of cancer cells. Considering the fact that the research to generate HMTs inhibitors has recently begun (Copeland, R.A., Solomon, M.E. & Richon, V.M. Nat Rev Drug Discov 8, 724-732 (2009) Spannhoff, A. et al. J Med Chem 50, 2319-2325 (2007)), SMYD2 appears to be an ideal therapeutic target in cancer with fewer adverse events. Further functional analyses of SMYD2 will serve to confirm the utility of SMYD2 as a novel target for anticancer therapy. Additionally, the combination of an HSP90 inhibitor and a SMYD2 inhibitor may serve to reinforce current strategies for cancer therapy. The relationship between SMYD2-dependent HSP90 methylation and sensitivity of the HSP90 inhibitor like 17-AAG is an important topic to be elucidated in the future.

### Industrial Applicability

The gene-expression analysis of cancers described herein has identified a specific gene, i.e., SMYD2, as a target for cancer prevention and therapy. Based on the expression of this differentially expressed gene, the present invention provides a novel molecular diagnostic marker for identifying and detecting cancers. Therefore, the present invention also provides a novel diagnostic strategy using SMYD2. Furthermore, as described herein, SMYD2 is involved in cancer cell survival. Therefore, the present invention also provides a novel molecular target for the treatment and/or prevention of cancer and the inhibition of cancer cell growth. Moreover, as demonstrated herein, novel substrates to be methylated by SMYD2 polypeptide were identified. Therefore, the present invention also provides a novel screening strategy for the treatment and/or prevention of cancer.
The present invention also identified HSP90AB1 and RB1 as genes interacting with SMYD2. Accordingly, the present invention also provides a novel screening strategy that utilizes SMYD2 and HSP90AB1 or RB1 for anti-cancer agents.
As demonstrated herein, RB1 methylation by SMYD2 enhanced cell cycle progression through an increase of RB1 phosphorylation. Thus, the present invention also provides a novel screening strategy for the identification of anti-cancer agents that inhibit the RB1 phosphorylation through RB1 methylation by SMYD2.

The materials and methods described herein are also useful for the identification of additional molecular targets for prevention, diagnosis, and treatment of cancers. The data provided herein add to a comprehensive understanding of cancers, facilitate development of novel diagnostic strategies, and provide clues for identification of molecular targets for therapeutic drugs and preventative agents. Such information contributes to a more profound understanding of tumorigenesis, and provides indicators for developing novel strategies for diagnosis, treatment, and ultimately prevention of cancers.

While the invention has been described in detail and with reference to specific embodiments thereof, it is to be understood that the foregoing description is exemplary and explanatory in nature and is intended to illustrate the invention and its preferred embodiments. Through routine experimentation, one skilled in the art will readily recognize that various changes and modifications can be made therein. Thus, the invention is intended to be defined not by the above description, but by the following claims and their equivalents.

## Claims

1. A method of screening for a candidate substance for either or both of treating and preventing cancer or inhibiting cancer cell growth, said method comprising the following steps:
(a) contacting an SMYD2 polypeptide with an HSP90AB1 polypeptide consisting of SEQ ID NO: 65 under the condition capable of methylation of the HSP90AB1 polypeptide,
(b) detecting the methylation level of the HSP90AB1 polypeptide, and
(c) selecting as the candidate substance the test substance that decreases the methylation level of the HSP90AB1 polypeptide as compared to the methylation level detected in the absence of the test substance.

2. The method of claim 1, wherein the methylation level of the HSP90AB1 polypeptide is detected at lysine 531 and/or lysine 574 of SEQ ID NO: 65.

## Patentansprüche

1. Verfahren zum Screenen auf eine Kandidatensubstanz für eines oder beides von Behandlung und Prophylaxe von Krebs oder Hemmung von Krebszellwachstum, wobei das Verfahren die folgenden Schritte umfasst:
(a) Inkontaktbringen eines SMYD2-Polypeptids mit einem HSP90AB1-Polypeptid, bestehend aus SEQ ID NO: 65, unter der Bedingung, das Methylierung des HSP90AB1-Polypeptids möglich ist,
(b) Detektieren des Methylierungsausmaßes des HSP90AB1-Polypeptids, und
(c) Auswählen der Testsubstanz, die das Methylierungsausmaß des HSP90AB1-Polypeptids im Vergleich zum ohne Testsubstanz detektierten Methylierungsausmaß verringert, als Kandidatensubstanz.

2. Verfahren nach Anspruch 1, wobei das Methylierungsausmaß des HSP90AB1-Polypeptids bei Lysin 531 und/oder Lysin 574 von SEQ ID NO: 65 detektiert wird.

## Revendications

1. Procédé de criblage d'une substance candidate pour traiter et prévenir un cancer ou inhiber la croissance de cellules cancéreuses, ou les deux, ledit procédé comprenant les étapes suivantes :
(a) la mise en contact d'un polypeptide SMYD2 avec un polypeptide HSP90AB1 consistant en SEQ ID NO: 65 dans la condition capable d'entraîner une méthylation du polypeptide HSP90AB1,
(b) la détection du niveau de méthylation du polypeptide HSP90AB1, et
(c) la sélection, en tant que substance candidate, de la substance de test qui réduit le niveau de méthylation du polypeptide HSP90AB1 par comparaison au niveau de méthylation détecté en l'absence de la substance de test.

2. Procédé selon la revendication 1, dans lequel le niveau de méthylation du polypeptide HSP90AB1 est détecté à la lysine 531 et/ou la lysine 574 de SEQ ID NO: 65.
